(19) Europäisches Patentamt
European Patent Office
Office européen des brevets

(11) EP 4 074 345 A1

(12) **EUROPEAN PATENT APPLICATION**
published in accordance with Art. 153(4) EPC

(43) Date of publication:
**19.10.2022 Bulletin 2022/42**

(21) Application number: **20898395.7**

(22) Date of filing: **11.12.2020**

(51) International Patent Classification (IPC):
***A61K 47/68*** (2017.01) ***A61K 39/395*** (2006.01)
***A61K 31/4745*** (2006.01) ***A61P 35/00*** (2006.01)

(52) Cooperative Patent Classification (CPC):
**A61K 31/4745; A61K 39/395; A61K 47/68; A61P 35/00**

(86) International application number:
**PCT/CN2020/135680**

(87) International publication number:
**WO 2021/115426 (17.06.2021 Gazette 2021/24)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **12.12.2019 CN 201911273041**
**30.09.2020 CN 202011060513**

(71) Applicants:
- **Jiangsu Hengrui Pharmaceuticals Co., Ltd.**
**Lianyungang, Jiangsu 222047 (CN)**
- **Shanghai Hengrui Pharmaceutical Co., Ltd.**
**Shanghai 200245 (CN)**

(72) Inventors:
- **YANG, Yang**
**Shanghai 200245 (CN)**
- **XU, Jianyan**
**Shanghai 200245 (CN)**
- **TAO, Weikang**
**Shanghai 200245 (CN)**

(74) Representative: **Viganò, Elena et al**
**Dragotti & Associati S.r.l.**
**Via Nino Bixio, 7**
**20129 Milano (IT)**

# (54) ANTI-CLAUDIN ANTIBODY-DRUG CONJUGATE AND PHARMACEUTICAL USE THEREOF

(57) An anti-claudin antibody-drug conjugate and a pharmaceutical use thereof, specifically relating to a ligand-drug conjugate represented by general formula (Pc-L-Y-D), wherein Pc is an anti-claudin 18.2 antibody or an antigen-binding fragment thereof, and L, Y, and n are as defined in the description.

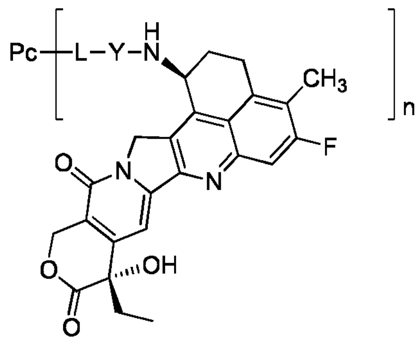

(Pc-L-Y-D)

EP 4 074 345 A1

## Description

The present application claims priority to the Chinese Patent Application (Application No. CN201911273041.7) filed on Dec. 12, 2019 and the Chinese Patent Application (Application No. CN202011060513.3) filed on Sep. 30, 2020.

## TECHNICAL FIELD

The present disclosure relates to an anti-claudin antibody-drug conjugate, and in particular to an anti-claudin18.2 antibody-exatecan analog conjugate, a preparation method therefor, a pharmaceutical composition comprising the antibody-drug conjugate and use thereof in preparing a medicament for treating a claudin18.2-mediated disease or condition, particularly use in preparing an anti-cancer medicament.

## BACKGROUND

The statement herein merely provide background information related to the present disclosure and may not necessarily constitute the prior art.

Claudin-18 (CLDN18), a protein encoded by the claudin18 gene in humans, belongs to the cellular tight-junction protein family, and can control the flowing of molecules between layer cells. The claudin protein comprises four transmembrane regions and two extracellular loops in its structure, with its N-terminus and C-terminus in the cytoplasm. There are two splice variants of claudin-18, claudin 18.1 and claudin 18.2, which differ in sequence by eight amino acids in the first extracellular loops. Claudin 18.1 and claudin 18.2 are different in terms of expression distribution. Claudin 18.1 is selectively expressed in normal lung cells, while the expression of claudin 18.2 is highly restricted in normal cells, but it is frequently ectopically activated and overexpressed in a variety of tumors (e.g., gastric cancer, lung cancer and pancreatic cancer). Claudin18.2 is considered a potential therapeutic target for gastric cancer and other types of cancer, and the discovery of the target also provides a new option for the treatment of gastric cancer. An antibody-drug conjugate (ADC) links a monoclonal antibody or an antibody fragment to a biologically active cytotoxin by a stable chemical linker compound, fully exploiting the binding specificity of the antibody to surface antigens of normal cells and tumor cells and the high-efficiency of the cytotoxic substance, and also avoiding the former's disadvantage of having a poor therapeutic effect, the latter's disadvantage of having serious toxic side effects, and the like. This means that the antibody-drug conjugate can bind to tumor cells more precisely and has a reduced effect on normal cells compared to conventional chemotherapeutic drugs in the past.

At present, some claudin18.2-targeted antibodies and ADC drugs have been reported in patents such as WO2020200196A1, WO2016166122 and WO2016165762. However, there is still a need to develop more effective and safer anti-claudin18.2 antibody-drug conjugates for better use in the treatment of claudin18.2-associated tumors.

## SUMMARY

The present disclosure relates to ADCs of anti-claudin18.2 antibodies and use thereof and provides an ADC drug in which an anti-claudin18.2 antibody or an antigen-binding fragment is conjugated with an exatecan analog, a cytotoxic substance.

Accordingly, the present disclosure is intended to provide a ligand-drug conjugate of general formula (Pc-L-Y-D) or a pharmaceutically acceptable salt thereof:

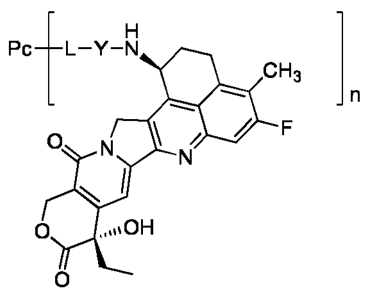

(Pc-L-Y-D)

wherein:

Y is selected from the group consisting of -O-$(CR^aR^b)_m$-$CR^1R^2$-C(O)-, -O-$CR^1R^2$-$(CR^aR^b)_m$-, -O-$CR^1R^2$-,

$-NH-(CR^aR^b)_m-CR^1R^2-C(O)-$ and $-S-(CR^aR^b)_m-CR^1R^2-C(O)-$;

$R^a$ and $R^b$ are identical or different and are each independently selected from the group consisting of hydrogen, deuterium, halogen, alkyl, haloalkyl, deuterated alkyl, alkoxy, hydroxy, amino, cyano, nitro, hydroxyalkyl, cycloalkyl and heterocyclyl; or, $R^a$ and $R^b$, together with carbon atoms connected thereto, form cycloalkyl or heterocyclyl;

$R^1$ is selected from the group consisting of halogen, haloalkyl, deuterated alkyl, cycloalkyl, cycloalkylalkyl, alkoxyalkyl, heterocyclyl, aryl and heteroaryl; $R^2$ is selected from the group consisting of hydrogen, halogen, haloalkyl, deuterated alkyl, cycloalkyl, cycloalkylalkyl, alkoxyalkyl, heterocyclyl, aryl and heteroaryl; or, $R^1$ and $R^2$, together with carbon atoms connected thereto, form cycloalkyl or heterocyclyl;

or, $R^a$ and $R^2$, together with carbon atoms connected thereto, form cycloalkyl or heterocyclyl; m is an integer from 0 to 4;

n is a decimal or an integer from 1 to 10;

L is a linker unit;

Pc is an anti-claudin18.2 antibody or an antigen-binding fragment thereof.

In some embodiments of the present disclosure, in the ligand-drug conjugate of general formula (Pc-L-Y-D) or the pharmaceutically acceptable salt thereof according to any one of the aforementioned embodiments, the anti-claudin18.2 antibody or the antigen-binding fragment thereof comprises a heavy chain variable region and a light chain variable region, wherein:

i) the heavy chain variable region comprises an HCDR1, an HCDR2 and an HCDR3 having sequences identical to those of an HCDR1, an HCDR2 and an HCDR3 of a heavy chain variable region set forth in SEQ ID NO: 3, and the light chain variable region comprises an LCDR1, an LCDR2 and an LCDR3 having sequences identical to those of an LCDR1, an LCDR2 and an LCDR3 of a light chain variable region set forth in SEQ ID NO: 4; or

ii) the heavy chain variable region comprises an HCDR1, an HCDR2 and an HCDR3 having sequences identical to those of an HCDR1, an HCDR2 and an HCDR3 of a heavy chain variable region set forth in SEQ ID NO: 5, and the light chain variable region comprises an LCDR1, an LCDR2 and an LCDR3 having sequences identical to those of an LCDR1, an LCDR2 and an LCDR3 of a light chain variable region set forth in SEQ ID NO: 6.

In some embodiments of the present disclosure, in the ligand-drug conjugate of general formula (Pc-L-Y-D) or the pharmaceutically acceptable salt thereof according to any one of the aforementioned embodiments, the anti-claudin18.2 antibody or the antigen-binding fragment thereof comprises a heavy chain variable region and a light chain variable region, wherein:

iii) the heavy chain variable region comprises an HCDR1, an HCDR2 and an HCDR3 having sequences set forth in SEQ ID NO: 9, SEQ ID NO: 10 and SEQ ID NO: 11, respectively, and the light chain variable region comprises an LCDR1, an LCDR2 and an LCDR3 having sequences set forth in SEQ ID NO: 12, SEQ ID NO: 13 and SEQ ID NO: 14, respectively; or

iv) the heavy chain variable region comprises an HCDR1, an HCDR2 and an HCDR3 having sequences set forth in SEQ ID NO: 15, SEQ ID NO: 16 and SEQ ID NO: 17, respectively, and the light chain variable region comprises an LCDR1, an LCDR2 and an LCDR3 having sequences set forth in SEQ ID NO: 18, SEQ ID NO: 19 and SEQ ID NO: 20, respectively.

In some embodiments of the present disclosure, in the ligand-drug conjugate of general formula (Pc-L-Y-D) or the pharmaceutically acceptable salt thereof according to any one of the aforementioned embodiments, the anti-claudin18.2 antibody is a murine antibody, a chimeric antibody or a humanized antibody.

In some embodiments of the present disclosure, in the ligand-drug conjugate of general formula (Pc-L-Y-D) or the pharmaceutically acceptable salt thereof according to any one of the aforementioned embodiments, the anti-claudin18.2 antibody or the antigen-binding fragment thereof comprises a heavy chain variable region and a light chain variable region, wherein:

(1) the heavy chain variable region has an amino acid sequence set forth in SEQ ID NO: 3 or having at least 90% identity thereto, and the light chain variable region has an amino acid sequence set forth in SEQ ID NO: 4 or having at least 90% identity thereto;

(2) the heavy chain variable region has an amino acid sequence set forth in SEQ ID NO: 24 or having at least 90% identity thereto, and the light chain variable region has an amino acid sequence set forth in SEQ ID NO: 21 or having at least 90% identity thereto;

(3) the heavy chain variable region has an amino acid sequence set forth in SEQ ID NO: 5 or having at least 90% identity thereto, and the light chain variable region has an amino acid sequence set forth in SEQ ID NO: 6 or having at least 90% identity thereto; or

(4) the heavy chain variable region has an amino acid sequence set forth in SEQ ID NO: 31 or having at least 90%

identity thereto, and the light chain variable region has an amino acid sequence set forth in SEQ ID NO: 28 or having at least 90% identity thereto.

In some embodiments of the present disclosure, in the ligand-drug conjugate of general formula (Pc-L-Y-D) or the pharmaceutically acceptable salt thereof according to any one of the aforementioned embodiments, the anti-claudin18.2 antibody is a humanized antibody comprising a framework region derived from a human antibody or a framework region variant thereof, and the framework region variant has reverse mutations of up to 10 (e.g., 1, 2, 3, 4, 5, 6, 7, 8, 9 or 10) amino acids in a light chain framework region and/or a heavy chain framework region of the human antibody; preferably, the framework region variant comprises mutations selected from (a) or (b):

(a) one or more amino acid reverse mutations optionally selected from the group consisting of 22S, 85I and 87H, comprised in the light chain variable region; and/or one or more amino acid reverse mutations optionally selected from the group consisting of 48I, 82T and 69M, comprised in the heavy chain variable region; or
(b) one or more amino acid reverse mutations optionally selected from the group consisting of 4L and 22S, comprised in the light chain variable region; and/or one or more amino acid reverse mutations optionally selected from the group consisting of 38K, 40R, 48I, 66K, 67A, 69L, 71L and 73K, comprised in the heavy chain variable region;

preferably, the framework region variant comprises mutations selected from the group consisting of:

(a-1) 22S, 85I and 87H amino acid reverse mutations comprised in the light chain variable region, and 48I and 82T amino acid reverse mutations comprised in the heavy chain variable region; or
(b-1) an amino acid reverse mutation selected from 4L, comprised in the light chain variable region.

In some embodiments of the present disclosure, in the ligand-drug conjugate of general formula (Pc-L-Y-D) or the pharmaceutically acceptable salt thereof according to any one of the aforementioned embodiments, the anti-claudin18.2 antibody or the antigen-binding fragment thereof comprises a heavy chain variable region and a light chain variable region shown below:

(vii) the heavy chain variable region having a sequence set forth in SEQ ID NO: 3, and the light chain variable region having a sequence set forth in SEQ ID NO: 4;
(viii) the heavy chain variable region having a sequence set forth in SEQ ID NO: 24, SEQ ID NO: 25, SEQ ID NO: 26 or SEQ ID NO: 27, and the light chain variable region having a sequence set forth in SEQ ID NO: 21, SEQ ID NO: 22 or SEQ ID NO: 23;
(ix) the heavy chain variable region having a sequence set forth in SEQ ID NO: 5, and the light chain variable region having a sequence set forth in SEQ ID NO: 6; or
(x) the heavy chain variable region having a sequence set forth in SEQ ID NO: 31, SEQ ID NO: 32, SEQ ID NO: 33 or SEQ ID NO: 34, and the light chain variable region having a sequence set forth in SEQ ID NO: 28, SEQ ID NO: 29 or SEQ ID NO: 30;

preferably, the anti-claudin18.2 antibody or the antigen-binding fragment thereof comprises a heavy chain variable region and a light chain variable region shown below:

(xi) the heavy chain variable region having a sequence set forth in SEQ ID NO: 31, and the light chain variable region having a sequence set forth in SEQ ID NO: 29; or
(xii) the heavy chain variable region having a sequence set forth in SEQ ID NO: 26, and the light chain variable region having a sequence set forth in SEQ ID NO: 23.

In some embodiments of the present disclosure, in the ligand-drug conjugate of general formula (Pc-L-Y-D) or the pharmaceutically acceptable salt thereof according to any one of the aforementioned embodiments, the anti-claudin18.2 antibody or the antigen-binding fragment thereof comprises a heavy chain constant region and a light chain constant region of the antibody; preferably, the heavy chain constant region is selected from the group consisting of human IgG1, IgG2, IgG3 and IgG4 constant regions and conventional variants thereof, and the light chain constant region is selected from the group consisting of human antibody $\kappa$ and $\lambda$ chain constant regions and conventional variants thereof; more preferably, the antibody comprises a heavy chain constant region having a sequence set forth in SEQ ID NO: 7 and a light chain constant region having a sequence set forth in SEQ ID NO: 8; most preferably, the antibody comprises: a heavy chain having at least 90% identity to a heavy chain having an amino acid sequence set forth in SEQ ID NO: 35 or SEQ ID NO: 42, and a light chain having at least 90% identity to a light chain having an amino acid sequence set forth in SEQ ID NO: 36 or SEQ ID NO: 39; or

a heavy chain having at least 90% sequence identity to a heavy chain having an amino acid sequence set forth in SEQ ID NO: 37 or SEQ ID NO: 49, and a light chain having at least 90% sequence identity to a light chain having an amino acid sequence set forth in SEQ ID NO: 38 or SEQ ID NO: 46.

In some embodiments of the present disclosure, in the ligand-drug conjugate of general formula (Pc-L-Y-D) or the pharmaceutically acceptable salt thereof according to any one of the aforementioned embodiments, the anti-claudin18.2 antibody or the antigen-binding fragment thereof comprises:

(c) a heavy chain having a sequence set forth in SEQ ID NO: 35 and a light chain having a sequence set forth in SEQ ID NO: 36;
(d) a heavy chain having a sequence set forth in SEQ ID NO: 42, SEQ ID NO: 43, SEQ ID NO: 44 or SEQ ID NO: 45 and a light chain having a sequence set forth in SEQ ID NO: 39, SEQ ID NO: 40 or SEQ ID NO: 41;
(e) a heavy chain having a sequence set forth in SEQ ID NO: 37 and a light chain having a sequence set forth in SEQ ID NO: 38; or
(f) a heavy chain having a sequence set forth in SEQ ID NO: 49, SEQ ID NO: 50, SEQ ID NO: 51 or SEQ ID NO: 52 and a light chain having a sequence set forth in SEQ ID NO: 46, SEQ ID NO: 47 or SEQ ID NO: 48.

In some embodiments of the present disclosure, in the ligand-drug conjugate of general formula (Pc-L-Y-D) or the pharmaceutically acceptable salt thereof according to any one of the aforementioned embodiments, the anti-claudin18.2 antibody is selected from the group consisting of:

h1901-11, comprising a heavy chain having an amino acid sequence set forth in SEQ ID NO: 44 and a light chain having a sequence set forth in SEQ ID NO: 41; and
h1902-5, comprising a heavy chain having an amino acid sequence set forth in SEQ ID NO: 49 and a light chain having a sequence set forth in SEQ ID NO: 47.

In some embodiments of the present disclosure, the antigen-binding fragment is selected from the group consisting of Fab, Fab', F(ab')2, single-chain antibody (scFv), dimerized V region (diabody) and disulfide-stabilized V region (dsFv).

In some embodiments of the present disclosure, in the ligand-drug conjugate of general formula (Pc-L-Y-D) or the pharmaceutically acceptable salt thereof according to any one of the aforementioned embodiments, n may be an integer or decimal from 1-10, and n may be a mean of 1, 2, 3, 4, 5, 6, 7, 8, 9 or 10. n is a decimal or integer from 2 to 8, preferably a decimal or integer from 3 to 8, more preferably a decimal or integer from 5 to 9, or preferably a decimal or integer from 2 to 7. In some embodiments, n is a decimal or integer from 3.5 to 4.5.

In some embodiments of the present disclosure, in the ligand-drug conjugate of general formula (Pc-L-Y-D) or the pharmaceutically acceptable salt thereof according to any one of the aforementioned embodiments,

Y is $-O-(CR^aR^b)_m-CR^1R^2-C(O)-$;
$R^a$ and $R^b$ are identical or different and are each independently selected from the group consisting of hydrogen, deuterium, halogen and alkyl;
$R^1$ is haloalkyl or $C_{3-6}$ cycloalkyl;
$R^2$ is selected from the group consisting of hydrogen, haloalkyl and $C_{3-6}$ cycloalkyl;
or, $R^1$ and $R^2$, together with carbon atoms connected thereto, form $C_{3-6}$ cycloalkyl;
m is 0 or 1.

In some embodiments of the present disclosure, in the ligand-drug conjugate of general formula (Pc-L-Y-D) or the pharmaceutically acceptable salt thereof according to any one of the aforementioned embodiments, Y is selected from the group consisting of:

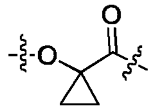 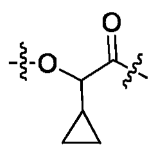 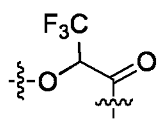 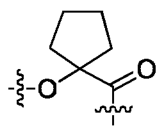 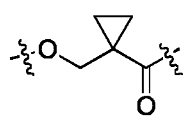 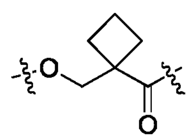

and

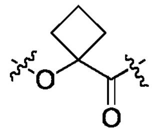

;

wherein an O-terminus of Y is connected to the linker unit L.

In some embodiments of the present disclosure, provided is a ligand-drug conjugate of general formula (Pc-L-Y-D) or a pharmaceutically acceptable salt or solvate thereof, wherein the linker unit -L- is $-L^1-L^2-L^3-L^4-$.

In some embodiments, $L^1$ is selected from the group consisting of -(succinimidyl-3-yl-*N*)-W-C(O)-, $-CH_2-C(O)-NR^3-W-C(O)-$ and -C(O)-W-C(O)-, wherein W is selected from the group consisting of $C_{1-8}$ alkyl, $C_{1-8}$ alkyl-$C_{3-6}$ cycloalkyl and linear heteroalkyl of 1 to 8 chain atoms, and the heteroalkyl comprises 1 to 3 heteroatoms selected from the group consisting of N, O and S, wherein the $C_{1-8}$ alkyl, $C_{1-8}$ alkyl-$C_{3-6}$ cycloalkyl or linear heteroalkyl of 1 to 8 chain atoms is independently optionally further substituted with one or more substituents selected from the group consisting of halogen, hydroxy, cyano, amino, alkyl, chloroalkyl, deuterated alkyl, alkoxy and cycloalkyl.

In some embodiments, $L^2$ is selected from the group consisting of $-NR^4(CH_2CH_2O)p^1CH_2CH_2C(O)-$, $-NR^4(CH_2CH_2O)p^1CH_2C(O)-$, $-S(CH_2)p^1C(O)-$ and a chemical bond, wherein $p^1$ is an integer from 1 to 20.

In some embodiments, $L^3$ is a peptide residue consisting of 2 to 7 amino acids, wherein the amino acids are selected from the group consisting of amino acid residues formed from amino acids from phenylalanine, glycine, valine, lysine, citrulline, serine, glutamic acid and aspartic acid, and are optionally further substituted with one or more substituents selected from the group consisting of halogen, hydroxy, cyano, amino, alkyl, chloroalkyl, deuterated alkyl, alkoxy and cycloalkyl.

In some embodiments, $L^4$ is selected from the group consisting of $-NR^5(CR^6R^7)_t-$, $-C(O)NR^5-$, $-C(O)NR^5(CH_2)_t-$ and a chemical bond, wherein t is an integer from 1 to 6. In some embodiments, $R^3$, $R^4$ and $R^5$ are identical or different and are each independently selected from the group consisting of hydrogen, alkyl, haloalkyl, deuterated alkyl and hydroxyalkyl.

In some embodiments, $R^6$ and $R^7$ are identical or different and are each independently selected from the group consisting of hydrogen, halogen, alkyl, haloalkyl, deuterated alkyl and hydroxyalkyl.

In some embodiments of the present disclosure, in the ligand-drug conjugate of general formula (Pc-L-Y-D) or the pharmaceutically acceptable salt or solvate thereof according to any one of the aforementioned embodiments, the linker unit -L- is $-L^1-L^2-L^3-L^4-$, wherein

$L^1$ is selected from the group consisting of -(succinimidyl-3-yl-*N*)-W-C(O)-, $-CH_2-C(O)-NR^3-W-C(O)-$ and -C(O)-W-C(O)-, wherein W is selected from the group consisting of $C_{1-8}$ alkyl, $C_{1-8}$ alkyl-cycloalkyl and linear heteroalkyl of 1 to 8 chain atoms, and the heteroalkyl comprises 1 to 3 heteroatoms selected from the group consisting of N, O and S, wherein the $C_{1-8}$ alkyl, cycloalkyl and linear heteroalkyl are each independently optionally further substituted with one or more substituents selected from the group consisting of halogen, hydroxy, cyano, amino, alkyl, chloroalkyl, deuterated alkyl, alkoxy and cycloalkyl;

$L^2$ is selected from the group consisting of $-NR^4(CH_2CH_2O)p^1CH_2CH_2C(O)-$, $-NR^4(CH_2CH_2O)p^1CH_2C(O)-$, $-S(CH_2)p^1C(O)-$ and a chemical bond, wherein $p^1$ is an integer from 1 to 20;

$L^3$ is a peptide residue consisting of 2 to 7 amino acids, wherein the amino acids are selected from the group consisting of amino acid residues formed from amino acids from phenylalanine, glycine, valine, lysine, citrulline, serine, glutamic acid and aspartic acid, and are optionally further substituted with one or more substituents selected from the group consisting of halogen, hydroxy, cyano, amino, alkyl, chloroalkyl, deuterated alkyl, alkoxy and cycloalkyl;

$L^4$ is selected from the group consisting of $-NR^5(CR^6R^7)_t-$, $-C(O)NR^5$, $-C(O)NR^5(CH_2)_t-$ and a chemical bond, wherein t is an integer from 1 to 6;

$R^3$, $R^4$ and $R^5$ are identical or different and are each independently selected from the group consisting of hydrogen, alkyl, haloalkyl, deuterated alkyl and hydroxyalkyl;

$R^6$ and $R^7$ are identical or different and are each independently selected from the group consisting of hydrogen, halogen, alkyl, haloalkyl, deuterated alkyl and hydroxyalkyl.

In some embodiments of the present disclosure, in the ligand-drug conjugate of general formula (Pc-L-Y-D) or the pharmaceutically acceptable salt thereof according to any one of the aforementioned embodiments, the linker unit -L- is $-L^1-L^2-L^3-L^4-$, wherein

$L^1$ is

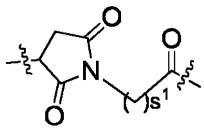,

and $s^1$ is an integer from 2 to 8;
$L^2$ is a chemical bond;
$L^3$ is a tetrapeptide residue, preferably a tetrapeptide residue of GGFG (SEQ ID NO: 55);
$L^4$ is $-NR^5(CR^6R^7)t-$, wherein $R^5$, $R^6$ and $R^7$ are identical or different and are each independently hydrogen or alkyl, and t is 1 or 2;
wherein the $L^1$ terminus is connected to Pc, and the $L^4$ terminus is connected to Y.

In some embodiments of the present disclosure, in the ligand-drug conjugate of general formula (Pc-L-Y-D) or the pharmaceutically acceptable salt thereof according to any one of the aforementioned embodiments, -L- is:

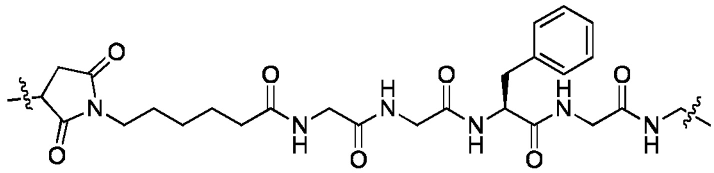.

In some embodiments of the present disclosure, in the ligand-drug conjugate of general formula (Pc-L-Y-D) or the pharmaceutically acceptable salt thereof according to any one of the aforementioned embodiments, -L-Y- is optionally selected from the group consisting of:

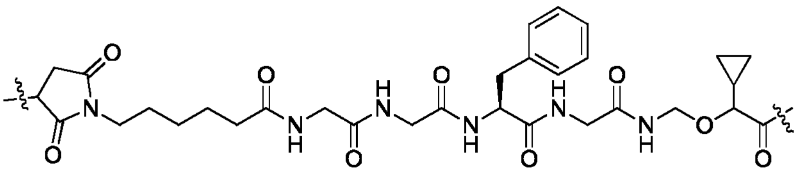

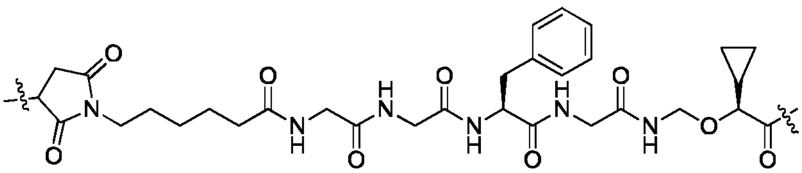

and

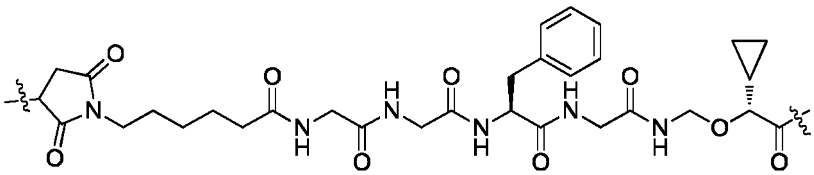.

In some embodiments of the present disclosure, the ligand-drug conjugate of general formula (Pc-L-Y-D) or the pharmaceutically acceptable salt thereof according to any one of the aforementioned embodiments is a ligand-drug conjugate of general formula (Pc-$L_a$-Y-D) or a pharmaceutically acceptable salt thereof,

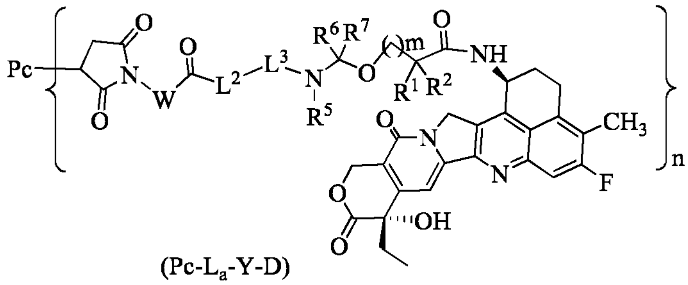

(Pc-$L_a$-Y-D)

wherein:

W, $L^2$, $L^3$, $R^5$, $R^6$ and $R^7$ are as defined in the aforementioned linker unit -L-;
Pc, n, $R^1$, $R^2$ and m are as defined in general formula (Pc-L-Y-D).

In some embodiments of the present disclosure, the ligand-drug conjugate of general formula (Pc-L-Y-D) or the pharmaceutically acceptable salt thereof according to any one of the aforementioned embodiments is a ligand-drug conjugate of general formula (Pc-$L_b$-Y-D) or a pharmaceutically acceptable salt thereof,

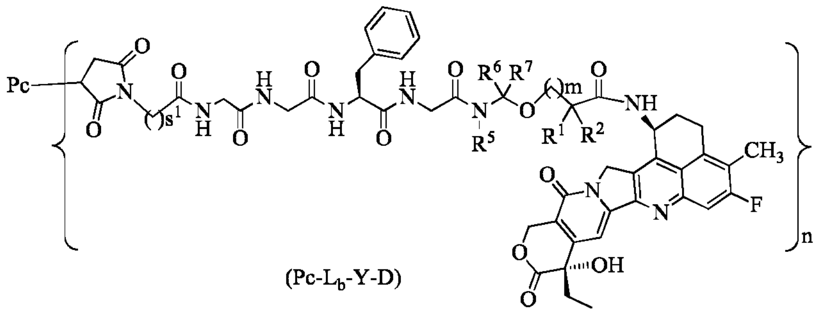

(Pc-$L_b$-Y-D)

wherein:

$s^1$ is an integer from 2 to 8;
Pc, $R^1$ , $R^2$, $R^5$-$R^7$, m and n are as defined in general formula (Pc-$L_a$-Y-D).

In some embodiments of the present disclosure, in the ligand-drug conjugate of general formula (Pc-L-Y-D) or the pharmaceutically acceptable salt thereof according to any one of the aforementioned embodiments, the ligand-drug conjugate is selected from the group consisting of:

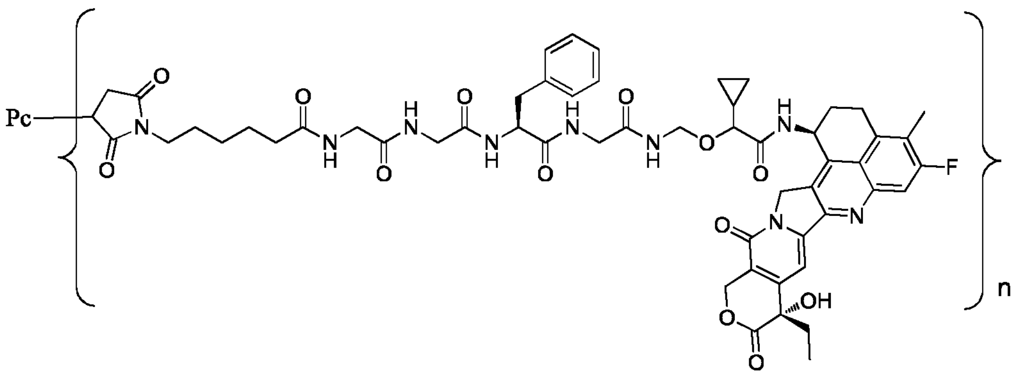

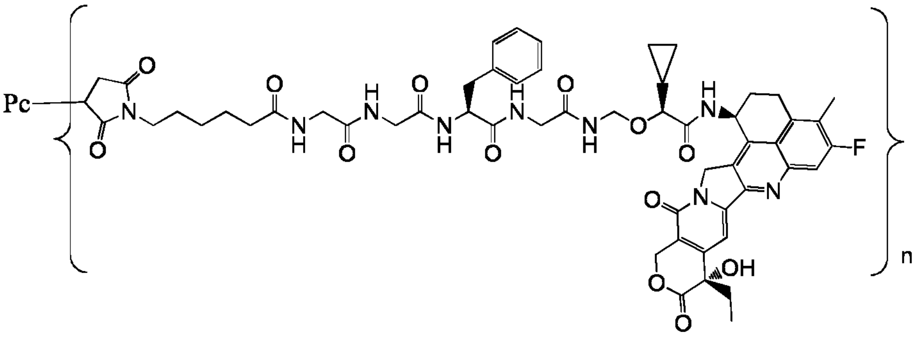

and

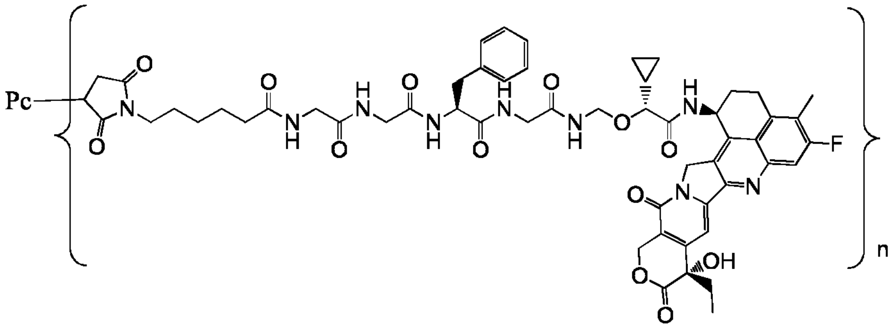

wherein Pc and n are as defined in general formula (Pc-L-Y-D).

In some embodiments of the present disclosure, provided is a ligand-drug conjugate of general formula (Pc-L-Y-D) or a pharmaceutically acceptable salt thereof, wherein the ligand-drug conjugate is selected from the group consisting of:

h1902-5

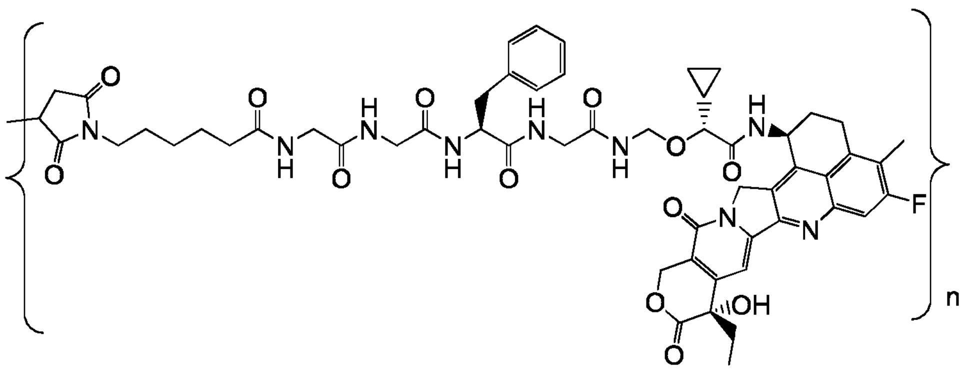

and

h1901-11

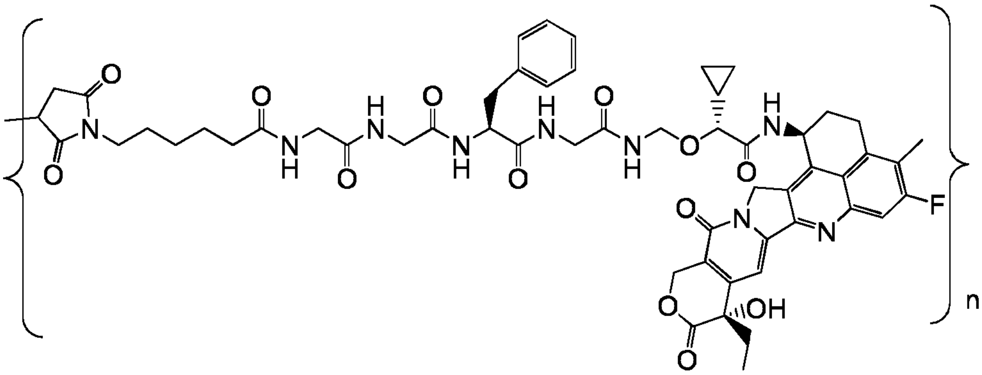

wherein n is as defined in general formula (Pc-L-Y-D), and the antibodies h1902-5 and h1901-11 are as previously defined.

The present disclosure further provides a method for preparing a ligand-drug conjugate of general formula (Pc-$L_a$-Y-D) or a pharmaceutically acceptable salt thereof comprising the following steps:

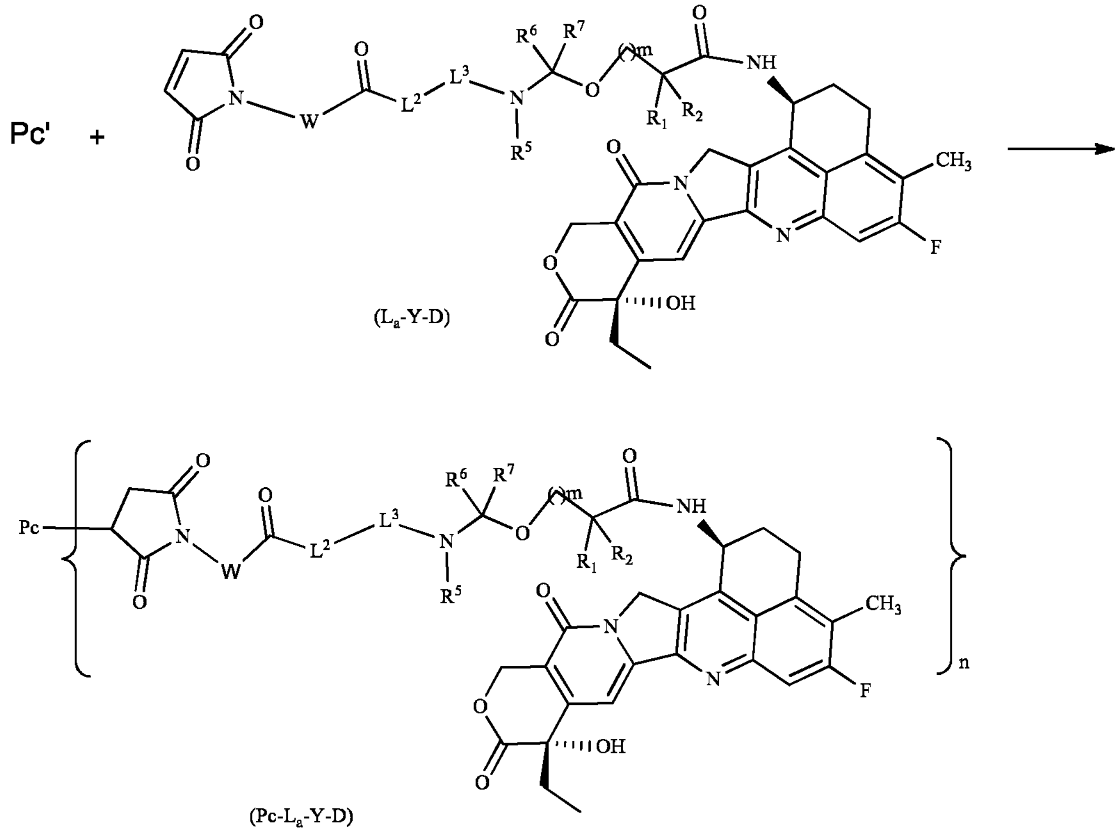

($L_a$-Y-D)

(Pc-$L_a$-Y-D)

subjecting Pc' and a compound of general formula ($L_a$-Y-D) to a coupling reaction to give a compound of general formula (Pc-$L_a$-Y-D); wherein:

Pc is the anti-claudin18.2 antibody or the antigen-binding fragment thereof described above, and Pc' is obtained by reduction of Pc;

W, $L^2$, $L^3$, $R^1$ , $R^2$, $R^5$-$R^7$, m and n are as defined in general formula (Pc-$L_a$-Y-D).

The present disclosure further provides a method for preparing an antibody drug conjugate of general formula (Pc-L'-D) comprising the following step:

Pc +

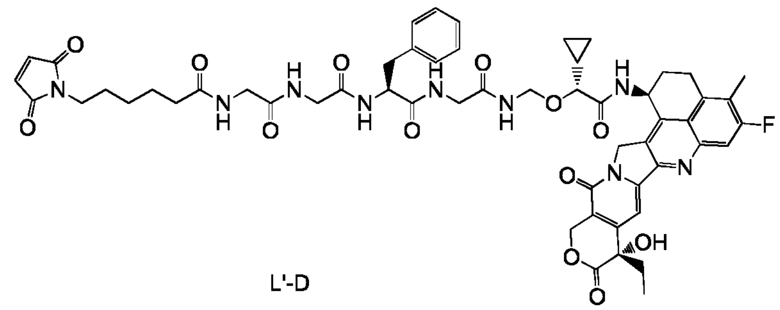

L'-D

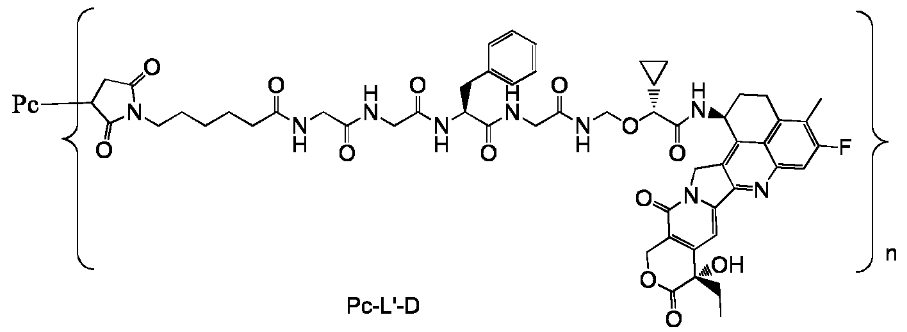

Pc-L'-D

subjecting reduced Pc and general formula (L'-D) to a coupling reaction to give a compound, wherein:

Pc is the anti-claudin18.2 antibody or the antigen-binding fragment thereof described above;
n is as defined in general formula (Pc-L-Y-D).

In another aspect, the present disclosure provides a pharmaceutical composition comprising the ligand-drug conjugate or the pharmaceutically acceptable salt thereof according to any one of the aforementioned embodiments and one or more pharmaceutically acceptable excipients, diluents or carriers.

In another aspect, the present disclosure provides use of the ligand-drug conjugate or the pharmaceutically acceptable salt thereof according to any one of the aforementioned embodiments or a pharmaceutical composition comprising the same as a medicament. In some embodiments, the medicament is for treating a claudin18.2-mediated disease or condition; the claudin18.2-mediated disease or condition is preferably a cancer with high claudin18.2 expression. In some embodiments, the medicament is for treating cancer. In some embodiments, the cancer is preferably head and neck squamous cell carcinoma, head and neck cancer, brain cancer, neuroglioma, glioblastoma multiforme, neuroblastoma, central nervous system carcinoma, neuroendocrine tumor, throat cancer, nasopharyngeal cancer, esophageal cancer, thyroid cancer, malignant pleural mesothelioma, lung cancer, breast cancer, liver cancer, hepatobiliary cancer, pancreatic cancer, stomach cancer, gastrointestinal cancer, intestinal cancer, colon cancer, colorectal cancer, kidney cancer, clear cell renal cell carcinoma, ovarian cancer, endometrial cancer, cervical cancer, bladder cancer, prostate cancer, testicular cancer, skin cancer, melanoma, leukemia, lymphoma, bone cancer, chondrosarcoma, myeloma, multiple myeloma, myelodysplastic syndrome, Krukenberg tumor, myeloproliferative tumor, squamous cell carcinoma, Ewing's sarcoma, systemic light chain amyloidosis or Merkel cell carcinoma; more preferably, the lymphoma is selected from the group consisting of Hodgkin's lymphoma, non-Hodgkin's lymphoma, diffuse large B-cell lymphoma, follicular lymphoma, primary mediastinal large B-cell lymphoma, mantle cell lymphoma, small lymphocytic lymphoma, large B-cell lymphoma rich in T-cells/histiocytes and lymphoplasmacytic lymphoma, the lung cancer is selected from the group consisting of non-small cell lung cancer and small cell lung cancer, and the leukemia is selected from the group consisting of chronic myeloid leukemia, acute myeloid leukemia, lymphocytic leukemia, lymphoblastic leukemia, acute lymphoblastic leukemia, chronic lymphocytic leukemia and myeloid cell leukemia.

In another aspect, the present disclosure provides use of the ligand-drug conjugate or the pharmaceutically acceptable salt thereof according to any one of the aforementioned embodiments or a pharmaceutical composition comprising the same in preparing a medicament for treating a claudin 18.2-mediated disease or condition, wherein the claudin18.2-mediated disease or condition is a cancer with high claudin18.2 expression. In some embodiments, the disease is preferably head and neck squamous cell carcinoma, head and neck cancer, brain cancer, neuroglioma, glioblastoma multiforme, neuroblastoma, central nervous system carcinoma, neuroendocrine tumor, throat cancer, nasopharyngeal cancer, esophageal cancer, thyroid cancer, malignant pleural mesothelioma, lung cancer, breast cancer, liver cancer, hepatobiliary cancer, pancreatic cancer, stomach cancer, gastrointestinal cancer, intestinal cancer, colon cancer, colorectal cancer, kidney cancer, clear cell renal cell carcinoma, ovarian cancer, endometrial cancer, cervical

cancer, bladder cancer, prostate cancer, testicular cancer, skin cancer, melanoma, leukemia, lymphoma, bone cancer, chondrosarcoma, myeloma, multiple myeloma, myelodysplastic syndrome, Krukenberg tumor, myeloproliferative tumor, squamous cell carcinoma, Ewing's sarcoma, systemic light chain amyloidosis or Merkel cell carcinoma; more preferably, the lymphoma is selected from the group consisting of Hodgkin's lymphoma, non-Hodgkin's lymphoma, diffuse large B-cell lymphoma, follicular lymphoma, primary mediastinal large B-cell lymphoma, mantle cell lymphoma, small lymphocytic lymphoma, large B-cell lymphoma rich in T-cells/histiocytes and lymphoplasmacytic lymphoma, the lung cancer is selected from the group consisting of non-small cell lung cancer and small cell lung cancer, and the leukemia is selected from the group consisting of chronic myeloid leukemia, acute myeloid leukemia, lymphocytic leukemia, lymphoblastic leukemia, acute lymphoblastic leukemia, chronic lymphocytic leukemia and myeloid cell leukemia.

In another aspect, the present disclosure provides use of the ligand-drug conjugate or the pharmaceutically acceptable salt thereof according to any one of the aforementioned embodiments or a pharmaceutical composition comprising the same in preparing a medicament for treating or preventing a tumor, wherein the tumor and cancer are preferably head and neck squamous cell carcinoma, head and neck cancer, brain cancer, neuroglioma, glioblastoma multiforme, neuroblastoma, central nervous system carcinoma, neuroendocrine tumor, throat cancer, nasopharyngeal cancer, esophageal cancer, thyroid cancer, malignant pleural mesothelioma, lung cancer, breast cancer, liver cancer, hepatobiliary cancer, pancreatic cancer, stomach cancer, gastrointestinal cancer, intestinal cancer, colon cancer, colorectal cancer, kidney cancer, clear cell renal cell carcinoma, ovarian cancer, endometrial cancer, cervical cancer, bladder cancer, prostate cancer, testicular cancer, skin cancer, melanoma, leukemia, lymphoma, bone cancer, chondrosarcoma, myeloma, multiple myeloma, myelodysplastic syndrome, Krukenberg tumor, myeloproliferative tumor, squamous cell carcinoma, Ewing's sarcoma, systemic light chain amyloidosis or Merkel cell carcinoma; more preferably, the lymphoma is selected from the group consisting of Hodgkin's lymphoma, non-Hodgkin's lymphoma, diffuse large B-cell lymphoma, follicular lymphoma, primary mediastinal large B-cell lymphoma, mantle cell lymphoma, small lymphocytic lymphoma, large B-cell lymphoma rich in T-cells/histiocytes and lymphoplasmacytic lymphoma, the lung cancer is selected from the group consisting of non-small cell lung cancer and small cell lung cancer, and the leukemia is selected from the group consisting of chronic myeloid leukemia, acute myeloid leukemia, lymphocytic leukemia, lymphoblastic leukemia, acute lymphoblastic leukemia, chronic lymphocytic leukemia and myeloid cell leukemia.

In another aspect, the present disclosure further relates to a method for treating and/or preventing a tumor, the method comprising administering to a subject in need thereof a therapeutically or prophylactically effective dose of the ligand-drug conjugate or the pharmaceutically acceptable salt thereof according to any one of the aforementioned embodiments or a pharmaceutical composition comprising the same, wherein the tumor is preferably a cancer associated with high claudin18.2 expression.

In another aspect, the present disclosure further relates to a method for treating or preventing cancer, the method comprising administering to a subject in need thereof a therapeutically or prophylactically effective dose of the ligand-drug conjugate or the pharmaceutically acceptable salt thereof according to any one of the aforementioned embodiments or a pharmaceutical composition comprising the same, wherein the tumor and cancer are preferably head and neck squamous cell carcinoma, head and neck cancer, brain cancer, neuroglioma, glioblastoma multiforme, neuroblastoma, central nervous system carcinoma, neuroendocrine tumor, throat cancer, nasopharyngeal cancer, esophageal cancer, thyroid cancer, malignant pleural mesothelioma, lung cancer, breast cancer, liver cancer, hepatobiliary cancer, pancreatic cancer, stomach cancer, gastrointestinal cancer, intestinal cancer, colon cancer, colorectal cancer, kidney cancer, clear cell renal cell carcinoma, ovarian cancer, endometrial cancer, cervical cancer, bladder cancer, prostate cancer, testicular cancer, skin cancer, melanoma, leukemia, lymphoma, bone cancer, chondrosarcoma, myeloma, multiple myeloma, myelodysplastic syndrome, Krukenberg tumor, myeloproliferative tumor, squamous cell carcinoma, Ewing's sarcoma, systemic light chain amyloidosis or Merkel cell carcinoma; more preferably, the lymphoma is selected from the group consisting of Hodgkin's lymphoma, non-Hodgkin's lymphoma, diffuse large B-cell lymphoma, follicular lymphoma, primary mediastinal large B-cell lymphoma, mantle cell lymphoma, small lymphocytic lymphoma, large B-cell lymphoma rich in T-cells/histiocytes and lymphoplasmacytic lymphoma, the lung cancer is selected from the group consisting of non-small cell lung cancer and small cell lung cancer, and the leukemia is selected from the group consisting of chronic myeloid leukemia, acute myeloid leukemia, lymphocytic leukemia, lymphoblastic leukemia, acute lymphoblastic leukemia, chronic lymphocytic leukemia and myeloid cell leukemia.

The active compound (e.g., the ligand-drug conjugate or the pharmaceutically acceptable salt thereof according to the present disclosure) may be formulated in a form suitable for administration by any suitable route, preferably in a form of a unit dose, or in a form of a single dose that can be self-administered by a subject. The unit dose of the present disclosure may be in a tablet, a capsule, a cachet, a vial, a powder, a granule, a lozenge, a suppository, a regenerating powder or a liquid formulation.

The administration dose of the active compound or composition used in the treatment method of the present disclosure will generally vary with the severity of the disease, the weight of the subject, and the efficacy of the active compound. However, as a general guide, a suitable unit dose may be 0.1 to 1000 mg.

The pharmaceutical composition of the present disclosure may comprise, in addition to the active compound,

one or more excipients selected from the group consisting of a filler, a diluent, a binder, a wetting agent, a disintegrant, an excipient and the like. Depending on the method of administration, the composition may comprise 0.1 to 99 wt.% of active compound.

The claudin18.2 antibody and the antibody-drug conjugate provided by the present disclosure have good affinity for cell surface antigens, good endocytosis efficiency and high tumor inhibition efficiency as well as wider drug application windows, and are suitable for clinical drug application.

## BRIEF DESCRIPTION OF THE DRAWINGS

FIG. 1 shows the results of FACS analysis of the binding of humanized antibodies to human claudin18.2 at the cellular level.

FIG. 2 shows endocytosis of humanized antibodies by NUGC4 cells.

FIGs. 3A to 3C show assays of antibodies for ADCC effects in NUGC4 cells with different levels of claudinl8.2 expression. FIG. 3A shows assays of antibodies for ADCC effects in wild-type NUGC4 cells (with low claudin18.2 expression); FIG. 3B shows assays of antibodies for ADCC effects in NUGC4 cells with moderate claudin18.2 expression; FIG. 3C shows assays of antibodies for ADCC effects in NUGC4 cells with high claudin18.2 expression.

FIG. 4 shows the results of inhibition of tumors by ADC-1 of the present disclosure.

FIG. 5 shows the results of inhibition of tumors by ADC-2 of the present disclosure.

## DETAILED DESCRIPTION

1. Terminology

Unless otherwise defined, all technical and scientific terms used herein have the same meaning as commonly understood by those of ordinary skill in the art to which the present disclosure belongs. Although any methods and materials similar or equivalent to those described herein can also be used to implement or test the present disclosure, preferred methods and materials are described herein. In describing and claiming the present disclosure, the following terms are used in accordance with the definitions below.

When a trade name is used in the present disclosure, it is intended to include the formulation of the product under the trade name and the non-patent drug and active drug components of the product under the trade name.

Unless otherwise stated, the terms used in the specification and claims have the following meanings.

The term "drug" refers to a chemical substance that can alter or ascertain an organism's physiology and pathological state and can be used for the prevention, diagnosis and treatment of diseases. The drug includes a cytotoxic drug. There is no clear boundary between a drug and a toxic substance. The toxic substance refers to a chemical substance that has a toxic effect on organisms and can cause damage to human health even in small doses. Any drug in large doses may induce toxic responses. The cytotoxic drug refers to a substance that inhibits or prevents cell functions and/or cause cell death or cell destruction. The cytotoxic drug can kill tumor cells in principle at a sufficiently high concentration; however, due to lack of specificity, the cytotoxic drug can cause apoptosis of normal cells while killing tumor cells, resulting in serious side effects. The cytotoxic drug includes toxins, such as small molecule toxins or enzymatically active toxins of bacterial, fungal, plant or animal origin, radioisotopes (e.g., $At^{211}$, $I^{131}$ , $I^{125}$, $Y^{90}$, $Re^{186}$, $Re^{188}$, $Sm^{153}$ , $Bi^{212}$, $P^{32}$ and radioactive isotopes of Lu), toxin drugs, chemotherapeutic drugs, antibiotics and nucleolytic enzymes.

The term "linker unit", "linker" or "linker fragment "refers to a chemical structural fragment or bond that is linked at one end to a ligand (e.g., an antibody or an antigen-binding fragment thereof) and at the other end to a drug or is linked to other linkers before being linked to the drug.

The linker may comprise one or more linker components. Exemplary linker components include 6-maleimidocaproyl ("MC"), maleimidopropionyl ("MP"), valine-citrulline ("val-cit" or "vc"), alanine-phenylalanine ("ala-phe"), p-aminobenzyloxycarbonyl ("PAB"), *N*-succinimidyl 4-(2-pyridylthio)pentanoate ("SPP"), *N*-succinimidyl 4-(*N*-maleimidomethyl)cyclohexane-1 carboxylate ("SMCC", also referred to herein as "MCC"), and *N*-succinimidyl(4-iodo-acetyl)aminobenzoate ("SIAB"). The linker may include stretcher units, spacer units and amino acid units, and may be synthesized using methods known in the art, such as those described in US2005-0238649A1. The linker may be a "cleavable linker" favoring the release of drugs in cells. For example, acid-labile linkers (e.g., hydrazones), protease-sensitive (e.g., peptidase-sensitive) linkers, photolabile linkers, dimethyl linkers or disulfide-containing linkers can be used (Chari et al., Cancer Research 52: 127-131(1992); U.S. Patent No. 5,208,020).

Abbreviations

Linker components include, but are not limited to:

MC = 6-maleimidocaproyl, with a structure:

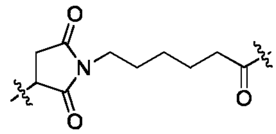

,

Val-Cit or "vc" = valine-citrulline (an exemplary dipeptide in a protease cleavable linker), citrulline = 2-amino-5-ureidopentanoic acid,
PAB =*p*-aminobenzyloxycarbonyl (an example of "self-immolative" linker components), Me-Val-Cit = *N*-methyl-valine-citrulline (where the linker peptide bond has been modified to prevent it from being cleaved by cathepsin B),
MC(PEG)6-OH = maleimidocaproyl-polyethylene glycol (attachable to antibody cysteine), SPP = *N*-succinimidyl 4-(2-pyridylthio)valerate,
SPDP = *N*-succinimidyl 3-(2-pyridyldithio)propionate,
SMCC = succinimidyl-4-(*N*-maleimidomethyl)cyclohexane-1-carboxylate,
IT = iminothiolane.

The term "ligand-drug conjugate" means that a ligand is linked to a biologically active drug by a linking unit. In the present disclosure, the "ligand-drug conjugate" is preferably an antibody-drug conjugate (ADC), which means that a monoclonal antibody or an antibody fragment is linked to a biologically active toxic drug by a linking unit. The antibody may be conjugated to the drug directly or via a linker. The mean number of drug modules conjugated to each antibody (the mean drug loading or drug loading, which may be expressed in terms of n) may range, for example, from about 0 to about 20 drug modules; in certain embodiments, from 1 to about 10 drug modules; and in certain embodiments, from 1 to about 8 drug modules.

The term "mean drug loading" or "drug loading" refers to the mean number of cytotoxic drug loaded per ligand in ligand-drug conjugate molecules, and may also be expressed in terms of the drug-to-antibody ratio. The drug loading may range from 0-12, preferably 1-10, cytotoxic drugs per ligand (Pc). In the embodiments of the present disclosure, the drug loading is expressed in terms of n, which may also be referred to as a DAR (drug-antibody ratio) value and may be a non-zero integer or decimal from 0 to 12, preferably an integer or decimal from 1 to 10, more preferably an integer or decimal from 2 to 8, and most preferably an integer or decimal from 3 to 8. Examples are means of 1, 2, 3, 4, 5, 6, 7, 8, 9 or 10. The mean number of drugs per ADC molecule after coupling reactions can be characterized by conventional methods such as UV/visible spectroscopy, mass spectrometry, ELISA assays and HPLC.

The three-letter and single-letter codes for amino acids used in the present disclosure are as described in J. biol. chem, 243, p3558 (1968).

Claudin18 (CLD18) molecules (Genbank Accession Numbers: splice variant 1 (CLD18A1): NP_057453, NM016369, and splice variant 2 (CLD18A2 or claudin18.2): NM_001002026, NP_001002026) are intrinsic transmembrane proteins, residing within tight junctions of the epithelium and endothelium. In tight junctions, occludins and claudins are predominant transmembrane protein components. Due to the strong intercellular adhesion property of claudins, they create a primary barrier that prevents and controls the paracellular transport of solutes and limits the lateral diffusion of membrane lipids and proteins to maintain cellular polarity. Proteins that form into tight junctions are involved in the structure of epithelium tissues. It is reported that these proteins can hardly get close to antibodies in well-constructed epithelia, but become exposed in tumor cells.

The term "antibody" refers to an immunoglobulin, which is of a tetrapeptide chain structure formed by connection between two heavy chains and two light chains by interchain disulfide bonds. According to differences in the amino acid composition and the order of arrangement of the heavy chain constant regions, immunoglobulins can be divided into five classes, otherwise called isotypes of immunoglobulins, namely IgM, IgD, IgG, IgA and IgE, with their corresponding heavy chains being μ chain, δ chain, γ chain, α chain and ε chain, respectively. Ig of the same class can be divided into different subclasses according to differences in the amino acid composition of the hinge regions and the number and positions of disulfide bonds of the heavy chains; for example, IgG may be divided into IgG1, IgG2, IgG3 and IgG4. Light chains are classified into κ or λ chains by the differences in the constant regions. Each of the five classes of Ig may

have a κ chain or λ chain.

In the heavy and light chains of full-length antibodies, the sequences of about 110 amino acids near the N-terminus vary considerably and thus are referred to as variable regions (Fv regions); the remaining amino acid sequences near the C-terminus are relatively stable and thus are referred to as constant regions. The variable regions comprise 3 hypervariable regions (HVRs) and 4 framework regions (FRs) with relatively conservative sequences. The 3 hypervariable regions determine the specificity of the antibody and thus are also known as complementarity determining regions (CDRs). Each light chain variable region (LCVR) or heavy chain variable region (HCVR) consists of 3 CDRs and 4 FRs arranged from the amino-terminus to the carboxy-terminus in the following order: FR1, CDR1, FR2, CDR2, FR3, CDR3 and FR4. The 3 CDRs of the light chain refer to LCDR1, LCDR2 and LCDR3, and the 3 CDRs of the heavy chain refer to HCDR1, HCDR2 and HCDR3.

The term "fully humanized antibody", "fully human antibody" or "completely human antibody", also known as "fully humanized monoclonal antibody", has both a humanized variable region and a constant region. The development of monoclonal antibodies has four stages, namely murine monoclonal antibodies, chimeric monoclonal antibodies, humanized monoclonal antibodies and fully humanized monoclonal antibodies. Major relevant technologies for the preparation of fully human antibodies include: human hybridoma technology, EBV-transformed B-lymphocyte technology, phage display technology, transgenic mouse antibody preparation technology, single B-cell antibody preparation technology, and the like.

The term "antigen-binding fragment" refers to one or more fragments of an antibody that retain the ability to bind to an antigen. It is shown that a fragment of a full-length antibody can be used to perform the antigen-binding function of the antibody. The binding fragment included in the "antigen-binding fragment" is selected from the group consisting of Fab, Fab', F(ab')2, single-chain antibody (scFv), dimerized V region (diabody), disulfide-stabilized V region (dsFv), and antigen-binding fragments of peptides comprising CDRs; examples include (i) Fab fragments, monovalent fragments consisting of VL, VH, CL and CH1 domains; (ii) F(ab')2 fragments, bivalent fragments comprising two Fab fragments connected by disulfide bridges in the hinge regions; (iii) Fd fragments consisting of VH and CH1 domains; (iv) Fv fragments consisting of VH and VL domains of a single arm of an antibody; (v) single domains or dAb fragments (Ward et al., (1989) Nature 341:544-546) consisting of VH domains; and (vi) isolated complementarity determining regions (CDRs) or (vii) combinations of two or more isolated CDRs which may optionally be linked by synthetic linkers. Furthermore, although the two domains of the Fv fragment, VL and VH, are encoded by separate genes, they can be linked by a synthetic linker by recombination, thereby enabling it to produce a single protein chain in which the VL and VH regions pair to form a monovalent molecule (referred to as single chain Fv (scFv); see, e.g., Bird et al. (1988) Science 242:423-426; and Huston et al. (1988) Proc. Natl. Acad. Sci USA 85:5879-5883). Such single-chain antibodies are also intended to be included in the term "antigen-binding fragment" of an antibody. Such antibody fragments are obtained using conventional techniques known to those skilled in the art, and screened for utility in the same manner as for intact antibodies. Antigen-binding portions may be produced using recombinant DNA technology or by enzymatic or chemical cleavage of intact immunoglobulins. Antibodies may be of different isotypes, e.g., IgG (e.g., subtype IgG1, IgG2, IgG3 or IgG4), IgA1, IgA2, IgD, IgE or IgM antibody.

In general, Fab is an antibody fragment having a molecular weight of about 50,000 and having antigen-binding activity, among fragments obtained by treating an IgG antibody molecule with a protease papain (e.g., cleaving the amino acid residue at position 224 of H chain), in which a portion on the N-terminal side of H chain is combined with L chain by a disulfide bond.

In general, F(ab')2 is an antibody fragment obtained by digesting the portion below the disulfide bond in the IgG hinge region with the enzyme pepsin. It has a molecular weight of about 100,000, has antigen-binding activity, and comprises two Fab regions linked at the hinge position.

In general, Fab' is an antibody fragment having a molecular weight of about 50,000 and having antigen-binding activity, obtained by cleaving the disulfide bond in the hinge region of the F(ab')2 described above.

In addition, Fab' may be produced by inserting DNA encoding the Fab' fragment into a prokaryotic or eukaryotic expression vector and introducing the vector into a prokaryote or a eukaryote to express the Fab'.

The term "single-chain antibody", "single-chain Fv" or "scFv" means a molecule comprising an antibody heavy chain variable domain (or VH) and an antibody light chain variable domain (or VL) linked by a linker. Such scFv molecules may have a general structure: $NH_2$-VL-linker-VH-COOH or $NH_2$-VH-linker-VL-COOH. Suitable linkers in the prior art consist of repeated GGGGS amino acid sequences or variants thereof, for example, 1-4 repeated variants (Holliger et al. (1993), Proc. Natl. Acad. Sci. USA 90:6444-6448). Other linkers that can be used in the present disclosure are described in Alfthan et al. (1995), Protein Eng. 8:725-731; Choi et al. (2001), Eur. J. Immunol. 31:94-106; Hu et al. (1996), Cancer Res. 56:3055-3061, Kipriyanov et al. (1999), J. Mol. Biol. 293:41-56; and Roovers et al. (2001), Cancer Immunol.

The term "CDR" refers to one of the 6 hypervariable regions within the variable domain of an antibody which primarily contribute to antigen binding. In general, there are three CDRs (HCDR1, HCDR2 and HCDR3) in each heavy chain variable region and three CDRs (LCDR1, LCDR2 and LCDR3) in each light chain variable region. The amino acid

sequence boundaries of the CDRs can be determined using any of a variety of well-known schemes. One of the most common definitions for the 6 CDRs is provided in Kabat E.A. et al., (1991) Sequences of proteins of immunological interest. NIH Publication 91-3242. As used herein, the Kabat definition of CDRs applies only to the CDR1, CDR2 and CDR3 of the light chain variable domain, and to the CDR2 and CDR3 of the heavy chain variable domain. Also included are the "Chothia" numbering scheme, the "ABM" numbering scheme, the "contact" numbering scheme (see Martin, ACR. Protein Sequence and Structure Analysis of Antibody Variable Domains[J]. 2001), the ImMunoGenTics(IMGT) numbering scheme (Lefranc M.P., Dev. Comp. Immunol., 27, 55-77(2003)), etc.

The term "antibody framework" refers to a portion of a variable domain VL or VH, which serves as a framework for the antigen-binding loops (CDRs) of the variable domain. It is essentially a variable domain without CDRs.

The term "epitope" or "antigenic determinant" refers to a site on an antigen to which an immunoglobulin or antibody binds. Epitopes typically comprise at least 3, 4, 5, 6, 7, 8, 9, 10, 11, 12, 13, 14 or 15 contiguous or non-contiguous amino acids in a unique spatial conformation. See, e.g., Epitope Mapping Protocols in Methods in Molecular B iology, volume 66, G.E.Morris, Ed. (1996).

The terms "specific binding", "selective binding", "selectively bind to" and "specifically bind to" refer to the binding of an antibody to an epitope on a predetermined antigen. In general, the antibody binds with an affinity (KD) of less than about $10^{-7}$ M, e.g., less than about $10^{-8}$ M, $10^{-9}$ M, or $10^{-10}$ M or less.

The term "KD" refers to the dissociation equilibrium constant for antibody-antigen interaction. In general, the antibody (or antigen-binding fragment) of the present disclosure binds to claudin18.2 (or an epitope thereof) with a dissociation equilibrium constant (KD) of less than about $10^{-7}$ M, e.g., less than about $10^{-8}$ M or $10^{-9}$ M; for example, the KD value is determined using FACS method for the affinity of the antibody of the present disclosure for cell surface antigens.

The term "nucleic acid molecule" refers to a DNA molecule or an RNA molecule. The nucleic acid molecule may be single-stranded or double-stranded, but is preferably double-stranded DNA. A nucleic acid is "operably linked" when it is placed into a functional relationship with another nucleic acid sequence. For example, a promoter or enhancer is operably linked to a coding sequence if it affects the transcription of the coding sequence.

The amino acid sequence "identity" refers to the percentage of amino acid residues shared by a first sequence and a second sequence, wherein in aligning the amino acid sequences, gaps are introduced, when necessary, to achieve maximum percent sequence identity, and any conservative substitution is not considered as part of the sequence identity. For the purpose of determining percent amino acid sequence identity, alignment can be achieved in a variety of ways that fall within the art, for example, using publicly available computer software such as BLAST, BLAST-2, ALIGN-2 or Megalign (DNASTAR) software. Those skilled in the art can determine parameters suitable for measuring alignment, including any algorithm required to achieve maximum alignment of the full length of the aligned sequences.

The term "expression vector" refers to a nucleic acid molecule capable of transporting another nucleic acid to which it is linked. In one embodiment, the vector is a "plasmid", which refers to a circular double-stranded DNA loop into which other DNA segments can be ligated. In another embodiment, the vector is a viral vector where other DNA segments can be ligated into the viral genome. The vectors disclosed herein are capable of autonomously replicating in a host cell into which they are introduced (e.g., bacterial vectors having a bacterial origin of replication and episomal mammalian vectors) or capable of integrating into the genome of a host cell after being introduced into the host cell and thus replicating with the host genome (e.g., non-episomal mammalian vectors). Methods of producing and purifying antibodies and antigen-binding fragments are well known in the art, for example, those described in chapters 5-8 and 15 of Antibodies: A Laboratory Manual, Cold Spring Harbor Press. Antigen-binding fragments can likewise be prepared using conventional methods. The antibody or antigen-binding fragment described in the present invention is genetically engineered to contain one or more additional human FRs in the non-human CDRs. Human FR germline sequences can be obtained at the website of ImMunoGeneTics(IMGT) http://imgt.cines.fr or from the immunoglobulin journal, Lefranc, G., the Immunoglobulin FactsBook, Academic Press, 2001ISBN012441351, by alignment with the IMGT human antibody variable region germline gene database and the MOE software.

The term "host cell" refers to a cell into which an expression vector is introduced. Host cells may include bacterial, microbial, plant or animal cells. Bacteria susceptible to transformation include members of the *Enterobacteriaceae* family, such as strains of *Escherichia coli* or *Salmonella;* members of the *Bacillaceae* family, such as *Bacillus subtilis; Pneumococcus; Streptococcus;* and *Haemophilus influenzae.* Suitable microorganisms include *Saccharomyces cerevisiae* and *Pichia pastoris.* Suitable animal host cell lines include CHO (Chinese hamster ovary cell line) and NS0 cells.

The engineered antibody or antigen-binding fragment of the present disclosure can be prepared and purified using conventional methods. For example, cDNA sequences encoding the heavy and light chains can be cloned and recombined into an expression vector. Recombinant immunoglobulin expression vectors can be stably transfected into host cells. As a more recommended prior art, mammalian expression systems will result in glycosylation of the antibody, particularly at the N-terminal site of the Fc region. Positive clones are expanded in a medium in a bioreactor to produce the antibody. The culture with the secreted antibody can be purified using conventional techniques, for example, using an A or G Sepharose FF column. Non-specifically bound fractions are washed away. The bound antibody is eluted using

pH gradient method, and the antibody fragments are detected by SDS-PAGE and collected. The antibody can be filtered and concentrated using conventional methods. Soluble mixtures and polymers can also be removed using conventional methods, such as molecular sieves and ion exchange. The resulting product needs to be immediately frozen, e.g., at -70 °C, or lyophilized.

The term "peptide" refers to a compound fragment between an amino acid and a protein. It is formed by connecting 2 or more amino acid molecules by peptide bonds, and is a structural and functional fragment of the protein.

The term "sugar" refers to biomacromolecules consisting of C, H and O elements. They can be classified into monosaccharides, disaccharides, polysaccharides, etc.

The term "alkyl" refers to a saturated aliphatic hydrocarbon group, which is a linear or branched group containing 1 to 20 carbon atoms, preferably an alkyl group containing 1 to 12 carbon atoms, more preferably an alkyl group containing 1 to 10 carbon atoms, and most preferably an alkyl group containing 1 to 6 carbon atoms (containing 1, 2, 3, 4, 5 or 6 carbon atoms). Non-limiting examples include methyl, ethyl, *n*-propyl, isopropyl, *n*-butyl, isobutyl, *tert*-butyl, *sec*-butyl, *n*-pentyl, 1,1-dimethylpropyl, 1,2-dimethylpropyl, 2,2-dimethylpropyl, 1-ethylpropyl, 2-methylbutyl, 3-methylbutyl, *n*-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, *n*-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, *n*-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl, *n*-nonyl, 2-methyl-2-ethylhexyl, 2-methyl-3-ethylhexyl, 2,2-diethylpentyl, *n*-decyl, 3,3-diethylhexyl, 2,2-diethylhexyl, various side-chain isomers thereof, etc. More preferred is a lower alkyl having 1 to 6 carbon atoms, and non-limiting examples include methyl, ethyl, *n*-propyl, isopropyl, *n*-butyl, isobutyl, *tert*-butyl, *sec*-butyl, *n*-pentyl, 1,1-dimethylpropyl, 1,2-dimethylpropyl, 2,2-dimethylpropyl, 1-ethylpropyl, 2-methylbutyl, 3-methylbutyl, *n*-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, etc. Alkyl may be substituted or unsubstituted. When substituted, the substituent may be substituted at any available connection site, wherein the substituent is preferably one or more of the following groups independently selected from the group consisting of alkyl, alkenyl, alkynyl, alkoxy, alkylthio, alkylamino, halogen, mercapto, hydroxy, nitro, cyano, cycloalkyl, heterocycloalkyl, aryl, heteroaryl, cycloalkoxy, heterocycloalkoxy, cycloalkylthio, heterocycloalkylthio and oxo.

The term "heteroalkyl" refers to an alkyl group containing one or more heteroatoms selected from the group consisting of N, O and S, wherein the alkyl is as defined above. The term "alkylene" refers to a saturated linear or branched aliphatic hydrocarbon group having 2 residues derived from the parent alkane by removal of two hydrogen atoms from the same carbon atom or two different carbon atoms. It is a linear or branched group containing 1 to 20 carbon atoms, preferably alkylene containing 1 to 12 carbon atoms, more preferably alkylene containing 1 to 6 carbon atoms (containing 1, 2, 3, 4, 5 or 6 carbon atoms). Non-limiting examples of alkylene groups include, but are not limited to, methylene($-CH_2-$), 1,1-ethylidene($-CH(CH_3)-$), 1,2-ethylidene($-CH_2CH_2$)-, 1,1-propylidene($-CH(CH_2CH_3)-$), 1 ,2-propylidene($-CH_2CH(CH_3)-$), 1,3-propylidene($-CH_2CH_2CH_2-$), 1,4-butylidene($-CH_2CH_2CH_2CH_2-$), 1,5-butylidene($-CH_2CH_2CH_2CH_2CH_2-$), etc. The alkylene may be substituted or unsubstituted. When substituted, the substituent may be substituted at any available connection site with one or more substituents preferably independently optionally selected from the group consisting of alkyl, alkenyl, alkynyl, alkoxy, alkylthio, alkylamino, halogen, mercapto, hydroxy, nitro, cyano, cycloalkyl, heterocyclyl, aryl, heteroaryl, cycloalkoxy, heterocycloalkoxy, cycloalkylthio, heterocycloalkylthio and oxo.

The term "alkoxy" refers to -O-(alkyl) and -O-(unsubstituted cycloalkyl), wherein the alkyl or cycloalkyl is as defined above. Non-limiting examples of alkoxy include: methoxy, ethoxy, propoxy, butoxy, cyclopropyloxy, cyclobutoxy, cyclopentyloxy, and cyclohexyloxy. Alkoxy may be optionally substituted or unsubstituted, and when it is substituted, the substituent is preferably one or more of the following groups independently selected from the group consisting of: alkyl, alkenyl, alkynyl, alkoxy, alkylthio, alkylamino, halogen, mercapto, hydroxy, nitro, cyano, cycloalkyl, heterocycloalkyl, aryl, heteroaryl, cycloalkoxy, heterocycloalkoxy, cycloalkylthio and heterocycloalkylthio.

The term "cycloalkyl" refers to a saturated or partially unsaturated monocyclic or polycyclic hydrocarbon substituent. The cycloalkyl ring contains 3 to 20 carbon atoms, preferably 3 to 12 carbon atoms, more preferably 3 to 10 carbon atoms, and most preferably 3 to 8 carbon atoms (containing 3, 4, 5, 6, 7 or 8 carbon atoms). Non-limiting examples of monocyclic cycloalkyl include cyclopropyl, cyclobutyl, cyclopentyl, cyclopentenyl, cyclohexyl, cyclohexenyl, cyclohexadienyl, cycloheptyl, cycloheptatrienyl, cyclooctyl, etc. Polycyclic cycloalkyl includes spiro cycloalkyl, fused cycloalkyl, and bridged cycloalkyl.

The term "heterocyclyl" refers to a saturated or partially unsaturated monocyclic or polycyclic hydrocarbon substituent containing 3 to 20 ring atoms, wherein one or more of the ring atoms are heteroatoms selected from the group consisting of nitrogen, oxygen and $S(O)_m$ (where m is an integer of 0, 1 or 2), excluding a cyclic portion of -O-O-, -O-S- or -S-S-, and the remaining ring atoms are carbon atoms. It preferably contains 3 to 12 ring atoms, of which 1 to 4 are heteroatoms (1, 2, 3 or 4 heteroatoms); more preferably, a cycloalkyl ring contains 3 to 10 ring atoms (3, 4, 5, 6,

7, 8, 9 or 10 ring atoms). Non-limiting examples of monocyclic heterocyclyl include pyrrolidinyl, piperidinyl, piperazinyl, morpholinyl, thiomorpholinyl, homopiperazinyl, etc. Polycyclic heterocyclyl includes spiro heterocyclyl, fused heterocyclyl, and bridged heterocyclyl.

The term "spiro heterocyclyl" refers to a 5- to 20-membered polycyclic heterocyclyl group in which monocyclic rings share one atom (referred to as the spiro atom), wherein one or more ring atoms are heteroatoms selected from the group consisting of nitrogen, oxygen and $S(O)_m$ (where m is an integer from 0 to 2), and the remaining ring atoms are carbon atoms. These rings may contain one or more double bonds, but none of them has a fully conjugated π-electron system. Preferably, the spiro heterocyclyl is 6- to 14-membered, and more preferably 7- to 10-membered. According to the number of spiro atoms shared among the rings, the spiro heterocyclyl may be monospiro heterocyclyl, bispiro heterocyclyl or polyspiro heterocyclyl, preferably monospiro heterocyclyl and bispiro heterocyclyl, and more preferably 4-membered/4-membered, 4-membered/5-membered, 4-membered/6-membered, 5-membered/5-membered or 5-membered/6-membered monospiro heterocyclyl. Non-limiting examples of spiro heterocyclyl include:

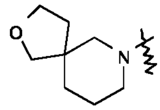 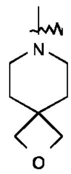 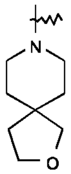 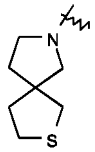 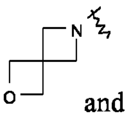 and 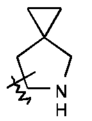 .

The term "fused heterocyclyl" refers to a 5- to 20-membered polycyclic heterocyclyl in which each ring shares a pair of adjacent atoms with the other rings in the system, wherein one or more of the rings may contain one or more double bonds, but none of them has a fully conjugated π-electron system, wherein one or more of the ring atoms are heteroatoms selected from the group consisting of nitrogen, oxygen or $S(O)_m$ (where m is an integer of 0, 1 or 2), and the remaining ring atoms are carbon atoms. Preferably, the fused heterocyclyl is 6- to 14-membered, and more preferably 7- to 10-membered (a 7-, 8-, 9- or 10-membered ring). According to the number of the formed rings, the fused heterocyclyl may be bicyclic, tricyclic, tetracyclic or polycyclic, preferably bicyclic or tricyclic, and more preferably 5-membered/5-membered or 5-membered/6-membered bicyclic fused heterocyclyl. Non-limiting examples of fused heterocyclyl include:

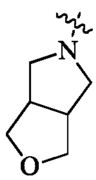 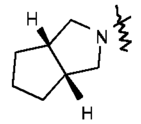 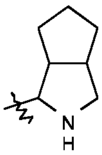 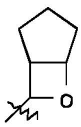 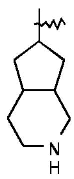 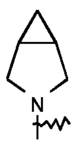 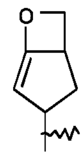 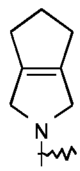

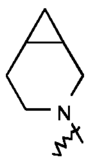 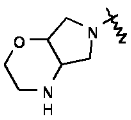 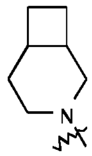 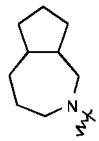 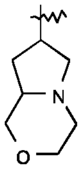 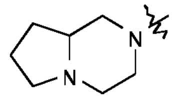 and 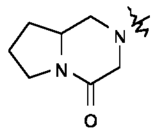 .

The term "bridged heterocyclyl" refers to a 5- to 14- membered polycyclic heterocyclyl in which any two rings share two carbon atoms that are not directly attached to each other, wherein these rings may contain one or more double bonds, but none of them has a fully conjugated π-electron system, wherein one or more of the ring atoms are heteroatoms selected from the group consisting of nitrogen, oxygen and $S(O)_m$ (where m is an integer of 0, 1 or 2), and the remaining ring atoms are carbon atoms. Preferably, the fused heterocyclyl is 6- to 14-membered, and more preferably 7- to 10-membered (a 7-, 8-, 9- or 10-membered ring). According to the number of the formed rings, the bridged heterocyclyl may be bicyclic, tricyclic, tetracyclic or polycyclic, preferably bicyclic, tricyclic or tetracyclic, and more preferably bicyclic or tricyclic. Non-limiting examples of bridged heterocyclyl include:

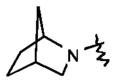 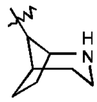 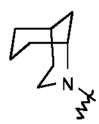 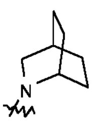

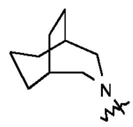 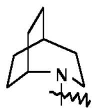 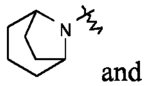 and 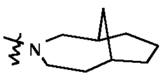 .

The heterocyclyl ring may be fused to an aryl, heteroaryl or cycloalkyl ring, wherein the ring attached to the parent structure is heterocyclyl; non-limiting examples include, but are not limited to:

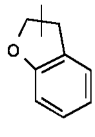 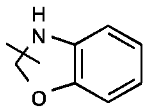 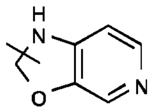 , 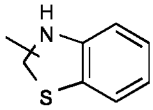

, etc.

Heterocyclyl may be optionally substituted or unsubstituted, and when it is substituted, the substituent is preferably one or more of the following groups independently selected from the group consisting of: alkyl, alkenyl, alkynyl, alkoxy, alkylthio, alkylamino, halogen, mercapto, hydroxy, nitro, cyano, cycloalkyl, heterocycloalkyl, aryl, heteroaryl, cycloalkoxy, heterocycloalkoxy, cycloalkylthio, heterocycloalkylthio and oxo.

The term "aryl" refers to a 6- to 14-membered, preferably 6- to 10-membered (6-, 7-, 8-, 9- or 10-membered), carbon monocyclic or fused polycyclic (i.e., rings sharing a pair of adjacent carbon atoms) group having a conjugated $\pi$-electron system such as phenyl and naphthyl, preferably phenyl. The aryl ring may be fused to a heteroaryl, heterocyclyl or cycloalkyl ring, wherein the ring attached to the parent structure is the aryl ring; non-limiting examples include, but are not limited to:

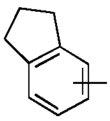 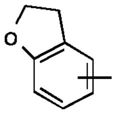 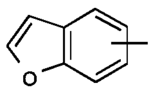 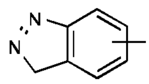 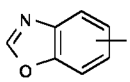 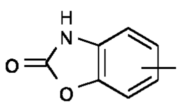 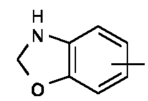

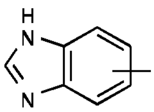 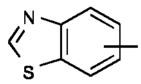 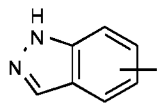 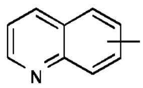 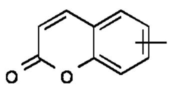 and 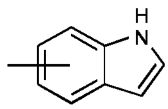 .

Aryl may be substituted or unsubstituted, and when it is substituted, the substituent is preferably one or more of the following groups independently selected from the group consisting of: alkyl, alkenyl, alkynyl, alkoxy, alkylthio, alkylamino, halogen, mercapto, hydroxy, nitro, cyano, cycloalkyl, heterocycloalkyl, aryl, heteroaryl, cycloalkoxy, heterocycloalkoxy, cycloalkylthio and heterocycloalkylthio.

The term "heteroaryl" refers to a heteroaromatic system containing 1 to 4 heteroatoms (1, 2, 3 or 4 heteroatoms) and 5 to 14 ring atoms, wherein the heteroatoms are selected from the group consisting of oxygen, sulfur and nitrogen. Heteroaryl is preferably 5- to 10-membered (5-, 6-, 7-, 8-, 9-, 10-membered heteroaryl), more preferably 5- or 6-membered, such as furanyl, thienyl, pyridinyl, pyrrolyl, *N*-alkylpyrrolyl, pyrimidinyl, pyrazinyl, imidazolyl and tetrazolyl. The heteroaryl ring may be fused to an aryl, heterocyclyl or cycloalkyl ring, wherein the ring attached to the parent structure is heteroaryl; non-limiting examples include, but are not limited to:

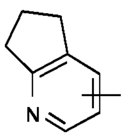 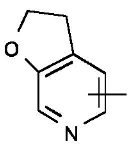 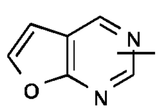 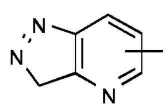 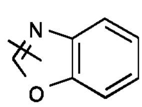 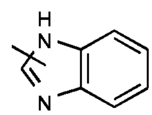

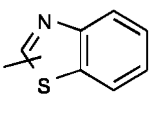 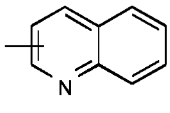 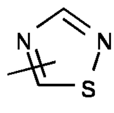 and 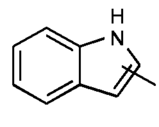 .

Heteroaryl may be optionally substituted or unsubstituted, and when it is substituted, the substituent is preferably one or more of the following groups independently selected from the group consisting of: alkyl, alkenyl, alkynyl, alkoxy, alkylthio, alkylamino, halogen, mercapto, hydroxy, nitro, cyano, cycloalkyl, heterocycloalkyl, aryl, heteroaryl, cycloalkoxy, heterocycloalkoxy, cycloalkylthio and heterocycloalkylthio.

The term "amino protecting group" refers to a group that can be easily removed and is intended to protect an amino group from being changed when a reaction is conducted elsewhere in the molecule. Non-limiting examples include 9-fluorenylmethoxycarbonyl, *tert*-butoxycarbonyl, acetyl, benzyl, allyl, *p*-methoxybenzyl, etc. These groups may be optionally substituted with 1-3 substituents (1, 2 or 3 substituents) selected from the group consisting of halogen, alkoxy and nitro. The amino protecting group is preferably 9-fluorenylmethoxycarbonyl.

The term "haloalkyl" refers to an alkyl group in which the hydrogen atoms are substituted with one or more halogens, wherein the alkyl group is as defined above. The term "deuterated alkyl" refers to an alkyl group in which the hydrogen atoms are substituted with one or more deuterium atoms, wherein the alkyl group is as defined above.

The term "hydroxyalkyl" refers to an alkyl group wherein the hydrogen of the alkyl group is replaced by one or more hydroxy groups, wherein alkyl is as defined above. The term "hydroxy" refers to -OH group.

The term "halogen" refers to fluorine, chlorine, bromine or iodine.

The term "amino" refers to $-NH_2$.

The term "nitro" refers to $-NO_2$.

The term "cyano" refers to -CN.

The term "acylamino" refers to -C(O)N(alkyl) or (cycloalkyl), wherein the alkyl and cycloalkyl are as defined above.

The term "optional" or "optionally" means that the event or circumstance subsequently described may, but not necessarily, occur, and that the description includes instances where the event or circumstance occurs or does not occur. For example, "a heterocyclyl group optionally substituted with alkyl" means that alkyl may be, but not necessarily, present, and that the description includes instances where the heterocyclyl group is or is not substituted with alkyl.

"Substituted" means that one or more, preferably up to 5, and more preferably 1, 2 or 3, hydrogen atoms in the group are independently substituted with a substituent. The substituent is only in its possible chemical position, and those skilled in the art will be able to determine (experimentally or theoretically) possible or impossible substitution without undue efforts. For example, it may be unstable when an amino or hydroxy group having a free hydrogen is bound to a carbon atom having an unsaturated (e.g., olefinic) bond.

The term "pharmaceutical composition" refers to a mixture containing one or more of the compounds described herein or a physiologically/pharmaceutically acceptable salt or pro-drug thereof, and other chemical components, and other components for example physiologically/pharmaceutically acceptable carriers and excipients. The purpose of the pharmaceutical composition is to promote the administration to an organism, which facilitates the absorption of the active ingredient, thereby exerting biological activities. The term "pharmaceutically acceptable salt" refers to a salt of the ligand-drug conjugate of the present disclosure, or a salt of the active compound of the present disclosure. Such salts are safe and effective when used in subjects and possess the required biological activity. The ligand-antibody drug conjugate of the present disclosure contains at least one amino group, and thus can form a salt with an acid. Non-limiting examples of pharmaceutically acceptable salts include: hydrochloride, hydrobromide, hydriodate, sulphate, bisulfate, citrate, acetate, succinate, ascorbate, oxalate, nitrate, sorbate, hydrophosphate, dihydrophosphate, salicylate, hydrocitrate, tartrate, maleate, fumarate, formate, benzoate, mesylate, ethanesulfonate, benzenesulphonate and *p*-toluenesulfonate.

In one embodiment of the present disclosure, the cytotoxic drug is conjugated to a mercapto group of the antibody by a linker unit.

The loading of the ligand-cytotoxic drug conjugate can be controlled using the following non-limiting methods, including:

(1) controlling a molar ratio of a linking reagent to a monoclonal antibody,
(2) controlling reaction time and temperature, and
(3) selecting different reagents.

For preparation of conventional pharmaceutical compositions, reference is made to *Chinese Pharmacopoeia.*

The term "pharmaceutically acceptable carrier" for the drug of the present disclosure refers to a system that can alters the manner in which the drug gets into a subject and the distribution of the drug in the subject, controls the release rate of the drug, and delivers the drug to a targeted organ. The drug carrier release and targeted system can reduce drug degradation and loss, reduce side effects and improve bioavailability. For example, polymeric surfactants that can be used as carriers can self-assemble due to their unique amphiphilic structures to form various forms of aggregates, such as micelles, microemulsions, gels, liquid crystals and vesicles, as preferred examples. The aggregates have the capability of encapsulating drug molecules and have good permeability for membranes, and therefore can be used as excellent drug carriers.

The term "excipient" is an addition, apart from the active compound, to a pharmaceutical composition. It may also be referred to as an adjuvant. For example, binders, fillers, disintegrants, lubricants in tablets; base part in semisolid ointment and cream preparations; preservatives, antioxidants, corrigents, fragrances, cosolvents, emulsifiers, solubilizers, tonicity adjusting agents, colorants and the like in liquid formulations can all be referred to as excipients.

The term "diluent", also referred to as a filler, is used primarily to increase the weight and volume of the tablet. The addition of the diluent not only ensures a certain volume, but also reduces the dose deviation of the main ingredients, and improves the drug's compression moldability and the like. When the drug in tablet form contains oily components, an absorbent is necessarily added to absorb the oily components so as to maintain a "dry" state and thus to facilitate the preparation of the tablet. Examples include starch, lactose, inorganic salts of calcium, microcrystalline cellulose and the like.

The pharmaceutical composition may be in the form of a sterile injectable aqueous solution. Available and acceptable vehicles or solvents include water, Ringer's solution and isotonic sodium chloride solution. The sterile injectable formulation may be a sterile injectable oil-in-water microemulsion in which the active ingredient is dissolved in the oil phase. For example, the active ingredient is dissolved in a mixture of soybean oil and lecithin. The oil solution is then added to a mixture of water and glycerol and treated to form a microemulsion. The injection or microemulsion can be locally injected into the bloodstream of a subject in large quantities. Alternatively, it may be desirable to administer the solution and microemulsion in such a way as to maintain a constant circulating concentration of the compound of the present disclosure. To maintain such a constant concentration, a continuous intravenous delivery device may be used. An example of such a device is a Deltec CADD-PLUS. TM. 5400 intravenous injection pump.

The pharmaceutical composition may be in the form of a sterile injectable aqueous or oily suspension for intramuscular and subcutaneous administration. The suspension can be prepared according to the prior art using those suitable dispersants or wetting agents and suspending agents mentioned above. The sterile injectable formulation may also be a sterile injection or suspension prepared in a parenterally acceptable non-toxic diluent or solvent, e.g., a solution prepared in 1,3-butanediol. In addition, a sterile fixed oil may be conventionally used as a solvent or a suspending medium. For this purpose, any blend fixed oil including synthetic mono- or di-glycerides can be used. In addition, fatty acids such as oleic acid may also be used in the preparation of injections.

2. Synthesis Method

For the synthesis purpose, the following technical schemes for synthesis are adopted:
A method for preparing a compound of general formula (Pc-$L_a$-Y-D) comprises the following steps:

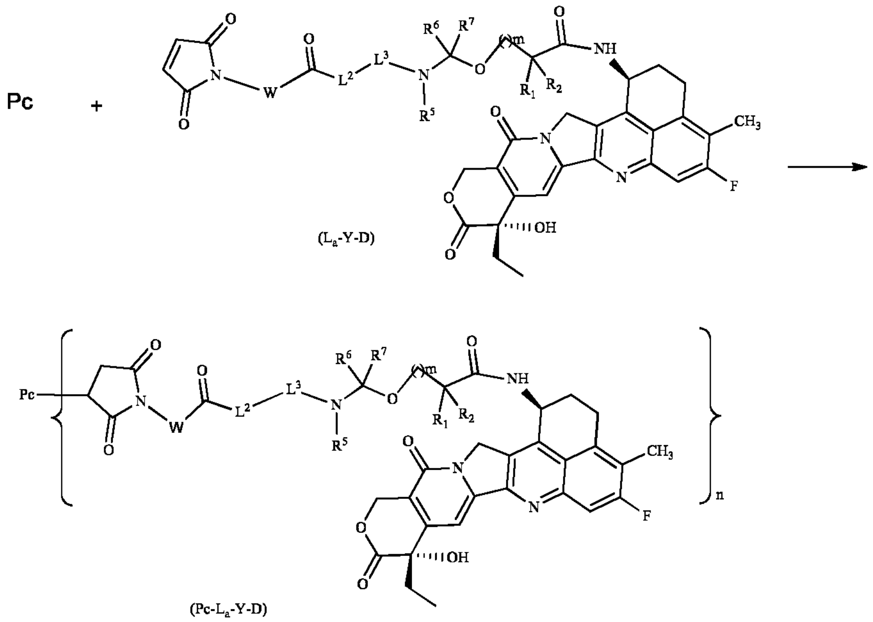

Pc +

($L_a$-Y-D)

(Pc-$L_a$-Y-D)

subjecting reduced Pc and general formula ($L_a$-Y-D) to a coupling reaction to give a compound of general formula (Pc-$L_a$-Y-D), wherein the reducing agent is preferably TCEP; particularly, the disulfide bonds in the antibody are preferably reduced; Pc, W, $L^2$, $L^3$, $R^1$, $R^2$, $R^5$-$R^7$, m and n are as defined in general formula (Pc-$L_a$-Y-D).

One or more embodiments of the present disclosure are described in detail in the specification above. Although any methods and materials similar or identical to those described herein can also be used to implement or test the

present disclosure, preferred methods and materials are described below. Other features, objects and advantages of the present disclosure will be apparent from the description and the claims. In the specification and claims, singular forms include plural referents unless otherwise indicated clearly in the context. Unless otherwise defined, all technical and scientific terms used herein have the meanings generally understood by those of ordinary skill in the art to which the present disclosure belongs. All the patents and publications cited in the specification are incorporated by reference. The following examples are set forth in order to more fully illustrate the preferred embodiments of the present disclosure. These examples should not be construed in any way as limiting the scope of the present disclosure, which is defined by the claims.

## DETAILED DESCRIPTION

### I. Preparation of Antibodies

#### Example 1-1. Construction of Cell Strain with High Claudinl8.2 expression

pCDH-hClaudin18.2 lentiviral expression vector plasmids, pVSV-G and pCMV-dR8.91 lentiviral system packaging vectors were transfected into viral packaging cells 293T using Lipofectamine 3000 transfection reagent. The medium supernatant containing viruses was collected, filtered, and centrifuged at ultra-high speed. The human gastric signet ring cell carcinoma cell strain NUGC4 was allowed to be infected with the concentrated virus, screened using puromycin for two to three weeks, and subjected to FACS single-cell sorting.

Claudin18.2 expression levels were determined according to tumor IHC scores. Cells with claudin18.2 expression levels similar to that of a tumor with a tumor IHC score of 3 points were considered cells with high expression, and cells with claudin18.2 expression levels similar to that of a tumor with a tumor IHC score of 2 points were considered cells with moderate expression. According to the claudin18.2 expression level on the NUGC4 cell surface determined by FACS, NUGC4/hClaudin18.2 monoclonal cell strains with high claudin18.2 expression were selected. The claudin18.2 expression level on the wild-type NUGC4 cell surface was also determined by FACS, and NUGC4 clonal cell strains with moderate claudin18.2 expression were selected. The wild-type NUGC4 cells were cells with low claudin18.2 expression.

The selected monoclonal cell strains were expanded and preserved by freezing for subsequent experiments.

Claudin18.2 sequence Genbank: NP_001002026: (SEQ ID NO: 1)

MAVTACQGLGFVVSLIGIAGIIAATCMDQWSTQDLYNNPVTAVFNYQGLWRSCV

RESSGFTECRGYFTLLGLPAMLQAVRALMIVGIVLGAIGLLVSIFALKCIRIGSME
DSAKANMTLTSGIMFIVSGLCAIAGVSVFANMLVTNFWMSTANMYTGMGGMV
QTVQTRYTFGAALFVGWVAGGLTLIGGVMMCIACRGLAPEETNYKAVSYHASG
HSVAYKPGGFKASTGFGSNTKNKKIYDGGARTEDEVQSYPSKHDYV;

Claudin18.2 DNA sequence: (SEQ ID NO: 2)

1 AGAATTGCGC TGTCCACTTG TCGTGTGGCT CTGTGTCGAC ACTGTGCGCC ACCATGGCCG

61 TGACTGCCTG TCAGGGCTTG GGGTTCGTGG TTTCACTGAT TGGGATTGCG GGCATCATTG

121 CTGCCACCTG CATGGACCAG TGGAGCACCC AAGACTTGTA CAACAACCCC GTAACAGCTG

181 TTTTCAACTA CCAGGGGCTG TGGCGCTCCT GTGTCCGAGA GAGCTCTGGC TTCACCGAGT

241 GCCGGGGCTA CTTCACCCTG CTGGGGCTGC CAGCCATGCT GCAGGCAGTG CGAGCCCTGA

301 TGATCGTAGG CATCGTCCTG GGTGCCATTG GCCTCCTGGT ATCCATCTTT GCCCTGAAAT

361 GCATCCGCAT TGGCAGCATG GAGGACTCTG CCAAAGCCAA CATGACACTG ACCTCCGGGA

421 TCATGTTCAT TGTCTCAGGT CTTTGTGCAA TTGCTGGAGT GTCTGTGTTT GCCAACATGC

481 TGGTGACTAA CTTCTGGATG TCCACAGCTA ACATGTACAC CGGCATGGGT GGGATGGTGC

541 AGACTGTTCA GACCAGGTAC ACATTTGGTG CGGCTCTGTT CGTGGGCTGG GTCGCTGGAG

601 GCCTCACACT AATTGGGGGT GTGATGATGT GCATCGCCTG CCGGGGCCTG GCACCAGAAG

661 AAACCAACTA CAAAGCCGTT TCTTATCATG CCTCAGGCCA CAGTGTTGCC TACAAGCCTG

721 GAGGCTTCAA GGCCAGCACT GGCTTTGGGT CCAACACCAA AAACAAGAAG ATATACGATG

781 GAGGTGCCCG CACAGAGGAC GAGGTACAAT CTTATCCTTC CAAGCACGAC TATGTGTAAT

841 GCTCTAAGAC CTCTCAGCAC GGGCGGAAGA AACTCCCGGA GAGCTCACCC AAAAAACAAG

901 GAGATCCCAT CTAGATTTCT TCTTGCTTTT GACTCACAGC TGGAAGTTAG AAAAGCCTCG

961 ATTTCATCTT TGGAGAGGCC AAATGGTCTT AGCCTCAGTC TCTGTCTCTA AATATTCCAC

1021 CATAAAACAG CTGAGTTATT TATGAATTAG AGGCTATAGC TCACATTTTC AATCCTCTAT

1081 TTCTTTTTTT AAATATAACT TTCTACTCTG ATGAGAGAAT GTGGTTTTAA TCTCTCTCTC
1141 ACATTTTGAT GATTTAGACA GACTCCCCCT CTTCCTCCTA GTCAATAAAC CCATTGATGA
1201 TCTATTTCCC AGCTTATCCC CAAGAAAACT TTTGAAAGGA AAGAGTAGAC CCAAAGATGT
1261 TATTTTCTGC TGTTTGAATT TTGTCTCCCC ACCCCCAACT TGGCTAGTAA TAAACACTTA
1321 CTGAAGAAGA AGCAATAAGA GAAAGATATT TGTAATCTCT CCAGCCCATG ATCTCGGTTT
1381 TCTTACACTG TGATCTTAAA AGTTACCAAA CCAAAGTCAT TTTCAGTTTG AGGCAACCAA
1441 ACCTTTCTAC TGCTGTTGAC ATCTTCTTAT TACAGCAACA CCATTCTAGG AGTTTCCTGA
1501 GCTCTCCACT GGAGTCCTCT TTCTGTCGCG GGTCAGAAAT TGTCCCTAGA TGAATGAGAA
1561 AATTATTTTT TTTAATTTAA GTCCTAAATA TAGTTAAAAT AAATAATGTT TTAGTAAAAT
1621 GATACACTAT CTCTGTGAAA TAGCCTCACC CCTACATGTG GATAGAAGGA AATGAAAAAA
1681 TAATTGCTTT GACATTGTCT ATATGGTACT TTGTAAAGTC ATGCTTAAGT ACAAATTCCA
1741 TGAAAAGCTC ACTGATCCTA ATTCTTTCCC TTTGAGGTCT CTATGGCTCT GATTGTACAT
1801 GATAGTAAGT GTAAGCCATG TAAAAAGTAA ATAATGTCTG GGCACAGTGG CTCACGCCTG
1861 TAATCCTAGC ACTTTGGGAG GCTGAGGAGG AAGGATCACT TGAGCCCAGA AGTTCGAGAC
1921 TAGCCTGGGC AACATGGAGA AGCCCTGTCT CTACAAAATA CAGAGAGAAA AAATCAGCCA
1981 GTCATGGTGG CCTACACCTG TAGTCCCAGC ATTCCGGGAG GCTGAGGTGG GAGGATCACT
2041 TGAGCCCAGG GAGGTTGGGG CTGCAGTGAG CCATGATCAC ACCACTGCAC TCCAGCCAGG
2101 TGACATAGCG AGATCCTGTC TAAAAAAATA AAAAATAAAT AATGGAACAC AGCAAGTCCT
2161 AGGAAGTAGG TTAAAACTAA TTCTTTAAAA AAAAAAAAAA GTTGAGCCTG AATTAAATGT
2221 AATGTTTCCA AGTGACAGGT ATCCACATTT GCATGGTTAC AAGCCACTGC CAGTTAGCAG
2281 TAGCACTTTC CTGGCACTGT GGTCGGTTTT GTTTTGTTTT

GCTTTGTTTA GAGACGGGGT

2341 CTCACTTTCC AGGCTGGCCT CAAACTCCTG CACTCAAGCA ATTCTTCTAC CCTGGCCTCC

2401 CAAGTAGCTG GAATTACAGG TGTGCGCCAT CACAACTAGC TGGTGGTCAG TTTTGTTACT

2461 CTGAGAGCTG TTCACTTCTC TGAATTCACC TAGAGTGGTT GGACCATCAG ATGTTTGGGC

2521 AAAACTGAAA GCTCTTTGCA ACCACACACC TTCCCTGAGC TTACATCACT GCCCTTTTGA

2581 GCAGAAAGTC TAAATTCCTT CCAAGACAGT AGAATTCCAT CCCAGTACCA AAGCCAGATA

2641 GGCCCCCTAG GAAACTGAGG TAAGAGCAGT CTCTAAAAAC TACCCACAGC AGCATTGGTG

2701 CAGGGGAACT TGGCCATTAG GTTATTATTT GAGAGGAAAG TCCTCACATC AATAGTACAT

2761 ATGAAAGTGA CCTCCAAGGG GATTGGTGAA TACTCATAAG GATCTTCAGG CTGAACAGAC

2821 TATGTCTGGG GAAAGAACGG ATTATGCCCC ATTAAATAAC AAGTTGTGTT CAAGAGTCAG

2881 AGCAGTGAGC TCAGAGGCCC TTCTCACTGA GACAGCAACA TTTAAACCAA ACCAGAGGAA

2941 GTATTTGTGG AACTCACTGC CTCAGTTTGG GTAAAGGATG AGCAGACAAG TCAACTAAAG

3001 AAAAAAGAAA AGCAAGGAGG AGGGTTGAGC AATCTAGAGC ATGGAGTTTG TTAAGTGCTC

3061 TCTGGATTTG AGTTGAAGAG CATCCATTTG AGTTGAAGGC CACAGGGCAC AATGAGCTCT

3121 CCCTTCTACC ACCAGAAAGT CCCTGGTCAG GTCTCAGGTA GTGCGGTGTG GCTCAGCTGG

3181 GTTTTTAATT AGCGCATTCT CTATCCAACA TTTAATTGTT TGAAAGCCTC CATATAGTTA

3241 GATTGTGCTT TGTAATTTTG TTGTTGTTGC TCTATCTTAT TGTATATGCA TTGAGTATTA

3301 ACCTGAATGT TTTGTTACTT AAATATTAAA AACACTGTTA TCCTACAGTT.

**Examples 1-2. Production of Anti-Human Claudinl8.2 Monoclonal Antibody**

1. Immunization

Anti-human claudin18.2 monoclonal antibodies were produced by immunizing mice. Laboratory SJL white mice, female, 6-8 weeks of age (Beijing Vital River Laboratory Animal Technology Co., Ltd., animal production license number:

SCXK(Beijing)2012-0001). Housing environment: SPF grade. The purchased mice were housed in a laboratory environment for 1 week, in a 12/12 hour light/dark cycle, at a temperature of 20-25 °C, with humidity at 40-60%. The acclimatized mice were immunized according to the following scheme. The antigens for immunization were huClaudin18.2-HEK293 cells (a HEK-293 cell strain stably transfected with human claudin18.2 plasmid).

Immunization scheme: Prior to the first cell immunization, each mouse was intraperitoneally (IP) injected with 0.1 mL of TiterMax® Gold Adjuvant (Sigma Cat No. T2684), and, a half hour later, with 0.1 mL of normal saline-diluted cellular fluid at a concentration of $1 \times 10^8$/mL. The cells were uniformly pipetted, and then inoculation was performed at days 0, 14, 28, 42 and 56. Blood was collected at days 21, 35, 49 and 63, and the antibody titer in mouse serum was determined by ELISA. After 4-5 immunizations, mice in which the antibody titer in serum was high and was reaching a plateau were selected for splenocyte fusion. The mice were immunized with a booster dose of $1 \times 10^7$ cells by intraperitoneal injection (IP) 3 days prior to splenocyte fusion.

2. Splenocyte fusion

Spleen lymphocytes and myeloma cells, Sp2/0 cells (ATCC® CRL-8287™), were fused by following an optimized PEG-mediated fusion procedure to give hybridoma cells. The resulting hybridoma cells were resuspended in complete medium (IMDM medium containing 20% FBS, 1× HAT and 1× OPI) at a density of 0.5-1 $\times 10^6$/mL and seeded in a 96-well plate at 100 μL/well. The plate was incubated at 37 °C with 5% $CO_2$ for 3-4 days, supplemented with HAT complete medium at 100 μL/well, and incubated for another 3-4 days to form pinpoint-like clones. The supernatant was removed and HT complete medium (IMDM medium containing 20% FBS, 1× HT and 1× OPI) was added at 200 μL/well. The plate was incubated at 37 °C with 5% $CO_2$ for 3 days, followed by an ELISA assay.

3. Screening of hybridoma cells

Hybridoma culture supernatants were assayed using a combined ELISA method according to the density the hybridoma cells were growing at. Cells that had good binding capacity to huClaudin18.2-HEK293 cells but were not bound to HEK293 were selected, expanded, and frozen. Subcloning was performed 2 to 3 times to obtain single-cell clones.

A cell binding assay was also performed for each cell subcloning. Hybridoma clones were obtained by the above screening process, and antibodies were further prepared using a serum-free cell culture method. The antibodies were purified, according to the purification example, for use in the test examples.

**Examples 1-3. Humanization of Murine Antibodies**

Monoclonal hybridoma cell strains mAb1901 and mAb1902 with high *in vitro* activity were selected. The monoclonal antibody sequences therein were cloned, followed by humanization, recombinant expression and activity evaluation.

The cloning of sequences from hybridomas is as follows. Hybridoma cells growing at log phase were harvested, and the RNA was extracted using Trizol (Invitrogen, 15596-018) (following the procedures in the kit instructions) and reverse transcribed (PrimeScript™ Reverse Transcriptase, Takara, cat # 2680A). The cDNA obtained by reverse transcription was amplified by PCR using mouse Ig-Primer Set (Novagen, TB326 Rev.B 0503) and then sent for sequencing by a sequencing company. The amino acid sequences corresponding to the obtained DNA sequences of the hybridoma cells are set forth in SEQ ID NO: 3-6:

Murine heavy chain variable region of mAb1901 (SEQ ID NO: 3)

EVQLMESGGGLVKPGGSLKLSCAASGFTFSDYGIHWVRQAPEMGLEWIAYISR
GSSTIYYADTVKGRFTMSRDNAKNTLFLQMTSLRSEDTAMYYCARGGYDTRN
AMDYWGQGTSVTVSS;

Murine light chain variable region of mAb1901 (SEQ ID NO: 4)

DIVMTQSPSSLSVSAGEKVTMSCKSSQSLLNSGNQKNYLAWYQQKPGQPPKLLI
YGASTRASGVPDRFTGSGSGTDFTLTISSVQAEDLAIYHCQNDLYYPLTFGAGTK
LELK;

Murine heavy chain variable region of mAb1902 (SEQ ID NO: 5)

EVQLQESGAELVKPGASVKLSCKASGYIFTSYWMHWVKQRPGQGLEWIGMIHP
NSGSTNYNEKFKGKATLTLDKSSSTAYMQLSSLPSEDSAVYYCARLKTGNSFDY
WGQGTTLTVSS;

Murine light chain variable region of mAb1902 (SEQ ID NO: 6)

DIVLTQSPSSLTVTAGEKVTMSCKSSQSLLNSGNQKNYLTWYQQKPGQPPKLLI
YWASTRESGVPDRFTGSGSGTDFTLTISSVQAEDLAIYYCQNAYTYPFTFGSGTK
LEIK;

The above murine heavy chain and light chain variable regions were joined to the heavy chain constant region of human IgG1 antibody and the human κ light chain constant region described below, respectively, to form chimeric antibodies ch1901 and ch1902. The constant regions were selected from the group consisting of the following sequences:

Heavy chain constant region of human IgG1 antibody: (SEQ ID NO: 7)

ASTKGPSVFPLAPSSKSTSGGTAALGCLVKDYFPEPVTVSWNSGALTSGVHTFPA
VLQSSGLYSLSSVVTVPSSSLGTQTYICNVNHKPSNTKVDKKVEPKSCDKTHTC
PPCPAPELLGGPSVFLFPPKPKDTLMISRTPEVTCVVVDVSHEDPEVKFNWYVD
GVEVHNAKTKPREEQYNSTYRVVSVLTVLHQDWLNGKEYKCKVSNKALPAPIE
KTISKAKGQPREPQVYTLPPSRDELTKNQVSLTCLVKGFYPSDIAVEWESNGQPE
NNYKTTPPVLDSDGSFFLYSKLTVDKSRWQQGNVFSCSVMHEALHNHYTQKSL
SLSPGK;

Human κ light chain constant region: (SEQ ID NO: 8)

RTVAAPSVFIFPPSDEQLKSGTASVVCLLNNFYPREAKVQWKVDNALQSGNSQE
SVTEQDSKDSTYSLSSTLTLSKADYEKHKVYACEVTHQGLSSPVTKSFNRGEC.

Humanization of the murine monoclonal antibodies was performed as described in many publications in the art. Briefly, human constant domains were used in place of parent (murine antibody) constant domains, and human germline antibody sequences were selected, based on the homology of the murine and human antibodies, for CDR grafting. The present invention selects candidate molecules with good activity for humanization, and the results are as follows.

1. CDRs of murine antibodies

The amino acid residues of the VH/VL CDRs in Table 1 were identified using the Kabat numbering system and annotated.

The CDR sequences of the murine antibodies are described in Table 1:

Table 1. CDR sequences of murine antibodies

| Antibody | mAb1901 |
|---|---|
| HCDR1 | DYGIH (SEQ ID NO: 9) |
| HCDR2 | YISRGSSTIYYADTVKG (SEQ ID NO: 10) |
| HCDR3 | GGYDTRNAMDY (SEQ ID NO: 11) |
| LCDR1 | KSSQSLLNSGNQKNYLA (SEQ ID NO: 12) |
| LCDR2 | GASTRAS (SEQ ID NO: 13) |
| LCDR3 | QNDLYYPLT (SEQ ID NO: 14) |
| Antibody | mAb1902 |
| HCDR1 | SYWMH (SEQ ID NO: 15) |
| HCDR2 | MIHPNSGSTNYNEKFKGR (SEQ ID NO: 16) |
| HCDR3 | LKTGNSFDY (SEQ ID NO: 17) |
| LCDR1 | KSSQSLLNSGNQKNYLT (SEQ ID NO: 18) |
| LCDR2 | WASTRES (SEQ ID NO: 19) |
| LCDR3 | QNAYTYPFT (SEQ ID NO: 20) |

2. Selection of human germline FR region sequences

On the basis of the typical structure of the murine antibody VH/VLCDR obtained, the heavy chain and light chain variable region sequences were compared with an antibody Germine database to obtain a human germline template with high homology. The human germline light chain framework region was derived from a human κ light chain gene.

2.1. Humanization of mAb1901 and reverse mutation design

A suitable human antibody germline was selected to perform humanization on mAb1901 murine antibody. The CDRs of murine antibody mAb1901 were grafted into the selected humanization template to replace humanized variable regions, followed by recombination with an IgG constant region to form a complete antibody. Meanwhile, reverse mutations were introduced into the FR region in the V region of the humanized antibody. Exemplary reverse mutations and combinations thereof are as follows:

Table 2. Humanized antibodies of mAb1901 and reverse mutations*

| Light chain variable regions of humanized antibodies of mAb1901 | | Heavy chain variable regions of humanized antibodies of mAb1901 | |
|---|---|---|---|
| VL1 | None | VH1 | None |
| VL2 | N22S | VH2 | N82T |
| VL3 | N22S, V85I, Y87H | VH3 | V48I, N82T |
| | | VH4 | I69M, N82T |
| * All amino acid positions in the table are numbered according to the Kabat numbering scheme; in N82T of the heavy chain variable region, 82 refers to position 82A according to the Kabat scheme. | | | |

Table 3. Light chain and heavy chain variable region sequences of humanized antibodies of mAb1901

| Name of variable region (SEQ ID NO:) | Sequence |
|---|---|
| VL1 (SEQ ID NO: 21) | DIVMTQSPDSLAVSLGERATINCKSSQSLLNSGNQKNYLAWYQQKPGQPPKLLIYGASTRASGVPDRFSGSGSGTDFTLTISSLQAEDVAVYYCQNDLYYPLTFGQGTKLEIK |
| VL2 (SEQ ID NO: 22) | DIVMTQSPDSLAVSLGERATISCKSSQSLLNSGNQKNYLAWYQQKPGQPPKLLIYGASTRASGVPDRFSGSGSGTDFTLTISSLQAEDVAVYYCQNDLYYPLTFGQGTKLEIK |
| VL3 (SEQ ID NO: 23) | DIVMTQSPDSLAVSLGERATISCKSSQSLLNSGNQKNYLAWYQQKPGQPPKLLIYGASTRASGVPDRFSGSGSGTDFTLTISSLQAEDVAIYHCQNDLYYPLTFGQGTKLEIK |
| VH1 (SEQ ID NO: 24) | EVQLVESGGGLVQPGGSLRLSCAASGFTFSDYGIHWVRQAPGKGLEWVAYISRGSSTIYYADTVKGRFTISRDNAKNSLYLQMNSLRAEDTAVYYCARGGYDTRNAMDYWGQGTTVTVSS |
| VH2 (SEQ ID NO: 25) | EVQLVESGGGLVQPGGSLRLSCAASGFTFSDYGIHWVRQAPGKGLEWVAYISRGSSTIYYADTVKGRFTISRDNAKNSLYLQMTSLRAEDTAVYYCARGGYDTRNAMDYWGQGTTVTVSS |
| VH3 (SEQ ID NO: 26) | EVQLVESGGGLVQPGGSLRLSCAASGFTFSDYGIHWVRQAPGKGLEWIAYISRGSSTIYYADTVKGRFTISRDNAKNSLYLQMTSLRAEDTAVYYCARGGYDTRNAMDYWGQGTTVTVSS |
| VH4 (SEQ ID NO: 27) | EVQLVESGGGLVQPGGSLRLSCAASGFTFSDYGIHWVRQAPGKGLEWVAYISRGSSTIYYADTVKGRFTMSRDNAKNSLYLQMTSLRAEDTAVYYCARGGYDTRNAMDYWGQGTTVTVSS |

The corresponding heavy chain variable region in the table above was joined to the human IgG1 heavy chain constant region set forth in SEQ ID NO: 7 to form a heavy chain of a full-length antibody, and the light chain variable region was joined to the human κ light chain constant region set forth in SEQ ID NO: 8 to form a light chain of a full-length antibody. In other embodiments, the heavy chain variable region and the light chain variable region may also be joined to other heavy chain constant regions and light chain constant regions, respectively, to form a full-length antibody.

2.2. Humanization of mAb1902 and reverse mutation design

A suitable human antibody germline was selected to perform humanization on mAb1902 murine antibody. The CDRs of murine antibody mAb1902 were grafted into the selected humanization template to replace humanized variable regions, followed by recombination with an IgG constant region to form a complete antibody. Meanwhile, reverse mutations were introduced into the FR region in the V region of the humanized antibody. Exemplary reverse mutations and combinations thereof are as follows:

Table 4. Humanized antibodies of mAb1902 and reverse mutation design therefor*

| Light chain variable region of humanized antibodies of mAb1902 | | Heavy chain variable region of humanized antibodies of mAb1902 | |
|---|---|---|---|
| VL11 | None | VH11 | None |

(continued)

| Light chain variable region of humanized antibodies of mAb1902 | | Heavy chain variable region of humanized antibodies of mAb1902 | |
|---|---|---|---|
| VL12 | M4L | VH12 | I69L, R71L, T73K |
| VL13 | M4L, N22S | VH13 | M48I, R66K, V67A, I69L, R71L, T73K |
| | | VH14 | R38K, A40R, M48I, R66K, V67A, I69L, R71L, T73K |
| * All amino acid positions in the table are numbered according to the Kabat numbering scheme. | | | |

Table 5. Light chain and heavy chain variable region sequences of mAb1902 humanized antibody

| Name of variable region (SEQ ID NO:) | Sequence |
|---|---|
| VL11 (SEQ ID NO: 28) | DIVMTQSPDSLAVSLGERATINCKSSQSLLNSGNQKNYLTWYQQKPGQPPKLLIYWASTRESGVPDRFSGSGSGTDFTLTISSLQAEDVAVYYCQNAYTYPFTFGQGTKLEIK |
| VL12 (SEQ ID NO: 29) | DIVLTQSPDSLAVSLGERATINCKSSQSLLNSGNQKNYLTWYQQKPGQPPKLLIYWASTRESGVPDRFSGSGSGTDFTLTISSLQAEDVAVYYCQNAYTYPFTFGQGTKLEIK |
| VL13 (SEQ ID NO: 30) | DIVLTQSPDSLAVSLGERATISCKSSQSLLNSGNQKNYLTWYQQKPGQPPKLLIYWASTRESGVPDRFSGSGSGTDFTLTISSLQAEDVAVYYCQNAYTYPFTFGQGTKLEIK |
| VH11 (SEQ ID NO: 31) | EVQLVQSGAEVKKPGASVKVSCKASGYTFTSYWMHWVRQAPGQRLEWMGMIHPNSGSTNYNEKFKGRVTITRDTSASTAYMELSSLRSEDTAVYYCARLKTGNSFDYWGQGTTVTVSS |
| VH12 (SEQ ID NO: 32) | EVQLVQSGAEVKKPGASVKVSCKASGYTFTSYWMHWVRQAPGQRLEWMGMIHPNSGSTNYNEKFKGRVTLTLDKSASTAYMELSSLRSEDTAVYYCARLKTGNSFDYWGQGTTVTVSS |
| VH13 (SEQ ID NO: 33) | EVQLVQSGAEVKKPGASVKVSCKASGYTFTSYWMHWVRQAPGQRLEWIGMIHPNSGSTNYNEKFKGKATLTLDKSASTAYMELSSLRSEDTAVYYCARLKTGNSFDYWGQGTTVTVSS |
| VH14 (SEQ ID NO: 34) | EVQLVQSGAEVKKPGASVKVSCKASGYTFTSYWMHWVKQRPGQRLEWIGMIHPNSGSTNYNEKFKGKATLTLDKSASTAYMELSSLRSEDTAVYYCARLKTGNSFDYWGQGTTVTVSS |

The corresponding heavy chain variable region in the table above was joined to the human IgG1 heavy chain constant region set forth in SEQ ID NO: 7 to form a heavy chain of a full-length antibody, and the light chain variable region was joined to the human κ light chain constant region set forth in SEQ ID NO: 8 to form a light chain of a full-length antibody.

Chimeric antibody ch1901

Heavy chain of ch1901: (SEQ ID NO: 35)

EVQLMESGGGLVKPGGSLKLSCAASGFTFSDYGIHWVRQAPEMGLEWIAYISR
GSSTIYYADTVKGRFTMSRDNAKNTLFLQMTSLRSEDTAMYYCARGGYDTRN
AMDYWGQGTSVTVSSASTKGPSVFPLAPSSKSTSGGTAALGCLVKDYFPEPVTV
SWNSGALTSGVHTFPAVLQSSGLYSLSSVVTVPSSSLGTQTYICNVNHKPSNTKV
DKKVEPKSCDKTHTCPPCPAPELLGGPSVFLFPPKPKDTLMISRTPEVTCVVVDV
SHEDPEVKFNWYVDGVEVHNAKTKPREEQYNSTYRVVSVLTVLHQDWLNGKE
YKCKVSNKALPAPIEKTISKAKGQPREPQVYTLPPSRDELTKNQVSLTCLVKGFY
PSDIAVEWESNGQPENNYKTTPPVLDSDGSFFLYSKLTVDKSRWQQGNVFSCSV
MHEALHNHYTQKSLSLSPGK;

Light chain of ch1901: (SEQ ID NO: 36)

DIVMTQSPSSLSVSAGEKVTMSCKSSQSLLNSGNQKNYLAWYQQKPGQPPKLLI
YGASTRASGVPDRFTGSGSGTDFTLTISSVQAEDLAIYHCQNDLYYPLTFGAGTK
LELKRTVAAPSVFIFPPSDEQLKSGTASVVCLLNNFYPREAKVQWKVDNALQSG
NSQESVTEQDSKDSTYSLSSTLTLSKADYEKHKVYACEVTHQGLSSPVTKSFNR
GEC;

Chimeric antibody ch1902

Heavy chain of ch1902 (SEQ ID NO: 37)

EVQLQESGAELVKPGASVKLSCKASGYIFTSYWMHWVKQRPGQGLEWIGMIHP
NSGSTNYNEKFKGKATLTLDKSSSTAYMQLSSLPSEDSAVYYCARLKTGNSFDY
WGQGTTLTVSSASTKGPSVFPLAPSSKSTSGGTAALGCLVKDYFPEPVTVSWNS
GALTSGVHTFPAVLQSSGLYSLSSVVTVPSSSLGTQTYICNVNHKPSNTKVDKKV
EPKSCDKTHTCPPCPAPELLGGPSVFLFPPKPKDTLMISRTPEVTCVVVDVSHED
PEVKFNWYVDGVEVHNAKTKPREEQYNSTYRVVSVLTVLHQDWLNGKEYKC
KVSNKALPAPIEKTISKAKGQPREPQVYTLPPSRDELTKNQVSLTCLVKGFYPSDI

AVEWESNGQPENNYKTTPPVLDSDGSFFLYSKLTVDKSRWQQGNVFSCSVMHE
ALHNHYTQKSLSLSPGK;

Light chain of ch1902 (SEQ ID NO: 38)

DIVLTQSPSSLTVTAGEKVTMSCKSSQSLLNSGNQKNYLTWYQQKPGQPPKLLI YWASTRESGVPDRFTGSGSGTDFTLTISSVQAEDLAIYYCQNAYTYPFTFGSGTK LEIKRTVAAPSVFIFPPSDEQLKSGTASVVCLLNNFYPREAKVQWKVDNALQSG NSQESVTEQDSKDSTYSLSSTLTLSKADYEKHKVYACEVTHQGLSSPVTKSFNR GEC.

Table 6 shows the humanized antibodies of mAb1901:

Table 6. Humanized antibodies of mAb1901

| Light and heavy chains | H1 | H2 | H3 | H4 |
|---|---|---|---|---|
| L1 | h1901-1 | h1901-2 | h1901-3 | h1901-4 |
| L2 | h1901-5 | h1901-6 | h1901-7 | h1901-8 |
| L3 | h1901-9 | h1901-10 | h1901-11 | h1901-12 |
| Note: In the table, the humanized antibody h1901-1 has the heavy chain H1 and the light chain L1. This applies to other humanized antibodies. The full-length antibody light chain and heavy chain sequences of the humanized antibodies of mAb1901 are shown in Table 7 below: | | | | |

Table 7. Light chain and heavy chain sequences of humanized antibodies of mAb1901

| Name of variable region (SEQ ID NO:) | Sequence |
|---|---|
| L1 (SEQ ID NO: 39) | DIVMTQSPDSLAVSLGERATINCKSSQSLLNSGNQKNYLAWYQ QKPGQPPKLLIYGASTRASGVPDRFSGSGSGTDFTLTISSLQAE DVAVYYCQNDLYYPLTFGQGTKLEIKRTVAAPSVFIFPPSDEQL KSGTASVVCLLNNFYPREAKVQWKVDNALQSGNSQESVTEQ DSKDSTYSLSSTLTLSKADYEKHKVYACEVTHQGLSSPVTKSF NRGEC |
| L2 (SEQ ID NO: 40) | DIVMTQSPDSLAVSLGERATISCKSSQSLLNSGNQKNYLAWYQ QKPGQPPKLLIYGASTRASGVPDRFSGSGSGTDFTLTISSLQAE DVAVYYCQNDLYYPLTFGQGTKLEIKRTVAAPSVFIFPPSDEQL KSGTASVVCLLNNFYPREAKVQWKVDNALQSGNSQESVTEQ DSKDSTYSLSSTLTLSKADYEKHKVYACEVTHQGLSSPVTKSF NRGEC |
| L3 (SEQ ID NO: 41) | DIVMTQSPDSLAVSLGERATISCKSSQSLLNSGNQKNYLAWYQ QKPGQPPKLLIYGASTRASGVPDRFSGSGSGTDFTLTISSLQAE DVAIYHCQNDLYYPLTFGQGTKLEIKRTVAAPSVFIFPPSDEQL KSGTASVVCLLNNFYPREAKVQWKVDNALQSGNSQESVTEQ DSKDSTYSLSSTLTLSKADYEKHKVYACEVTHQGLSSPVTKSF NRGEC |

(continued)

| Name of variable region (SEQ ID NO:) | Sequence |
|---|---|
| H1 (SEQ ID NO: 42) | EVQLVESGGGLVQPGGSLRLSCAASGFTFSDYGIHWVRQAPGKGLEWVAYISRGSSTIYYADTVKGRFTISRDNAKNSLYLQMNSLRAEDTAVYYCARGGYDTRNAMDYWGQGTTVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSRDELTKNQVSLTCLVKGFYPSDIAVEWESNGQPENNYKTTPPVLDSDGSFFLYSKLTVDKSRWQQGNVFSCSVMHEALHNHYTQKSLSLSPGK |
| H2 (SEQ ID NO: 43) | EVQLVESGGGLVQPGGSLRLSCAASGFTFSDYGIHWVRQAPGKGLEWVAYISRGSSTIYYADTVKGRFTISRDNAKNSLYLQMTSLRAEDTAVYYCARGGYDTRNAMDYWGQGTTVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSRDELTKNQVSLTCLVKGFYPSDIAVEWESNGQPENNYKTTPPVLDSDGSFFLYSKLTVDKSRWQQGNVFSCSVMHEALHNHYTQKSLSLSPGK |
| H3 (SEQ ID NO: 44) | EVQLVESGGGLVQPGGSLRLSCAASGFTFSDYGIHWVRQAPGKGLEWIAYISRGSSTIYYADTVKGRFTISRDNAKNSLYLQMTSLRAEDTAVYYCARGGYDTRNAMDYWGQGTTVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSRDELTKNQVSLTCLVKGFYPSDIAVEWESNGQPENNYKTTPPVLDSDGSFFLYSKLTVDKSRWQQGNVFSCSVMHEALHNHYTQKSLSLSPGK |

(continued)

| Name of variable region (SEQ ID NO:) | Sequence |
|---|---|
| H4 (SEQ ID NO: 45) | EVQLVESGGGLVQPGGSLRLSCAASGFTFSDYGIHWVRQAPG KGLEWVAYISRGSSTIYYADTVKGRFTMSRDNAKNSLYLQMT SLRAEDTAVYYCARGGYDTRNAMDYWGQGTTVTVSSASTKG PSVFPLAPSSKSTSGGTAALGCLVKDYFPEPVTVSWNSGALTS GVHTFPAVLQSSGLYSLSSVVTVPSSSLGTQTYICNVNHKPSNT KVDKKVEPKSCDKTHTCPPCPAPELLGGPSVFLFPPKPKDTLM ISRTPEVTCVVVDVSHEDPEVKFNWYVDGVEVHNAKTKPREE QYNSTYRVVSVLTVLHQDWLNGKEYKCKVSNKALPAPIEKTI SKAKGQPREPQVYTLPPSRDELTKNQVSLTCLVKGFYPSDIAV EWESNGQPENNYKTTPPVLDSDGSFFLYSKLTVDKSRWQQGN VFSCSVMHEALHNHYTQKSLSLSPGK |

Table 8 shows the humanized antibodies of mAb1902:

Table 8. Humanized antibodies of mAb1902

| Light and heavy chains | H11 | H12 | H13 | H14 |
|---|---|---|---|---|
| L11 | h1902-1 | h1902-2 | h1902-3 | hl902-4 |
| L12 | h1902-5 | h1902-6 | h1902-7 | h1902-8 |
| L13 | h1902-9 | h1902-10 | h1902-11 | h1902-12 |
| Note: In the table, the humanized antibody h1902-1 has the heavy chain H11 and the light chain L11. This applies to other humanized antibodies. | | | | |

The light chain and heavy chain sequences of the humanized antibodies of mAb1902 are shown in Table 9 below:

Table 9. Light chain and heavy chain sequences of humanized antibodies of mAb1901

| Name of variable region (SEQ ID NO:) | Sequence |
|---|---|
| L11 (SEQ ID NO: 46) | DIVMTQSPDSLAVSLGERATINCKSSQSLLNSGNQKNYLTWY QQKPGQPPKLLIYWASTRESGVPDRFSGSGSGTDFTLTISSLQ AEDVAVYYCQNAYTYPFTFGQGTKLEIKRTVAAPSVFIFPPSD EQLKSGTASVVCLLNNFYPREAKVQWKVDNALQSGNSQES VTEQDSKDSTYSLSSTLTLSKADYEKHKVYACEVTHQGLSSP VTKSFNRGEC |
| L12 (SEQ ID NO: 47) | DIVLTQSPDSLAVSLGERATINCKSSQSLLNSGNQKNYLTWY QQKPGQPPKLLIYWASTRESGVPDRFSGSGSGTDFTLTISSLQ AEDVAVYYCQNAYTYPFTFGQGTKLEIKRTVAAPSVFIFPPSD EQLKSGTASVVCLLNNFYPREAKVQWKVDNALQSGNSQES VTEQDSKDSTYSLSSTLTLSKADYEKHKVYACEVTHQGLSSP VTKSFNRGEC |

(continued)

| Name of variable region (SEQ ID NO:) | Sequence |
|---|---|
| L13 (SEQ ID NO: 48) | DIVLTQSPDSLAVSLGERATISCKSSQSLLNSGNQKNYLTWYQ QKPGQPPKLLIYWASTRESGVPDRFSGSGSGTDFTLTISSLQA EDVAVYYCQNAYTYPFTFGQGTKLEIKRTVAAPSVFIFPPSDE QLKSGTASVVCLLNNFYPREAKVQWKVDNALQSGNSQESV TEQDSKDSTYSLSSTLTLSKADYEKHKVYACEVTHQGLSSPV TKSFNRGEC |
| H11 (SEQ ID NO: 49) | EVQLVQSGAEVKKPGASVKVSCKASGYTFTSYWMHWVRQ APGQRLEWMGMIHPNSGSTNYNEKFKGRVTITRDTSASTAY MELSSLRSEDTAVYYCARLKTGNSFDYWGQGTTVTVSSAST KGPSVFPLAPSSKSTSGGTAALGCLVKDYFPEPVTVSWNSGA LTSGVHTFPAVLQSSGLYSLSSVVTVPSSSLGTQTYICNVNHK PSNTKVDKKVEPKSCDKTHTCPPCPAPELLGGPSVFLFPPKPK DTLMISRTPEVTCVVVDVSHEDPEVKFNWYVDGVEVHNAK TKPREEQYNSTYRVVSVLTVLHQDWLNGKEYKCKVSNKAL PAPIEKTISKAKGQPREPQVYTLPPSRDELTKNQVSLTCLVKG FYPSDIAVEWESNGQPENNYKTTPPVLDSDGSFFLYSKLTVD KSRWQQGNVFSCSVMHEALHNHYTQKSLSLSPGK |
| H12 (SEQ ID NO: 50) | EVQLVQSGAEVKKPGASVKVSCKASGYTFTSYWMHWVRQ APGQRLEWMGMIHPNSGSTNYNEKFKGRVTLTLDKSASTAY MELSSLRSEDTAVYYCARLKTGNSFDYWGQGTTVTVSSAST KGPSVFPLAPSSKSTSGGTAALGCLVKDYFPEPVTVSWNSGA LTSGVHTFPAVLQSSGLYSLSSVVTVPSSSLGTQTYICNVNHK PSNTKVDKKVEPKSCDKTHTCPPCPAPELLGGPSVFLFPPKPK DTLMISRTPEVTCVVVDVSHEDPEVKFNWYVDGVEVHNAK TKPREEQYNSTYRVVSVLTVLHQDWLNGKEYKCKVSNKAL PAPIEKTISKAKGQPREPQVYTLPPSRDELTKNQVSLTCLVKG FYPSDIAVEWESNGQPENNYKTTPPVLDSDGSFFLYSKLTVD KSRWQQGNVFSCSVMHEALHNHYTQKSLSLSPGK |

(continued)

| Name of variable region (SEQ ID NO:) | Sequence |
|---|---|
| H13 (SEQ ID NO: 51) | EVQLVQSGAEVKKPGASVKVSCKASGYTFTSYWMHWVRQ APGQRLEWIGMIHPNSGSTNYNEKFKGKATLTLDKSASTAY MELSSLRSEDTAVYYCARLKTGNSFDYWGQGTTVTVSSAST KGPSVFPLAPSSKSTSGGTAALGCLVKDYFPEPVTVSWNSGA LTSGVHTFPAVLQSSGLYSLSSVVTVPSSSLGTQTYICNVNHK PSNTKVDKKVEPKSCDKTHTCPPCPAPELLGGPSVFLFPPKPK DTLMISRTPEVTCVVVDVSHEDPEVKFNWYVDGVEVHNAK TKPREEQYNSTYRVVSVLTVLHQDWLNGKEYKCKVSNKAL PAPIEKTISKAKGQPREPQVYTLPPSRDELTKNQVSLTCLVKG FYPSDIAVEWESNGQPENNYKTTPPVLDSDGSFFLYSKLTVD KSRWQQGNVFSCSVMHEALHNHYTQKSLSLSPGK |
| H14 (SEQ ID NO: 52) | EVQLVQSGAEVKKPGASVKVSCKASGYTFTSYWMHWVKQ RPGQRLEWIGMIHPNSGSTNYNEKFKGKATLTLDKSASTAY MELSSLRSEDTAVYYCARLKTGNSFDYWGQGTTVTVSSAST KGPSVFPLAPSSKSTSGGTAALGCLVKDYFPEPVTVSWNSGA LTSGVHTFPAVLQSSGLYSLSSVVTVPSSSLGTQTYICNVNHK PSNTKVDKKVEPKSCDKTHTCPPCPAPELLGGPSVFLFPPKPK DTLMISRTPEVTCVVVDVSHEDPEVKFNWYVDGVEVHNAK TKPREEQYNSTYRVVSVLTVLHQDWLNGKEYKCKVSNKAL PAPIEKTISKAKGQPREPQVYTLPPSRDELTKNQVSLTCLVKG FYPSDIAVEWESNGQPENNYKTTPPVLDSDGSFFLYSKLTVD KSRWQQGNVFSCSVMHEALHNHYTQKSLSLSPGK |

A positive control antibody of the present disclosure is IMAB-362 (from WO2016166122)
Heavy chain of IMAB-362 (SEQ ID NO: 53):

1 QVQLQQPGAE LVRPGASVKL SCKASGYTFT SYWINWVKQR PGQGLEWIGN
51 IYPSDSYTNY NQKFKDKATL TVDKSSSTAY MQLSSPTSED SAVYYCTRSW
101 RGNSFDYWGQ GTTLTVSSAS TKGPSVFPLA PSSKSTSGGT AALGCLVKDY
151 FPEPVTVSWN SGALTSGVHT FPAVLQSSGL YSLSSVVTVP SSSLGTQTYI
201 CNVNHKPSNT KVDKRVEPKS CDKTHTCPPC PAPELLGGPS VFLFPPKPKD
251 TLMISRTPEV TCVVVDVSHE DPEVKFNWYV DGVEVHNAKT KPREEQYNST
301 YRVVSVLTVL HQDWLNGKEY KCKVSNKALP APIEKTISKA KGQPREPQVY
351 TLPPSREEMT KNQVSLTCLV KGFYPSDIAV EWESNGQPEN NYKTTPPVLD
401 SDGSFFLYSK LTVDKSRWQQ GNVFSCSVMH EALHNHYTQK SLSLSPGK;

Heavy chain of IMAB-362 (SEQ ID NO: 54):

1 DIVMTQSPSS LTVTAGEKVT MSCKSSQSLL NSGNQKNYLT WYQQKPGQPP
51 KLLIYWASTR ESGVPDRFTG SGSGTDFTLT ISSVQAEDLA VYYCQNDYSY
101 PFTFGSGTKL EIKRTVAAPS VFIFPPSDEQ LKSGTASVVC LLNNFYPREA
151 KVQWKVDNAL QSGNSQESVT EQDSKDSTYS LSSTLTLSKA DYEKHKVYAC
201 EVTHQGLSSP VTKSFNRGEC.

The above antibodies were cloned, expressed and purified using conventional gene cloning and recombinant expression methods.

## 2. Preparation of Compounds

Experimental procedures without conditions specified in the examples of the present disclosure, are generally conducted according to conventional conditions, or according to conditions recommended by the manufacturer of the starting materials or commercial products. Reagents without specific origins indicated are commercially available conventional reagents.

The structures of the compounds were determined by nuclear magnetic resonance (NMR) or mass spectrometry (MS). NMR spectra were measured using a Bruker AVANCE-400 nuclear magnetic resonance instrument, with deuterated dimethyl sulfoxide (*DMSO-d6*), deuterated chloroform ($CDCl_3$) and deuterated methanol ($CD_3OD$) as solvents, and tetramethylsilane (TMS) as internal standard. Chemical shifts are given in unit of $10^{-6}$ (ppm).

MS analysis was performed using a FINNIGAN LCQAd (ESI) mass spectrometer (manufacturer: Thermo, model: Finnigan LCQ advantage MAX).

UPLC analysis was performed using a Waters Acquity UPLC SQD liquid chromatography-mass spectrometry system.

HPLC analysis was performed using an Agilent 1200DAD high pressure liquid chromatograph (Sunfire C18 150 × 4.6 mm chromatography column) and a Waters 2695-2996 high pressure liquid chromatograph (Gimini C18 150 × 4.6 mm chromatography column).

UV-HPLC analysis was performed using a Thermo nanodrop2000 ultraviolet spectrophotometer.

Proliferation inhibition rates and $IC_{50}$ values were measured using a PHERA starFS microplate reader (BMG, Germany).

Huanghai HSGF254 or Qingdao GF254 silica gel plates of specifications 0.15 mm to 0.2 mm were adopted for thin layer chromatography (TLC) analysis and 0.4 mm to 0.5 mm for TLC separation and purification.

Yantai Yellow Sea silica gel of 200-300 mesh is generally used as a carrier in column chromatography.

Known starting materials of the present disclosure may be synthesized using or according to methods known in the art, or may be purchased from ABCR GmbH & Co.KG, Acros Organnics, Aldrich Chemical Company, Accela ChemBio Inc, Chembee Chemicals, etc.

In the examples, the reactions were all performed in an argon atmosphere or a nitrogen atmosphere unless otherwise specified.

The argon atmosphere or nitrogen atmosphere means that the reaction flask is connected to a balloon containing about 1 L of argon or nitrogen.

A hydrogen atmosphere means that the reaction flask is connected to a balloon containing about 1 L of hydrogen.

Parr 3916EKX hydrogenator, Qinglan QL-500 hydrogenator or HC2-SS hydrogenator was used in pressurized hydrogenation reactions.

The hydrogenation reaction usually involves 3 cycles of vacuumization and hydrogen purge.

A CEM Discover-S 908860 microwave reactor was used in microwave reactions.

In the examples, the solution in the reaction refers to an aqueous solution unless otherwise stated.

In the examples, the reaction temperature is room temperature unless otherwise stated. The room temperature is the optimum reaction temperature, which ranges from 20 °C to 30 °C.

Preparation of PBS buffer at pH 6.5 in examples: 8.5 g of $KH_2PO_4$, 8.56 g of $K_2HPO_4.3H_2O$, 5.85 g of NaCl, and 1.5 g of EDTA were added to a flask, and the volume was brought to 2 L. The additions were all ultrasonically dissolved, and the solution was well mixed by shaking to give the desired buffer.

The eluent system for column chromatography and the developing solvent system for thin layer chromatography used for compound purification include: A: dichloromethane and isopropanol system, B: dichloromethane and methanol system, and C: petroleum ether and ethyl acetate system. The volume ratio of solvents was adjusted according to the polarity of the compound, or by adding a small amount of triethylamine and acidic or basic reagent.

Some of the compounds of the present disclosure are characterized by Q-TOF LC/MS. Q-TOF LC/MS analysis used an Agilent 6530 accurate-mass quadrupole time-of-flight mass spectrometer and an Agilent 1290-Infinity ultra-high performance liquid chromatograph (Agilent Poroshell 300SB-C8 5 μm, 2.1 × 75 mm chromatography column).

See PCT/CN2019/107873 for the Y-D drug portion of the antibody-drug conjugates of the present disclosure, and the synthesis and tests of relevant compounds are incorporated herein by reference. Non-limiting examples of synthesis are incorporated by reference as follows:

**Example 1**

*N*-((1*S*,9*S*)-9-ethyl-5-fluoro-9-hydroxy-4-methyl-10,13-dioxo-2,3,9,10,13,15-hexahydro -1*H,*12*H*-benzo[*de*]pyrano[3',4':6,7]indolizino[1,2-*b*]quinolin-1-yl)-1-hydroxycycloprop ane-1-carboxamide **1**

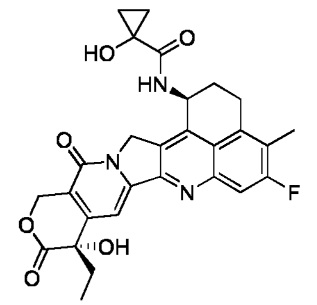

1

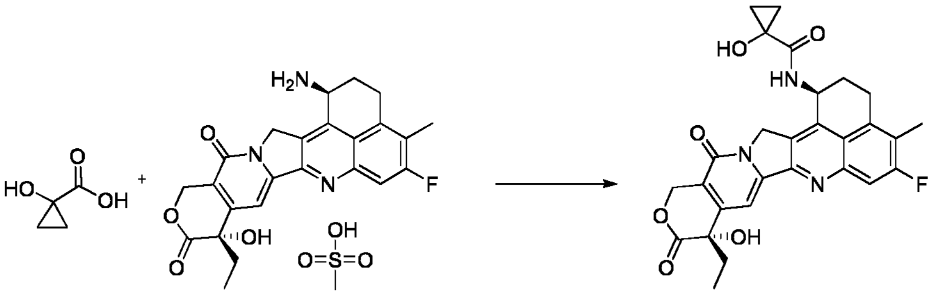

1a 1b 1

To exatecan mesylate 1b (2.0 mg, 3.76 μmol, prepared as disclosed in Patent Application "EP0737686A1") was added 1 mL of *N,N*-dimethylformamide. The mixture was cooled to 0-5 °C in an ice-water bath, and a drop of triethylamine was added. The reaction was stirred until it became clear. To the reaction mixture were successively added 1-hydroxycyclopropylcarboxylic acid 1a (1.4 mg, 3.7 μmol, prepared using known method "Tetrahedron Letters, 25(12), 1269-72; 1984") and 4-(4,6-dimethoxy-1,3,5-triazin-2-yl)-4-methylmorpholinium chloride (3.8 mg, 13.7 μmol). After addition, the reaction mixture was stirred at 0-5 °C for 2 h, quenched with 5 mL of water, and extracted with ethyl acetate (8 mL × 3). The organic phases were combined, washed with saturated sodium chloride solution (5 mL × 2), dried over anhydrous sodium sulfate, and filtered. The filtrate was concentrated under reduced pressure, and the resulting residue was purified by thin layer chromatography with developing solvent system B to give the title product 1 (1.6 mg, 82.1% yield).

MS m/z (ESI): 520.2 [M+1]

$^1$H NMR (400 MHz, $CDCl_3$): δ 7.90-7.84 (m, 1H), 7.80-7.68(m, 1H), 5.80-5.70 (m, 1H), 5.62-5.54(m, 2H), 5.44-5.32 (m, 2H), 5.28-5.10(m, 2H), 3.40-3.15 (m, 3H), 2.44 (s, 3H), 2.23(t, 1H), 2.06-1.75 (m, 2H), 1.68-1.56 (m, 1H), 1.22-1.18 (m, 2H), 1.04-0.98 (m, 2H), 0.89 (t, 3H).

**Example 2**

(*S*)-2-cyclopropyl-*N*-((1*S*,9*S*)-9-ethyl-5-fluoro-9-hydroxy-4-methyl-10,13-dioxo-2,3,9,1 0,13,15-hexahydro-1*H*,12*H*-benzo[*de*]pyrano[3',4':6,7]indolizino[1,2-*b*]quinolin-1-yl)-2 -hydroxyacetamide **2-A**

(*R*)-2-cyclopropyl-*N*-((1*S*,9*S*)-9-ethyl-5-fluoro-9-hydroxy-4-methyl-10,13-dioxo-2,3,9,1 0,13,15-hexahydro-1*H*,12*H*-benzo[*de*]pyrano[3',4':6,7]indolizino[1,2-*b*]quinolin-1-yl)-2 -hydroxyacetamide **2-B**

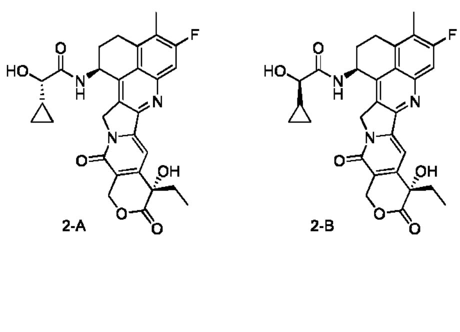

2-A 2-B

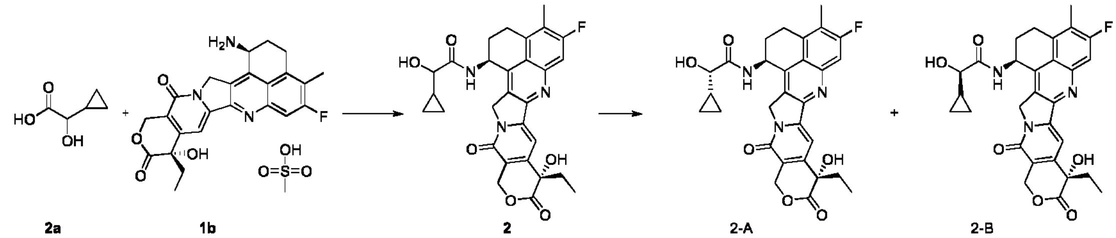

2a 1b 2 2-A 2-B

To **1b** (4 mg, 7.53 μmol) were added 2 mL of ethanol and 0.4 mL of *N,N*-dimethylformamide. The system was purged with argon three times, and the mixture was cooled to 0-5 °C in an ice-water bath, followed by dropwise addition of 0.3 mL of *N*-methylmorpholine. The reaction mixture was stirred until it became clear. To the reaction mixture were successively added 2-cyclopropyl-2-hydroxyacetic acid **2a** (2.3 mg, 19.8 μmol, prepared as disclosed in Patent Application "WO2013106717"), 1-hydroxybenzotriazole (3 mg, 22.4 μmol) and 1-(3-dimethylaminopropyl)-3-ethylcarbodiimide hydrochloride (4.3 mg, 22.4 μmol). After addition, the reaction mixture was stirred at 0-5 °C for 1 h. The ice-water bath was removed, and the reaction mixture was heated to 30 °C, stirred for 2 h, and concentrated under reduced pressure. The resulting crude compound **2** was purified by high performance liquid chromatography (separation conditions: chromatography column: XBridge Prep C18 OBD 5 μm 19 × 250 mm; mobile phase: A-water (10 mmol of $NH_4OAc$), B-acetonitrile, gradient elution, flow rate: 18 mL/min), and the corresponding fractions were collected and concentrated under reduced pressure to give the title product (2-A: 1.5 mg, 2-B: 1.5 mg).

MS m/z (ESI): 534.0 [M+1].

Single-configuration compound 2-B (shorter retention time)

UPLC analysis: retention time: 1.06 min; purity: 88% (chromatography column: ACQUITY UPLC BEHC18 1.7 μm 2.1 × 50 mm; mobile phase: A-water (5 mmol of $NH_4OAc$), B-acetonitrile).

$^1$H NMR (400 MHz, DMSO-$d_6$): δ 8.37 (d, 1H), 7.76 (d, 1H), 7.30 (s, 1H), 6.51 (s, 1H), 5.58-5.56 (m, 1H), 5.48 (d, 1H), 5.41 (s, 2H), 5.32-5.29 (m, 2H), 3.60 (t, 1H), 3.19-3.13 (m, 1H), 2.38 (s, 3H), 2.20-2.14 (m, 1H), 1.98 (q, 2H), 1.87-1.83 (m, 1H), 1.50-1.40 (m, 1H), 1.34-1.28 (m, 1H), 0.86 (t, 3H), 0.50-0.39 (m, 4H).

Single-configuration compound 2-A (longer retention time)

CPLC analysis: retention time: 1.10 min; purity: 86% (chromatography column: ACQUITY UPLC BEHC18 1.7 μm 2.1 × 50 mm; mobile phase: A-water (5 mmol of $NH_4OAc$), B-acetonitrile).

$^1$H NMR (400 MHz, DMSO-$d_6$): δ 8.35 (d, 1H), 7.78 (d, 1H), 7.31 (s, 1H), 6.52 (s, 1H), 5.58-5.53 (m, 1H), 5.42 (s, 2H), 5.37 (d, 1H), 5.32 (t, 1H), 3.62 (t, 1H), 3.20-3.15 (m, 2H), 2.40 (s, 3H), 2.25-2.16 (m, 1H), 1.98 (q, 2H), 1.87-1.82 (m, 1H), 1.50-1.40 (m, 1H), 1.21-1.14 (m, 1H), 0.87 (t, 3H), 0.47-0.35 (m, 4H).

## Example 3

(*S*)-*N*-((1*S*,9*S*)-9-ethyl-5-fluoro-9-hydroxy-4-methyl-10,13-dioxo-2,3,9,10,13,15-hexah ydro-1*H*,12*H*-benzo[*de*]pyrano[3',4':6,7]indolizino[1,2-*b*]quinolin-1-yl)-3,3,3-trifluoro-2-hydroxypropionamide **3-A**

(*R*)-*N*-((1*S*,9*S*)-9-ethyl-5-fluoro-9-hydroxy-4-methyl-10,13-dioxo-2,3,9,10,13,15-hexah ydro-1*H*,12*H*-benzo[*de*]pyrano[3',4':6,7]indolizino[1,2-*b*]quinolin-1-yl)-3,3,3-trifluoro-2-hydroxypropionamide **3-B**

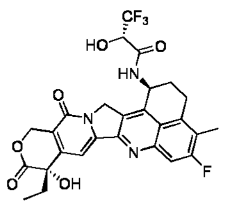

3-A

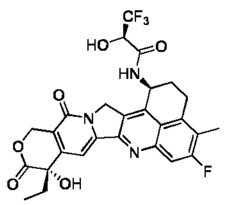

3-B

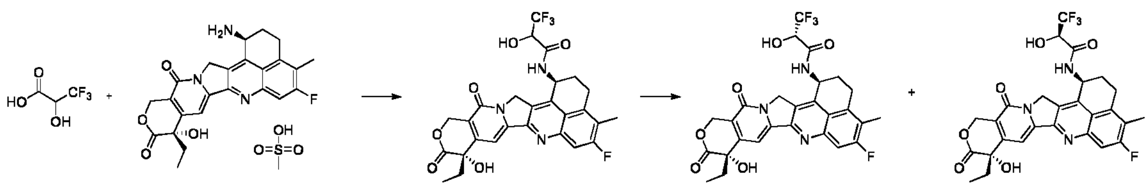

3a 1b 3 3-A 3-B

To **1b** (5.0 mg, 9.41 μmol) were added 2 mL of ethanol and 0.4 mL of *N,N*-dimethylformamide, and the mixture was cooled to 0-5 °C in an ice-water bath, followed by dropwise addition of 0.3 mL of *N*-methylmorpholine. The reaction mixture was stirred until it became clear. To the reaction mixture were successively added 3,3,3-trifluoro-2-hydroxypropionic acid **3a** (4.1 mg, 28.4 μmol, supplied by Alfa), 1-hydroxybenzotriazole (3.8 mg, 28.1 μmol) and 1-(3-dimethylaminopropyl)-3-ethylcarbodiimide hydrochloride (5.4 mg, 28.2 μmol). After addition, the reaction mixture was stirred at 0-5 °C for 10 min. The ice-water bath was removed, and the reaction mixture was heated to 30 °C, stirred for 8 h, and concentrated under reduced pressure. The resulting crude compound **3** was purified by high performance liquid chromatography (separation conditions: chromatography column: XBridge Prep C18 OBD 5 μm 19 × 250 mm; mobile phase: A-water (10 mmol of $NH_4OAc$), B-acetonitrile, gradient elution, flow rate: 18 mL/min), and the corresponding fractions were collected and concentrated under reduced pressure to give the title product (3-A: 1.5 mg, 3-B: 1.5 mg).

MS m/z (ESI): 561.9 [M+1] .

Single-configuration compound (shorter retention time)

UPLC analysis: retention time: 1.11 min; purity: 88% (chromatography column: ACQUITY UPLC BEHC18 1.7 μm 2.1 × 50 mm; mobile phase: A-water (5 mmol of $NH_4OAc$), B-acetonitrile).

$^1H$ NMR (400 MHz, DMSO-$d_6$): δ 8.94 (d, 1H), 7.80 (d, 1H), 7.32 (s, 1H), 7.20 (d, 1H), 6.53 (s, 1H), 5.61-5.55 (m, 1H), 5.45-5.23 (m, 3H), 5.15-5.06 (m, 1H), 4.66-4.57 (m, 1H), 3.18-3.12 (m, 1H), 2.40 (s, 3H), 2.26-2.20 (m, 1H), 2.16-2.08 (m, 1H), 2.02-1.94 (m, 1H), 1.89-1.82 (m, 1H), 1.50-1.40 (m, 1H), 0.87 (t, 3H).

Single-configuration compound (longer retention time)

UPLC analysis: retention time: 1.19 min; purity: 90% (chromatography column: ACQUITY UPLC BEHC18 1.7 μm 2.1 × 50 mm; mobile phase: A-water (5 mmol of $NH_4OAc$), B-acetonitrile).

$^1H$ NMR (400 MHz, DMSO-$d_6$): δ 8.97 (d, 1H), 7.80 (d, 1H), 7.31 (s, 1H), 7.16 (d, 1H), 6.53 (s, 1H), 5.63-5.55 (m, 1H), 5.45-5.20 (m, 3H), 5.16-5.07 (m, 1H), 4.66-4.57 (m, 1H), 3.18-3.12 (m, 1H), 2.40 (s, 3H), 2.22-2.14 (m, 1H), 2.04-1.95 (m, 2H), 1.89-1.82 (m, 1H), 1.50-1.40 (m, 1H), 0.87 (t, 3H).

## Example 4

*N*-((1*S*,9*S*)-9-ethyl-5-fluoro-9-hydroxy-4-methyl-10,13-dioxo-2,3,9,10,13,15-hexahydro -1*H,*12*H*-benzo[*de*]pyrano[3',4':6,7]indolizino[1,2-*b]*quinolin-1-yl)-1-hydroxycyclopent ane-1-carboxamide 4

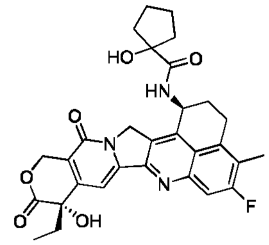

4

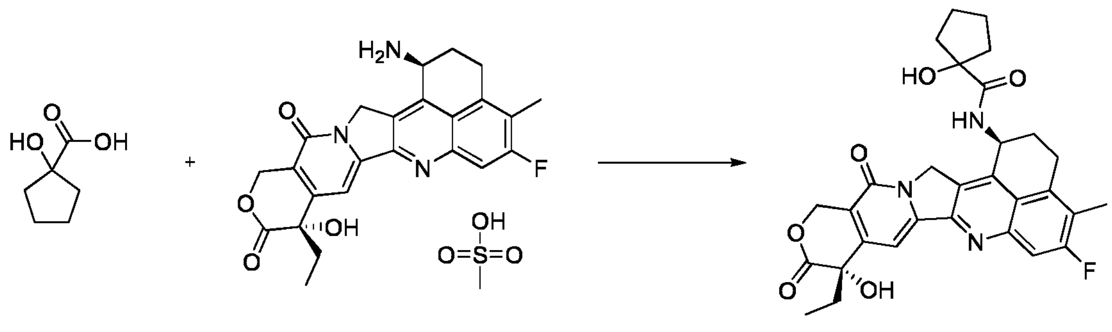

4a 1b 4

To **1b** (3.0 mg, 5.64 μmol) was added 1 mL of *N,N*-dimethylformamide. The mixture was cooled to 0-5 °C in an ice-water bath, and a drop of triethylamine was added. The reaction mixture was stirred until it became clear. To the reaction mixture were successively added 1-hydroxy-cyclopentanecarboxylic acid **4a** (2.2 mg, 16.9 μmol, prepared as disclosed in Patent Application "WO2013106717") and 4-(4,6-dimethoxy-1,3,5-triazin-2-yl)-4-methylmorpholinium chloride (4.7 mg, 16.9 μmol). After addition, the reaction mixture was stirred at 0-5 °C for 1 h, quenched with 5 mL of water,

and extracted with ethyl acetate (10 mL × 3). The organic phases were combined, washed with saturated sodium chloride solution (5 mL × 2), dried over anhydrous sodium sulfate, and filtered. The filtrate was concentrated under reduced pressure, and the resulting residue was purified by thin layer chromatography with developing solvent system B to give the title product **4** (2.5 mg, 80.9% yield).

MS m/z (ESI): 548.0 [M+1] .

$^1$H NMR (400 MHz, $CDCl_3$): δ 7.73-7.62 (m, 2H), 5.75-5.62 (m, 1H), 5.46-5.32 (m, 2H), 5.26-5.10 (m, 1H), 3.30-3.10 (m, 1H), 2.43 (s, 3H), 2.28-2.20 (m, 2H), 2.08-1.84 (m, 8H), 1.69-1.58 (m, 2H), 1.04-1.00 (m, 2H), 0.89 (t, 3H).

## Example 5

*N*-((1*S*,9*S*)-9-ethyl-5-fluoro-9-hydroxy-4-methyl-10,13-dioxo-2,3,9,10,13,15-hexa hydro-1*H*,12*H*-benzo[*de*]pyrano[3',4':6,7]indolizino[1,2-*b*]quinolin-1-yl)-1-(hydroxyme thyl)cyclopropane-1-carboxamide **5**

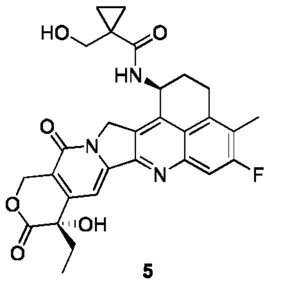

**5**

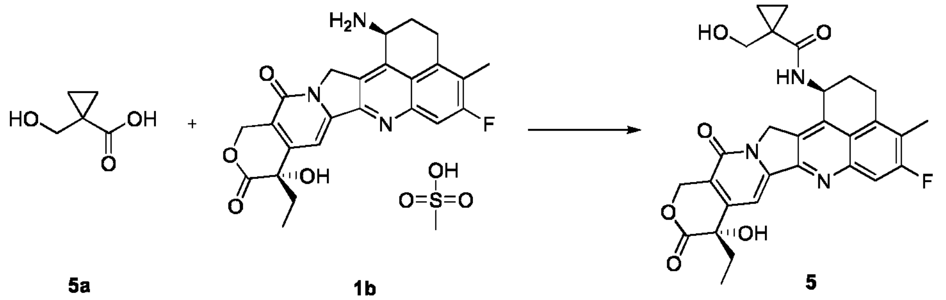

**5a** **1b** **5**

To **1b** (2.0 mg, 3.76 μmol) was added 1 mL of *N,N*-dimethylformamide. The mixture was cooled to 0-5 °C in an ice-water bath, and a drop of triethylamine was added. The reaction mixture was stirred until it became clear. To the reaction mixture were successively added 1-(hydroxymethyl)-cyclopentanecarboxylic acid **5a** (0.87 mg, 7.5 μmol, prepared as disclosed in Patent Application "WO201396771") and 4-(4,6-dimethoxy-1,3,5-triazin-2-yl)-4-methylmorpholinium chloride (2 mg, 7.24 μmol). After addition, the reaction mixture was stirred at 0-5 °C for 2 h, quenched with 5 mL of water, and extracted with ethyl acetate (8 mL × 3). The organic phases were combined, washed with saturated sodium chloride solution (5 mL × 2), dried over anhydrous sodium sulfate, and filtered. The filtrate was concentrated under reduced pressure, and the resulting residue was purified by thin layer chromatography with developing solvent system B to give the title product **5** (1.0 mg, 50% yield).

MS m/z (ESI): 533.9 [M+1] .

$^1$H NMR (400 MHz, $CDCl_3$): δ 8.07 (s, 1H), 7.23-7.18 (m, 2H), 6.71-6.64 (m, 1H), 6.55-6.51 (m, 1H), 5.36-5.27 (m, 2H), 4.67-4.61 (m, 2H), 3.53-3.48 (m, 1H), 3.30-3.22 (m, 2H), 3.18-3.13 (m, 1H), 2.71-2.61 (m, 2H), 2.35-2.28 (m, 1H), 2.04-1.91 (m, 4H), 1.53-1.40 (m, 3H), 0.91-0.75 (m, 4H).

## Example 6

*N*-((1*S*,9*S*)-9-ethyl-5-fluoro-9-hydroxy-4-methyl-10,13-dioxo-2,3,9,10,13,15-hexahydro -1*H*,12*H*-benzo[*de*]pyrano[3',4':6,7]indolizino[1,2-*b*]quinolin-1-yl)-1-(hydroxymethyl)c yclobutane-1-carboxamide **6**

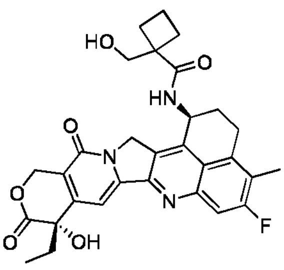

**6**

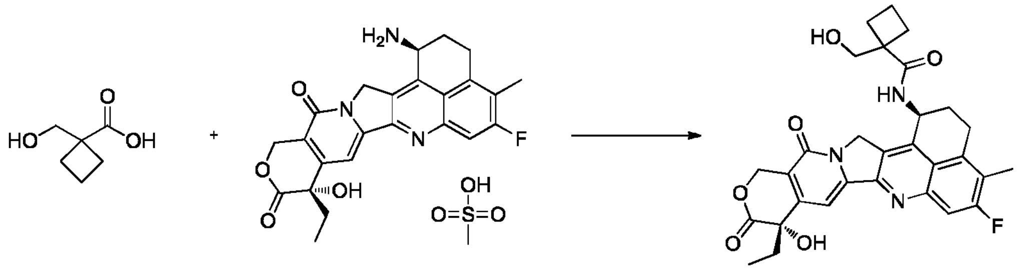

**6a** **1b** **6**

To **1b** (3.0 mg, 5.64 μmol) was added 1 mL of *N,N*-dimethylformamide. The mixture was cooled to 0-5 °C in an ice-water bath, and a drop of triethylamine was added. The reaction mixture was stirred until it became clear. To the reaction mixture were successively added 1-(hydroxymethyl)cyclobutane-1-carboxylic acid **6a** (2.2 mg, 16.9 μmol, prepared as disclosed in "Journal of the American Chemical Society, 2014, vol.136, #22, p.8138-8142") and 4-(4,6-dimethoxy-1,3,5-triazin-2-yl)-4-methylmorpholinium chloride (4.7 mg, 16.9 μmol). After addition, the reaction mixture was stirred at 0-5 °C for 1 h, quenched with 5 mL of water, and extracted with ethyl acetate (10 mL × 3). The organic phases were combined, washed with saturated sodium chloride solution (5 mL × 2), dried over anhydrous sodium sulfate, and filtered. The filtrate was concentrated under reduced pressure, and the resulting residue was purified by thin layer chromatography with developing solvent system B to give the title product **6** (2.1 mg, 67.9% yield).

MS m/z (ESI): 548.0 [M+1].

$^1$H NMR (400 MHz, DMSO-$d_6$): δ 7.85-7.62 (m, 1H), 6.88 (br, 1H), 5.87-5.48 (m, 2H), 5.47-5.33 (m, 1H), 5.31-5.06 (m, 1H), 4.25-3.91 (m, 2H), 3.25 (br, 1H), 2.60-2.32 (m, 3H), 2.23 (t, 1H), 2.15-1.95 (m, 3H), 1.70-1.56 (m, 2H), 1.41-1.17 (m, 9H), 1.03 (s, 1H), 0.95-0.80 (m, 2H).

## Example 7

*N*-((1*S*,9*S*)-9-ethyl-5-fluoro-9-hydroxy-4-methyl-10,13-dioxo-2,3,9,10,13,15-hexahydro -1*H*,12*H*-benzo[*de*]pyrano[3',4':6,7]indolizino[1,2-*b*]quinolin-1-yl)-1-hydroxycyclobuta ne-1-carboxamide **7**

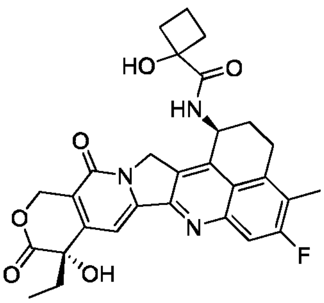

**7**

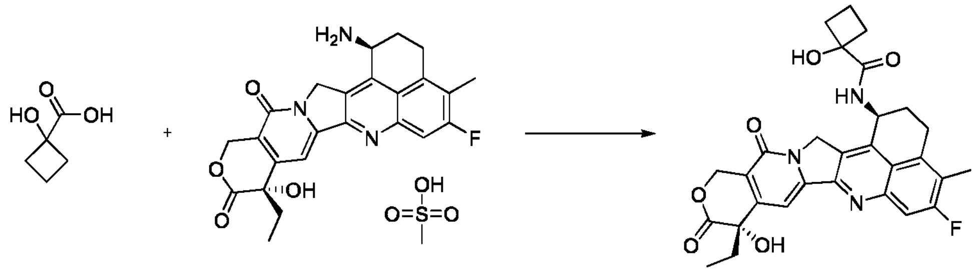

**7a** **1b** **7**

To **1b** (3.0 mg, 5.64 μmol) were added 2 mL of ethanol and 0.4 mL of *N,N*-dimethylformamide, and the mixture was cooled to 0-5 °C in an ice-water bath, followed by dropwise addition of 0.3 mL of *N*-methylmorpholine. The reaction mixture was stirred until it became clear. To the reaction mixture were successively added 1-hydroxycyclobutanecarboxylic acid **7a** (2.0 mg, 17.22 μmol, supplied by PharmaBlock), 1-hydroxybenzotriazole (2.3 mg, 17.0 μmol) and 1-(3-dimethylaminopropyl)-3-ethylcarbodiimide hydrochloride (3.2 mg, 16.7 μmol). After addition, the reaction mixture was stirred at 0-5 °C for 10 min. The ice-water bath was removed, and the reaction mixture was stirred at room temperature for 2 h and concentrated under reduced pressure. The resulting residue was purified by thin layer chromatography with developing solvent system B to give the title product **7** (2.5 mg, 83.1% yield).

MS m/z (ESI): 534.0 [M+1].

$^1$H NMR (400 MHz, DMSO-$d_6$): δ 8.28 (d, 1H), 7.75 (d, 1H), 7.29 (s, 1H), 6.51 (s, 1H), 6.12 (s, 1H), 5.59-5.51 (m, 1H), 5.41 (s, 2H), 5.20-5.01 (m, 2H), 3.27-3.17 (m, 1H), 3.15-3.05 (m, 1H), 2.71-2.63 (m, 1H), 2.37 (s, 3H), 2.12-2.05 (m, 1H), 2.03-1.94 (m, 2H), 1.92-1.78 (m, 4H), 1.50-1.42 (m, 1H), 0.90-0.83 (m, 4H).

## Example 8

1-((((*S*)-7-benzyl-20-(2,5-dioxo-2,5-dihydro-1*H*-pyrrol-1-yl)-3,6,9,12,15-pentaoxo-2,5,8, 11,14-pentaazaeicosyl)oxy)-*N*-((1*S*,9*S*)-9-ethyl-5-fluoro-9-hydroxy-4-methyl-10,13-dio xo-2,3,9,10,13,15-hexahydro-1*H*,12*H*-benzo[*de*]pyrano[3',4':6,7]indolizino[1,2-*b*]quino lin-1-yl)cyclopropane-1-carboxamide **8**

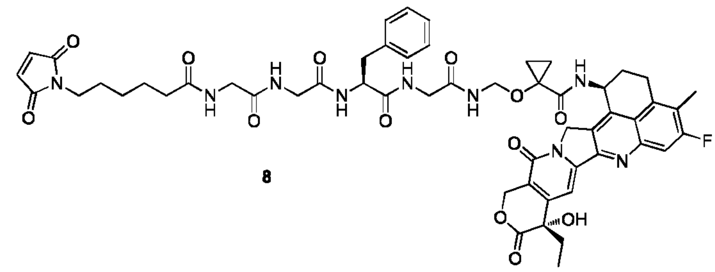

8

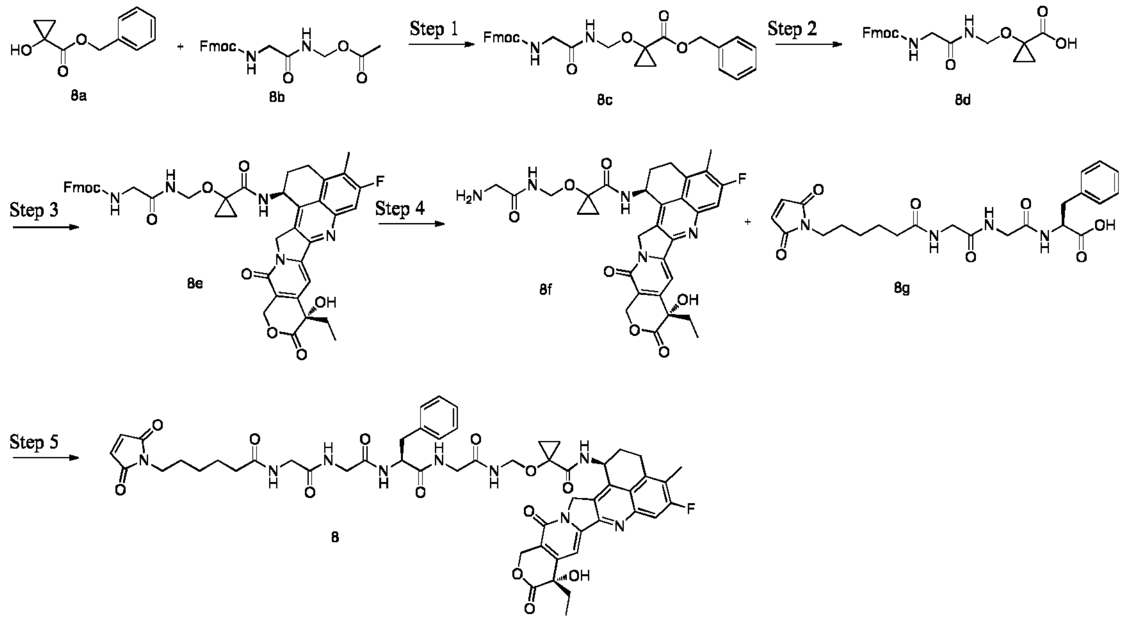


8a 8b 8c 8d 8e 8f 8g 8

Step 1

Benzyl 1-((2-((((9*H*-fluoren-9-yl)methoxy)carbonyl)amino)acetamido)methoxy)cyclopropane-1 -carboxylate **8c**

Benzyl 1-hydroxycyclopropane-1-carboxylate **8a** (104 mg, 0.54 mmol; prepared as disclosed in Patent Application "US2005/20645") and 2-((((9*H*-fluoren-9-yl)methoxy)carbonyl)amino)acetamido)methyl acetate **8b** (100 mg, 0.27 mmol; prepared as disclosed in Patent Application "CN105829346A") were added to a reaction flask, and 5 mL of tetrahydrofuran was added. The system was purged with argon three times, and the mixture was cooled to 0-5 °C in an ice-water bath, followed by addition of potassium *tert*-butoxide (61 mg, 0.54 mmol). The ice bath was removed, and the reaction mixture was warmed to room temperature and stirred for 10 min, followed by addition of 20 mL of ice water and by extraction with ethyl acetate (5 mL × 2) and chloroform (5 mL × 5). The organic phases were combined and concentrated. The resulting residue was dissolved in 3 mL of 1,4-dioxane, followed by addition of 0.6 mL of water, sodium bicarbonate (27 mg, 0.32 mmol) and 9-fluorenylmethyl chloroformate (70 mg, 0.27 mmol). The mixture was stirred at room temperature for 1 h. 20 mL of water was added, followed by extraction with ethyl acetate (8 mL × 3). The organic phase was washed with saturated sodium chloride solution (20 mL), dried over anhydrous sodium sulfate, and filtered. The filtrate was concentrated under reduced pressure, and the resulting residue was purified by silica gel column chromatography with developing solvent system B to give the title product **8c** (100 mg, 73.6% yield). MS m/z (ESI): 501.0 [M+1].

Step 2

1-((2-((((9*H*-fluoren-9-yl)methoxy)carbonyl)amino)acetamido)methoxy)cyclopropane-1 -carboxylicacid **8d**

**8c** (50 mg, 0.10 mmol) was dissolved in 3 mL of a solvent mixture of tetrahydrofuran and ethyl acetate (V:V = 2:1), and palladium on carbon (25 mg, 10% loading) was added. The system was purged with hydrogen three times, and the reaction mixture was stirred at room temperature for 1 h. The reaction mixture was filtered through celite, and the filter cake was rinsed with tetrahydrofuran. The filtrate was concentrated to give the title product **8d** (41 mg, 100% yield).

MS m/z (ESI): 411.0 [M+1].

Step 3

(9*H*-fluoren-9-yl)methyl(2-(((1-(((1*S*,9*S*)-9-ethyl-5-fluoro-9-hydroxy-4-methyl-10,13-di oxo-2,3,9,10,13,15-hexahydro-1*H*,12*H*-benzo[*de*]pyrano[3',4':6,7]indolizino[1,2-*b*]quin olin-1-yl)aminocarbonyl)cyclopropoxy)methyl)amino)-2-oxoethyl)carbamate **8e**

**1b** (7 mg, 0.013 mmol) was added to a reaction flask, and 1 mL of *N*,*N*-dimethylformamide was added. The system was purged with argon three times, and the mixture was cooled to 0-5 °C in an ice-water bath, followed by addition of a drop of triethylamine, a solution of **8d** (7 mg, 0.017 mmol) in 0.5 mL of *N*,*N*-dimethylformamide, and 4-(4,6-dimethoxy-1,3,5-triazin-2-yl)-4-methylmorpholinium chloride (7 mg, 0.026 mmol). The reaction mixture was stirred in an ice bath for 35 min. 10 mL of water was added, followed by extraction with ethyl acetate (5 mL × 3). The organic phase was washed with saturated sodium chloride solution (10 mL), dried over anhydrous sodium sulfate, and filtered. The filtrate was concentrated under reduced pressure, and the resulting residue was purified by thin layer chromatography with developing solvent system B to give the title product **8e** (8.5 mg, 78.0% yield).

MS m/z (ESI): 828.0 [M+1].

Step 4

1-((2-Aminoacetylamino)methoxy)-*N*-((1*S*,9*S*)-9-ethyl-5-fluoro-9-hydroxy-4-methyl-10 ,13-dioxo-2,3,9,10,13,15-hexahydro-1*H*,12*H*-benzo[*de*]pyrano[3',4':6,7]indolizino[1,2-*b* ]quinolin-1-yl)cyclopropane-1-carboxamide **8f**

**8e** (4 mg, 4.84 μmol) was dissolved in 0.2 mL of dichloromethane, and 0.1 mL of diethylamine was added. The reaction mixture was stirred at room temperature for 2 h and concentrated under reduced pressure. 2 mL of toluene was added, folowed by concentration under reduced pressure; the procedures were repeated twice. The residue was slurried with 3 mL of *n*-hexane, and the upper *n*-hexane layer was removed; the procedures were repeated three times. The slurry was concentrated under reduced pressure to give the crude title product **8f** (2.9 mg), which was directly used in the next step without purification.

MS m/z (ESI): 606.0 [M+1].

Step 5

1-(((*S*)-7-benzyl-20-(2,5-dioxo-2,5-dihydro-1*H*-pyrrol-1-yl)-3,6,9,12,15-pentaoxo-2,5,8, 11,14-pentaazaeicosyl)oxy)-*N*-((1*S*,9*S*)-9-ethyl-5-fluoro-9-hydroxy-4-methyl-10,13-dio xo-2,3,9,10,13,15-hexahydro-1*H*,12*H*-benzo[*de*]pyrano[3',4':6,7]indolizino[1,2-*b*]quino lin-1-yl)cyclopropane-1-carboxamide **8**

Crude **8f** (2.9 mg, 4.84 μmol) was dissolved in 0.5 mL of *N*,*N*-dimethylformamide. The system was purged with argon three times, and the solution was cooled to 0-5 °C in an ice-water bath. A solution of (*S*)-2(-2-(-2-(6-(2,5-dioxo-1*H*-pyrrol-1-yl) hexanamido)acetylamino)acetylamino)-3-phenylpropionic acid **8g** (2.7 mg, 5.80 μmol, prepared as disclosed in Patent Application "EP2907824") in 0.3 mL of *N*,*N*-dimethylformamide was added, followed by addition of 4-(4,6-dimethoxy-1,3,5-triazin-2-yl)-4-methylmorpholinium chloride (2.7 mg, 9.67 μmol). The reaction mixture was stirred in an ice bath for 30 min. Then, the ice bath was removed, and the reaction mixture was warmed to room temperature, stirred for 15 min, and purified by high performance liquid chromatography (separation conditions: chromatography column: XBridge Prep C18 OBD 5 μm 19 × 250 mm; mobile phase: A-water (10 mmol of $NH_4OAc$), B-acetonitrile, gradient elution, flow rate: 18 mL/min). The corresponding fractions were collected and concentrated under reduced pressure to give the title product 8 (2 mg, 39.0% yield).

MS m/z (ESI): 1060.0 [M+1].

$^1$H NMR (400 MHz, DMSO-$d_6$): δ 9.01 (d, 1H), 8.77 (t, 1H), 8.21 (t, 1H), 8.08-7.92 (m, 2H), 7.73 (d, 1H), 7.28 (s, 1H), 7.24-7.07 (m, 4H), 6.98 (s, 1H), 6.50 (s, 1H), 5.61 (q, 1H), 5.40 (s, 2H), 5.32 (t, 1H), 5.12 (q, 2H), 4.62 (t, 1H), 4.52 (t, 1H), 4.40-4.32 (m, 1H), 3.73-3.47 (m, 8H), 3.16-3.04 (m, 2H), 2.89 (dd, 1H), 2.69-2.55 (m, 2H), 2.37-2.23 (m, 4H), 2.12-1.93 (m, 4H), 1.90-1.74 (m, 2H), 1.52-1.38 (m, 4H), 1.33-1.11 (m, 5H), 0.91-0.81 (m, 4H).

## Example 9

*N*-((2*R*,10*S*)-10-benzyl-2-cyclopropyl-1-(((1*S*,9*S*)-9-ethyl-5-fluoro-9-hydroxy-4-methyl-10,13-dioxo-2,3,9,10,13,15-hexahydro-1*H*,12*H*-benzo[*de*]pyrano[3',4':6,7]indolizino[1, 2-*b*]quinolin-1-yl)amino)-1,6,9,12,15-pentaoxo-3-oxa-5,8,11,14-tetraazahexadecan-16-y l)-6-(2,5-dioxo-2,5-dihydro-1*H*-pyrrol-1-yl)hexanamide **9-A**

*N*-((2*S*,10*S*)-10-benzyl-2-cyclopropyl-1-(((1*S*,9*S*)-9-ethyl-5-fluoro-9-hydroxy-4-methyl-10,13-dioxo-2,3,9,10,13,15-hexahydro-1*H*,12*H*-benzo[*de*]pyrano[3',4':6,7]indolizino[1, 2-*b*]quinolin-1-yl)amino)-1,6,9,12,15-pentaoxo-3-oxa-5,8,11,14-tetraazahexadecan-16-y l)-6-(2,5-dioxo-2,5-dihydro-1*H*-pyrrol-1-yl)hexanamide **9-B**

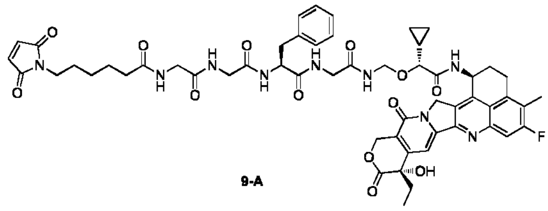
9-A

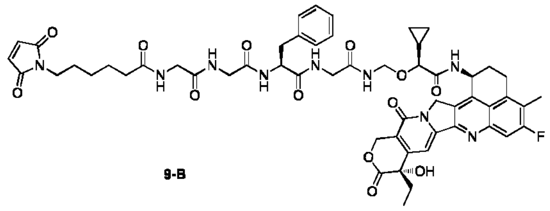
9-B

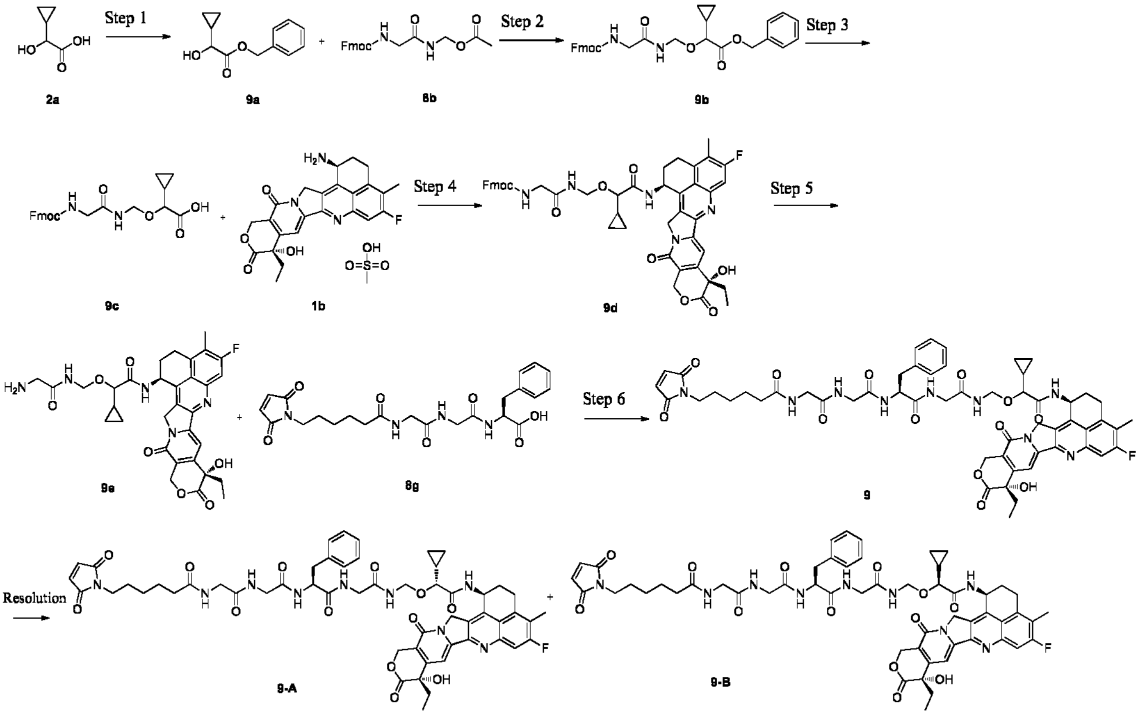

2a 9a 8b 9b 9c 1b 9d 9e 8g 9 9-A 9-B

Step 1

Benzyl 2-cyclopropyl-2-hydroxyacetate **9a**

**2a** (1.3 g, 11.2 mmol; prepared as disclosed in Patent Application "WO2013/106717") was dissolved in 50 mL of acetonitrile, and potassium carbonate (6.18 g, 44.8 mmol), benzyl bromide (1.33 mL, 11.2 mmol) and tetrabutylammonium iodide (413 mg, 1.1 mmol) were successively added. The reaction mixture was stirred at room temperature for 48 h and filtered through celite, and the filter cake was rinsed with ethyl acetate (10 mL). The filtrates were combined and concentrated under reduced pressure, and the resulting residue was purified by silica gel column chromatography with developing solvent system C to give the title product **9a** (2 g, 86.9% yield).

Step 2

Benzyl

10-cyclopropyl-1-(9*H*-fluoren-9-yl)-3,6-dioxo-2,9-dioxa-4,7-diazaundecan-11-oate **9b 9a** (120.9 mg, 0.586 mmol) and **8b** (180 mg, 0.489 mmol) were added to a reaction flask, and 4 mL of tetrahydrofuran was added. The system was purged with argon three times, and the reaction mixture was cooled to 0-5 °C in an ice-water bath, followed by addition of potassium *tert*-butoxide (109 mg, 0.98 mmol). The ice bath was removed, and the reaction mixture was warmed to room temperature and stirred for 40 min, followed by addition of 10 mL of ice water and by extraction with ethyl acetate (20 mL × 2) and chloroform (10 mL × 5). The organic phases were combined and concentrated. The resulting residue was dissolved in 4 mL of dioxane, and 2 mL of water, sodium bicarbonate (49.2 mg, 0.586 mmol) and 9-fluorenylmethyl chloroformate (126 mg, 0.49 mmol) were added. The mixture was stirred at room temperature for 2 h. 20 mL of water was added, followed by extraction with ethyl acetate (10 mL × 3). The organic phase was washed with saturated sodium chloride solution (20 mL), dried over anhydrous sodium sulfate, and filtered. The filtrate was concentrated under reduced pressure. The resulting residue was purified by silica gel column chromatography with developing solvent system C to give the title product **9b** (48 mg, 19% yield).
MS m/z (ESI): 515.0 [M+1].

Step 3

10-Cyclopropyl-1-(9*H*-fluoren-9-yl)-3,6-dioxo-2,9-dioxa-4,7-diazaundecan-11-oic acid **9c**

**9b** (20 mg, 0.038 mmol) was dissolved in 4.5 mL of a solvent mixture of tetrahydrofuran and ethyl acetate (V:V = 2:1), and palladium on carbon (12 mg, 10% loading, dry basis) was added. The system was purged with hydrogen three times, and the reaction mixture was stirred at room temperature for 1 h. The reaction mixture was filtered through celite, and the filter cake was rinsed with ethyl acetate. The filtrate was concentrated to give the crude title product **9c** (13 mg), which was directly used in the next step without purification.
MS m/z (ESI): 424.9 [M+1].

Step 4

(9*H*-fluoren-9-yl)methyl(2-(((1-cyclopropyl-2-(((1*S*,9*S*)-9-ethyl-5-fluoro-9-hydroxy-4-methyl-10,13-dioxo-2,3,9,10,13,15-hexahydro-1*H*,12*H*-benzo[*de*]pyrano[3',4':6,7]indoli zino[1,2-*b*]quinolin-1-yl)amino)-2-oxoethoxy)methyl)amino)-2-oxoethyl)carbamate **9d 1b** (10 mg, 18.8 μmol) was added to a reaction flask, and 1 mL of *N,N*-dimethylformamide was added. The system was purged with argon three times, and the mixture was cooled to 0-5 °C in an ice-water bath, followed by addition of a drop of triethylamine, crude **9c** (13 mg, 30.6 μmol), and 4-(4,6-dimethoxy-1,3,5-triazin-2-yl)-4-methylmorpholinium chloride (16.9 mg, 61.2 μmol). The reaction mixture was stirred in an ice bath for 40 min. 10 mL of water was added, followed by extraction with ethyl acetate (10 mL × 3). The organic phases were combined, washed with saturated sodium chloride solution (10 mL × 2), dried over anhydrous sodium sulfate, and filtered. The filtrate was concentrated under reduced pressure. The resulting residue was purified by thin layer chromatography with developing solvent system B to give the title product **9d** (19 mg, 73.6% yield).
MS m/z (ESI): 842.1 [M+1].

Step 5

2-((2-Aminoacetamido)methoxy)-2-cyclopropyl-*N*-((1*S*,9*S*)-9-ethyl-5-fluoro-9-hydroxy -4-methyl-10,13-dioxo-2,3,9,10,13,15-hexahydro-1*H*,12*H*-benzo[*de*]pyrano[3',4':6,7]in dolizino[1,2-*b*]quinolin-1-yl)acetamide **9e**

**9d** (19 mg, 22.6 μmol) was dissolved in 2 mL of dichloromethane, and 1 mL of diethylamine was added. The reaction mixture was stirred at room temperature for 2 h and concentrated under reduced pressure. 1 mL of toluene was added, followed by concentration under reduced pressure; the procedures were repeated twice. The residue was slurried with 3 mL of *n*-hexane and let stand. Then, the supernatant was removed, and the solid was kept. The solid residue was concentrated under reduced pressure and dried using an oil pump to give the crude title product **9e** (17 mg), which was directly used in the next step without purification.
MS m/z (ESI): 638.0 [M+18].

Step 6

*N*-((2*R*,10*S*)-10-benzyl-2-cyclopropyl-1-(((1*S*,9*S*)-9-ethyl-5-fluoro-9-hydroxy-4-methyl-10,13-dioxo-2,3,9,10,13,15-hexahydro-1*H*,12*H*-benzo[*de*]pyrano[3',4':6,7]indolizino[1, 2-*b*]quinolin-1-yl)amino)-1,6,9,12,15-pentaoxo-3-oxa-5,8,11,14-tetraazahexadecan-16-y l)-6-(2,5-dioxo-2,5-dihydro-1*H*-pyrrol-1-yl)hexanamide **9-A**

*N*-((2*S*,10*S*)-10-benzyl-2-cyclopropyl-1-(((1*S*,9*S*)-9-ethyl-5-fluoro-9-hydroxy-4-methyl-10,13-dioxo-2,3,9,10,13,15-hexahydro-1*H*,12*H*-benzo[*de*]pyrano[3',4':6,7]indolizino[1, 2-*b*]quinolin-1-yl)amino)-1,6,9,12,15-pentaoxo-3-oxa-5,8,11,14-tetraazahexadecan-16-y l)-6-(2,5-dioxo-2,5-dihydro-1*H*-pyrrol-1-yl)hexanamide **9-B**

Crude **9e** (13.9 mg, 22.4 μmol) was dissolved in 0.6 mL of *N*,*N*-dimethylformamide. The system was purged with argon three times, and the solution was cooled to 0-5 °C in an ice-water bath. A solution of **8g** (21.2 mg, 44.8 μmol) in 0.3 mL of *N*,*N*-dimethylformamide was added, followed by addition of 4-(4,6-dimethoxy-1,3,5-triazin-2-yl)-4-methylmorpholinium chloride (18.5 mg, 67.3 μmol). The reaction mixture was stirred in an ice bath for 10 min. Then, the ice bath was removed, and the reaction mixture was warmed to room temperature and stirred for 1 h to produce compound **9.** The reaction mixture was purified by high performance liquid chromatography (separation conditions: chromatography column: XBridge Prep C18 OBD 5 μm 19 × 250 mm; mobile phase: A-water (10 mmol of $NH_4OAc$), B-acetonitrile, gradient elution, flow rate: 18 mL/min). The corresponding fractions were collected and concentrated under reduced pressure to give the title products (9-A: 2.4 mg, 9-B: 1.7 mg).
MS m/z (ESI): 1074.4 [M+1].
Single-configuration compound 9-A (shorter retention time):
UPLC analysis: retention time: 1.14 min; purity: 85% (chromatography column: ACQUITY UPLC BEHC18 1.7 μm 2.1 × 50 mm; mobile phase: A-water (5 mmol of $NH_4OAc$), B-acetonitrile).
$^1$H NMR (400 MHz, DMSO-$d_6$): δ 8.60 (t, 1H), 8.51-8.49 (d, 1H), 8.32-8.24 (m, 1H), 8.13-8.02 (m, 2H), 8.02-7.96 (m, 1H), 7.82-7.75 (m, 1H), 7.31 (s, 1H), 7.26-7.15 (m, 4H), 6.99 (s, 1H), 6.55-6.48 (m, 1H), 5.65-5.54 (m, 1H), 5.41 (s, 2H), 5.35-5.15 (m, 3H), 4.74-4.62 (m, 1H), 4.54-4.40 (m, 2H), 3.76-3.64 (m, 4H), 3.62-3.48 (m, 2H), 3.20-3.07 (m, 2H), 3.04-2.94 (m, 1H), 2.80-2.62 (m, 1H), 2.45-2.30 (m, 3H), 2.25-2.15 (m, 2H), 2.15-2.04 (m, 2H), 1.93-1.78 (m, 2H), 1.52-1.39 (m, 3H), 1.34-1.12 (m, 5H), 0.87 (t, 3H), 0.64-0.38 (m, 4H).
Single-configuration compound 9-B (longer retention time):
UPLC analysis: retention time: 1.16 min; purity: 89% (chromatography column: ACQUITY UPLC BEHC18 1.7 μm 2.1 × 50 mm; mobile phase: A-water (5 mmol of $NH_4OAc$), B-acetonitrile).
$^1$H NMR (400 MHz, DMSO-$d_6$): δ 8.68-8.60 (m, 1H), 8.58-8.50 (m, 1H), 8.32-8.24 (m, 1H), 8.13-8.02 (m, 2H), 8.02-7.94 (m, 1H), 7.82-7.75 (m, 1H), 7.31 (s, 1H), 7.26-7.13 (m, 3H), 6.99 (s, 1H), 6.55-6.48 (m, 1H), 5.60-5.50 (m, 1H), 5.41 (s, 2H), 5.35-5.15 (m, 2H), 4.78-4.68 (m, 1H), 4.60-4.40 (m, 2H), 3.76-3.58 (m, 4H), 3.58-3.48 (m, 1H), 3.20-3.10 (m, 2H), 3.08-2.97 (m, 2H), 2.80-2.72 (m, 2H), 2.45-2.30 (m, 3H), 2.25-2.13 (m, 2H), 2.13-2.04 (m, 2H), 2.03-1.94 (m, 2H), 1.91-1.78 (m, 2H), 1.52-1.39 (m, 3H), 1.34-1.12 (m, 4H), 0.91-0.79 (m, 3H), 0.53-0.34 (m, 4H).

**III. Preparation of anti-claudin18.2 antibody ADC conjugates**

Drug-loading analysis of ADC stock solution

A. UV-HPLC method

The DAR value n was calculated by UV-HPLC for some ADC examples of the present disclosure, specifically as follows:

1. Determination method:
Cuvettes containing sodium succinate buffer were placed into the reference cell and sample cell, and the absorbance of the solvent blank was subtracted. Then, a cuvette containing test solution was placed into the sample cell, and the absorbances at 280 nm and 370 nm were determined.
2. Calculation for results: The loading capacity of the ADC stock solution was determined by ultraviolet spectrophotometry (instrument: Thermo nanodrop2000 ultraviolet spectrophotometer), based on the principle that the total absorbance of the ADC stock solution at a certain wavelength is the sum of the absorbances of the drug and the monoclonal antibody at that wavelength, namely:

$$(1)\ A_{280\ nm} = \varepsilon_{mab\text{-}280} bC_{mab} + \varepsilon_{Drug\text{-}280} bC_{Drug}$$

$\varepsilon_{Drug-280}$: the mean molar attenuation coefficient of the drug at 280 nm is 5100;
$C_{Drug}$: the concentration of the drug;
$\varepsilon_{mab-280}$: the mean molar attenuation coefficient of the monoclonal antibody stock solution at 280 nm is 214,600;
$C_{mab}$: the concentration of the monoclonal antibody stock solution;
b: the optical path length is 1 cm.

Similarly, an equation for the total absorbance of the sample at 370 nm can be given as:

$$(2)\ A_{370\ nm} = \varepsilon_{mab\text{-}370} b C_{mab} + \varepsilon_{Drug\text{-}370} b C_{Drug}$$

$\varepsilon_{Drug-370}$: the mean molar attenuation coefficient of the drug at 370 nm was 19000;
$C_{Drug}$: the concentration of the drug;
$\varepsilon_{mab-370}$: the attenuation coefficient of the monoclonal antibody stock solution at 370 nm is 0;
$C_{mab}$: the concentration of the monoclonal antibody stock solution;
b: the optical path length is 1 cm.

The drug loading can be calculated using both equations (1) and (2) as well as the attenuation coefficients of the monoclonal antibody and the drug at both wavelengths and their concentrations.

$$\text{Drug loading} = C_{Drug}/C_{mab}.$$

B. RP-HPLC method

The DAR value was calculated by RP-HPLC (reversed-phase high performance liquid chromatography) for some ADC examples of the present disclosure, specifically as follows:

1. Determination method:

A naked antibody (unconjugated antibody) and an ADC test sample (at concentration 1 mg/mL) were reduced with 4 μL of DDT (sigma) in a water bath at 37 °C for 1 h, and then transferred to an insert. Analysis was performed on a high performance liquid chromatograph Agilent 1200, with Agilent PLRP-S 1000A 8 μm 4.6 × 250 mm selected as the chromatography column, the column temperature at 80 °C, the DAD detector at wavelength 280 nm, the flowrate at 1 mL/min, and the injection volume at 40 μL. Comparisons were made to the spectra of the sample and the naked antibody to identify the locations of the light chain and heavy chain, and then integration was performed on the spectrum of the test sample to calculate the DAR value n.

2. Preparation of solutions

1) 0.25 M DTT solution:
Example of preparation: 5.78 mg of DTT was weighed into 150 μL of purified water and completely dissolved to give 0.25 M DTT solution, which was then stored at -20 °C.
2) Mobile phase A (0.1% TFA in water):
Example of preparation: 1000 mL of purified water was measured out using a graduated cylinder, and 1 mL of TFA (sigma) was added. The solution was well mixed before use and was stored at 2-8 °C for 14 days.
3) Mobile phase B (0.1% TFA in acetonitrile):
Example of preparation: 1000 mL of acetonitrile was measured out using a graduated cylinder, and 1 mL of TFA was added. The solution was well mixed before use and was stored at 2-8 °C for 14 days.

3. Data analysis

Comparisons were made to the spectra of the sample and the naked antibody to identify the locations of the light chain and heavy chain, and then integration was performed on the spectrum of the test sample to calculate the DAR value (n).
The calculation formula is as follows:

| Name | Number of linked drugs |
|---|---|
| LC | 0 |
| LC+1 | 2 |
| HC | 0 |
| HC+1 | 2 |
| HC+2 | 4 |
| HC+3 | 6 |

Total LC peak area = LC peak area + LC+1 peak area

Total HC peak area = HC peak area + HC+1 peak area + HC+2 peak area + HC+3 peak area

LC DAR = ∑(number of linked drugs × percent peak area)/total LC peak area

HC DAR = ∑(number of linked drugs × percent peak area)/total HC peak area

DAR = LC DAR + HC DAR.

Preparation Examples of Claudin18.2 Antibody-Drug Conjugates

**Examples 3-1 and 3-2: ADC-1 and ADC-2**

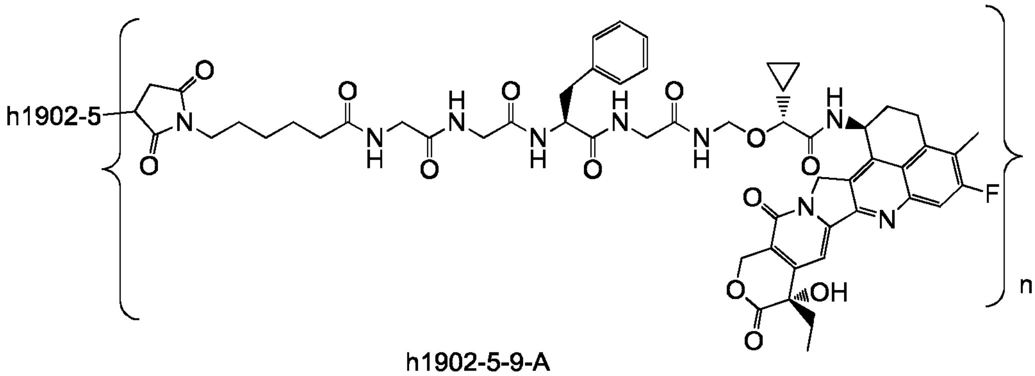

h1902-5-9-A

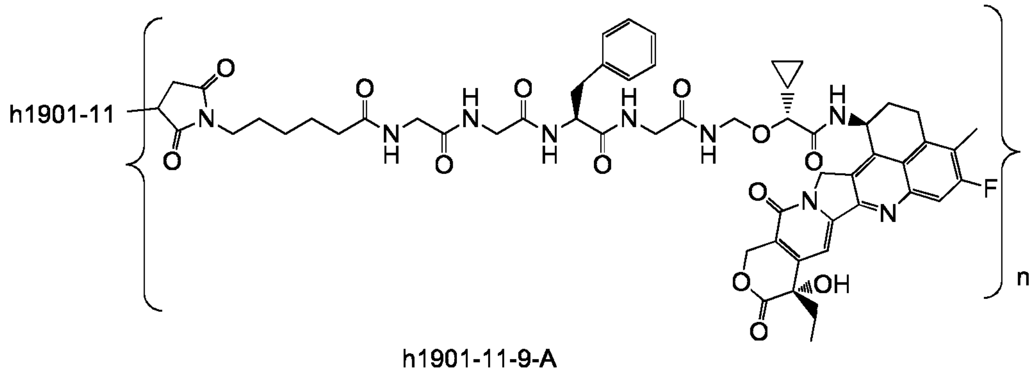

h1901-11-9-A

To a PBS buffer containing antibody h1902-5 (0.05 M aqueous PBS buffer at pH 6.5; 10.0 mg/mL, 320.0 mL, 21.62 μmol) was added at 37 °C an aqueous solution of tris(2-carboxyethyl)phosphine (TCEP) (10 mM, 11.03 mL, 110.3 μmol). The reaction mixture was shaken on a water bath shaker at 37 °C for 3 h before the reaction was terminated. The reaction mixture was cooled to 25 °C in a water bath.

Compound 9-A (350 mg, 303 μmol) was dissolved in 13.2 mL of acetonitrile and 6.6 mL of DMSO, and the resulting solution was added to the above reaction mixture that was cooled to 25 °C, which was then shaken on a water bath shaker at 25 °C for 3 h before the reaction was terminated.

The resulting reaction mixture was purified through an ultrafiltration membrane successively with 5 L of PBS

buffer (50 mM, pH = 6.5, 4% acetonitrile, 2% DMSO) and 5 L of succinic acid buffer (10 mM, pH = 5.3) to remove small molecules. Sucrose was added at concentration 60 mg/mL and tween-20 at concentration 0.2 mg/mL to give final exemplary product ADC-1 of general formula antibody-drug conjugate h1902-5-9-A (10 mM succinic acid buffer at pH 5.3; 10 mg/mL, 2.626 g). Yield: 81.81 %.

Mean calculated by UV-HPLC: n = 6.8.

Using the methods described above, exemplary product ADC-2 of general formula antibody-drug conjugate h1901-11-9-A can be prepared using compound 9-A, and antibody h1901-11 in place of h1902-5, with the DAR value n being 7.1.

**Example 3-3. ADC-3**

To an aqueous PBS buffer of antibody h1901-11 (0.05 M aqueous PBS buffer at pH 6.5; 10.0 mg/mL, 1 mL, 67.5 nmol) was added at 37 °C a prepared aqueous solution of tris(2-carboxyethyl)phosphine (TCEP) (10 mM, 10.1 μL, 101 nmol). The reaction mixture was shaken on a water bath shaker at 37 °C for 3 h before the reaction was terminated. The reaction mixture was cooled to 25 °C in a water bath.

Compound **9-A** (0.58 mg, 540 nmol) was dissolved in 34 μL of DMSO, and the resulting solution was added to the above reaction mixture, which was then shaken on a water bath shaker at 25 °C for 3 h before the reaction was terminated. The reaction mixture was desalted and purified through a Sephadex G25 gel column (elution phase: 0.05 *M* PBS buffer at pH 6.5, containing 0.001 M EDTA) to give exemplary product **ADC-3** of antibody-drug conjugate h1901-11-9-A in PBS buffer (0.72 mg/mL, 11.2 mL), which was then stored at 4 °C. Mean calculated by RP-HPLC: n = 2.51.

**Example 3-4. ADC-4**

To an aqueous PBS buffer of antibody h1901-11 (0.05 M aqueous PBS buffer at pH 6.5; 10.0 mg/mL, 1 mL, 67.5 nmol) was added at 37 °C a prepared aqueous solution of tris(2-carboxyethyl)phosphine (TCEP) (10 mM, 16.9 μL, 169 nmol). The reaction mixture was shaken on a water bath shaker at 37 °C for 3 h before the reaction was terminated. The reaction mixture was cooled to 25 °C in a water bath.

Compound **9-A** (0.73 mg, 680 nmol) was dissolved in 43 μL of DMSO, and the resulting solution was added to the above reaction mixture, which was then shaken on a water bath shaker at 25 °C for 3 h before the reaction was terminated. The reaction mixture was desalted and purified through a Sephadex G25 gel column (elution phase: 0.05 *M* PBS buffer at pH 6.5, containing 0.001 M EDTA) to give exemplary product **ADC-4** of antibody-drug conjugate h1901-11-9-A in PBS buffer (0.62 mg/mL, 12.5 mL), which was then stored at 4 °C. Mean calculated by RP-HPLC: n = 4.06.

**Example 3-5. ADC-5**

To an aqueous PBS buffer of antibody h1901-11 (0.05 M aqueous PBS buffer at pH 6.5; 10.0 mg/mL, 1 mL, 67.5 nmol) was added at 37 °C a prepared aqueous solution of tris(2-carboxyethyl)phosphine (TCEP) (10 mM, 35.8 μL, 358 nmol). The reaction mixture was shaken on a water bath shaker at 37 °C for 3 h before the reaction was terminated. The reaction mixture was cooled to 25 °C in a water bath.

Compound **9-A** (1.09 mg, 1015 nmol) was dissolved in 64 μL of DMSO, and the resulting solution was added to the above reaction mixture, which was then shaken on a water bath shaker at 25 °C for 3 h before the reaction was terminated. The reaction mixture was desalted and purified through a Sephadex G25 gel column (elution phase: 0.05 *M* PBS buffer at pH 6.5, containing 0.001 M EDTA) to give exemplary product **ADC-5** of antibody-drug conjugate h1901-11-9-A in PBS buffer (0.54 mg/mL, 12.5 mL), which was then stored at 4 °C. Mean calculated by RP-HPLC: n = 6.8.

**Example 3-6. ADC-6**

To an aqueous PBS buffer of antibody h1902-5 (0.05 M aqueous PBS buffer at pH 6.5; 10.0 mg/mL, 1.08 mL, 72.9 nmol) was added at 37 °C a prepared aqueous solution of tris(2-carboxyethyl)phosphine (TCEP) (10 mM, 10.9 μL, 109 nmol). The reaction mixture was shaken on a water bath shaker at 37 °C for 3 h before the reaction was terminated. The reaction mixture was cooled to 25 °C in a water bath.

Compound **9-A** (0.63 mg, 587 nmol) was dissolved in 40 μL of DMSO, and the resulting solution was added to the above reaction mixture, which was then shaken on a water bath shaker at 25 °C for 3 h before the reaction was terminated. The reaction mixture was desalted and purified through a Sephadex G25 gel column (elution phase: 0.05 *M* PBS buffer at pH 6.5, containing 0.001 M EDTA) to give exemplary product **ADC-6** of h1902-5-9-A in PBS buffer (0.7 mg/mL, 13.0 mL), which was then stored at 4 °C. Mean calculated by RP-HPLC: n = 2.69.

**Example 3-7. ADC-7**

To an aqueous PBS buffer of antibody hl902-5 (0.05 M aqueous PBS buffer at pH 6.5; 10.0 mg/mL, 1.08 mL, 72.9 nmol) was added at 37 °C a prepared aqueous solution of tris(2-carboxyethyl)phosphine (TCEP) (10 mM, 18.3 μL, 183 nmol). The reaction mixture was shaken on a water bath shaker at 37 °C for 3 h before the reaction was terminated. The reaction mixture was cooled to 25 °C in a water bath.

Compound **9-A** (0.79 mg, 736 nmol) was dissolved in 50 μL of DMSO, and the resulting solution was added to the above reaction mixture, which was then shaken on a water bath shaker at 25 °C for 3 h before the reaction was terminated. The reaction mixture was desalted and purified through a Sephadex G25 gel column (elution phase: 0.05 *M* PBS buffer at pH 6.5, containing 0.001 M EDTA) to give exemplary product **ADC-7** of hl902-5-9-A in PBS buffer (0.6 mg/mL, 14.0 mL), which was then stored at 4 °C. Mean calculated by RP-HPLC: n = 4.25.

**Example 3-8. ADC-8**

To an aqueous PBS buffer of antibody h1902-5 (0.05 M aqueous PBS buffer at pH 6.5; 10.0 mg/mL, 1.08 mL, 72.9 nmol) was added at 37 °C a prepared aqueous solution of tris(2-carboxyethyl)phosphine (TCEP) (10 mM, 38.7 μL, 387 nmol). The reaction mixture was shaken on a water bath shaker at 37 °C for 3 h before the reaction was terminated. The reaction mixture was cooled to 25 °C in a water bath.

Compound **9-A** (1.18 mg, 1099 nmol) was dissolved in 70 μL of DMSO, and the resulting solution was added to the above reaction mixture, which was then shaken on a water bath shaker at 25 °C for 3 h before the reaction was terminated. The reaction mixture was desalted and purified through a Sephadex G25 gel column (elution phase: 0.05 *M* PBS buffer at pH 6.5, containing 0.001 M EDTA) to give exemplary product **ADC-8** of h1902-5-9-A in PBS buffer (0.56 mg/mL, 14.2 mL), which was then stored at 4 °C. Mean calculated by RP-HPLC: n = 7.01.

**Example 3-9. ADC-9**

To histidine-acetate-Tris/EDTA buffer (10 mM histidine-acetate-Tris buffer at pH 7.2, 2.5 mM EDTA buffer; 20.6 g/L, 6.49 L, 0.91 mmol) containing antibody h1902-5 was added at 12 °C a prepared TCEP histidine buffer (10 mM histidine buffer; 1.717 mM, 1.16 L, 1.99 mmol). The reaction mixture was stirred in a water bath at 12 °C for 2 h before the reaction was terminated to give a solution of intermediate I.

Compound 9-A (4.72 g, 4.39 mmol) was dissolved in 0.38 L of DMSO to give a solution of compound 9-A in DMSO. 0.38 L of DMSO was added to the above solution of intermediate I, and then the above solution of compound 9-A in DMSO was added. The reaction mixture was stirred in a water bath at 12 °C for 1 h before the reaction was terminated.

The reaction mixture was purified through a Capto S Impact cation chromatography column, which was washed with 9 column volumes of 0.05 M acetate buffer containing 10% (v/v) DMSO (pH = 5.0) and with 6 column volumes of 0.05 M acetate buffer (pH = 5.0), followed by elution with 0.05 M acetic acid and 0.30 M sodium chloride buffer (pH = 5.5) to remove free toxins and the residual solvent from the reaction mixture. The cation eluate was subjected to 7-fold volume equal-volume ultrafiltration (polycellulose membrane of 30 KD was used as the ultrafiltration membrane) at 22 °C to give exemplary product ADC-9 of h1902-5-9-A. Mean calculated by RP-HPLC: n = 4.1. The drug loading obtained in this example is a non-limiting example, and one skilled in the art can obtain conjugates of different DAR values (1-10, preferably 1-8, and more preferably 2-8 and 2-7) by adjusting the reaction conditions and reagents.

**Biological Evaluation**

**Test Example 1. Cell-Level ELISA Binding Assay**

A Cell-based ELISA assay was used for testing the binding properties of claudin18.2 antibodies. The stably transfected claudin18.2-expressing NUGC4 cells were cultured in a 96-well cell plate. When growing at 90% density, the cells were immobilized with 4% paraformaldehyde for 1 h. The plate was washed 3 times with PBST buffer (pH 7.4 PBS containing 0.05% Tween-20), and a PBS-diluted 5% skim milk (powdered skim milk from Brightdairy) blocking buffer was added at 200 μL/well. The plate was incubated in a 37 °C incubator for 2.5 h or was let stand at 4 °C overnight (16-18 h) for blocking. After blocking, the blocking buffer was removed. The plate was washed 3 times with the PBST buffer, and then a test antibody that was diluted with a sample diluent (pH 7.4 PBS containing 1% r milk) to different concentrations was added at 50 μL/well. The plate was incubated in a 37 °C incubator for 2 h. After incubation, the plate was washed 5 times with PBST, and an HRP-labeled goat anti-human secondary antibody (Jackson Immuno Research, 109-035-003) that was diluted with the sample diluent was added at 100 μL/well. The plate was incubated at 37 °C for 1 h. The plate was washed 6 times with PBST, and then TMB chromogenic substrate (KPL, 52-00-03) was added at 50

μL/well. The plate was incubated at room temperature for 10-15 min, and the reaction was terminated by adding 1 M $H_2SO_4$ at 50 μL/well. The absorbance at 450 nm was read using an MD Versa Max TM microplate reader, and the binding $EC_{50}$ value of the claudin18.2 antibody to claudin18.2 was calculated.

Table 10. Binding activity of hybridoma antibodies

| Antibody | IMAB362 | ch1901 | ch1902 |
|---|---|---|---|
| Emax | 1.175 | 1.399 | 1.272 |
| $EC_{50}$ (nM) | 0.108 | 0.098 | 0.074 |

Table 11. Binding activity of humanized antibodies of mAb1901

| Antibody | IMAB362 | h1901-2 | h1901-3 | h1901-4 | h1901-6 |
|---|---|---|---|---|---|
| Emax | 1.115 | 1.039 | 1.1055 | 0.986 | 0.937 |
| $EC_{50}$ (nM) | 0.086 | 0.076 | 0.22 | 0.201 | 0.091 |
| Antibody | h1901-7 | h1901-8 | h1901-11 | h1901-12 | |
| Emax | 0.921 | 1.047 | 1.44 | 1.22 | |
| $EC_{50}$ (nM) | 0.166 | 0.091 | 0.076 | 0.116 | |

Table 12. Binding activity of humanized antibodies of mAb1902

| Antibody | IMAB362 | h1902-1 | h1902-2 | h1902-3 | h1902-4 | h1902-5 |
|---|---|---|---|---|---|---|
| Emax | 0.88 | 0.87 | 0.88 | 0.84 | 0.82 | 0.90 |
| $EC_{50}$ (nM) | 0.187 | 0.113 | 0.107 | 0.175 | 0.087 | 0.098 |
| Antibody | h1902-6 | h1902-7 | h1902-8 | h1902-9 | h1902-10 | |
| Emax | 0.78 | 0.75 | 0.89 | 0.75 | 0.89 | |
| $EC_{50}$ (nM) | 0.141 | 0.121 | 0.132 | 0.137 | 0.133 | |

**Test Example 2. Antibody Cell-Level Binding Assay**

The stably transfected claudin18.2-expressing NUGC4 cells were suspended in FACS buffer (2% fetal bovine serum (Gibco, 10099141) pH 7.4 PBS (Sigma, P4417-100TAB)) to give a 1 × $10^6$/mL cell suspension, which was then added to a 96-well round-bottom plate (Corning, 3795) at 100 μL/well. After centrifugation and removal of the supernatant, the test claudin18.2 antibody that was diluted with FACS buffer to different concentrations was added at 50 μL/well. The plate was incubated in the dark in a 4 °C refrigerator for 1 h. The plate was washed 3 times with FACS buffer by centrifugation at 300 g, and Alexa Fluor 488 goat anti-human IgG (H+L) (invitrogen, A-11013) at working concentration was added. The plate was incubated in the dark in a 4 °C refrigerator for 40 min. The plate was washed 3 times with FACS buffer by centrifugation at 300 g and tested on a BD FACS CantoII flow cytometer for geometric mean fluorescence intensity. The binding $EC_{50}$ value of the claudinl8.2 antibody to the stably transfected claudin18.2-expressing NUGC4 cells was calculated. The results are shown in FIG. 1.

**Test Example 3. Antibody Endocytosis Assay**

A test claudin18.2 antibody pre-labeled with DyLight 488 NHS Ester (thermofisher, 46403) was added to 1×$10^6$/mL stably transfected claudin18.2-expressing NUGC4 cells at a final concentration of 5 μg/mL. The mixture was incubated in the dark on ice for 1 h and washed 3 times with pre-cooled FACS buffer (pH 7.4 PBS, 2% fetal bovine serum) by centrifugation. After removal of the supernatant, the remainder was added to a pre-heated complete medium, followed by incubation in a 37 °C cell incubator with 5% $CO_2$. The cells were taken out after 0, 0.5, 1, 2 and 4 h and stored in the dark on ice. After all samples were collected, they are centrifuged at 300 g at low temperature and the supernatants were removed. An elution buffer (pH 1.7 0.05 M glycine, 0.1 M sodium chloride) was added, and then the mixtures were incubated at room temperature for 7 min, washed once with FACS buffer by centrifugation at 300 g, and

tested on a BD FACS CantoII flow cytometer for geometric mean fluorescence intensity. The efficiency of endocytosis of the claudin18.2 antibody by the stably transfected claudinl8.2-expressing NUGC4 cells was calculated. The results (see FIG. 2) show that the humanized antibodies have good endocytosis efficiency.

**Test Example 4. Antibody Affinity Assay Based on Flow Cytometry**

On the day of experiment, HEK293/hClaudin18.2 cells were collected into a U-bottomed 96-well plate at 1-2 $\times 10^5$ cells per well. A human claudin18.2 antibody that was 2× diluted serially (12 concentration points) from an initial concentration of 5 μg/mL was added, and the plate was incubated at 4 °C for 1 h. IMAB362 was used as a positive control, and a negative control with no antibody was also set. The antibody was removed by centrifugation, and FITC anti-human IgG Fc antibody (200×) was added at 100 μL/well. The plate was incubated in the dark at 4 °C for 30 min and washed twice with PBS + 2% FBS before flow cytometry analysis. BD FACS CantoII was started and preheated, and then the BD FACSDiva software was run to start a new experiment. The HEK293/hClaudin18.2 negative control sample was tested, and the FSC and SSC voltages were adjusted to appropriate values and saved. Blank sample B and standard curve 1 were tested according to the instructions for Quantum™ FITC-5 MESF Kit, and the FITC voltage was adjusted to an appropriate value and saved. The samples in the U-bottomed 96-well plate were tested at the saved voltage, and data were recorded. The experimental data were analyzed using Flowjo software to obtain a Geo mean, and an MESF-Geo Mean standard curve was fit according to the instructions for Quantum™ FITC-5 MESF Kit. The molar concentration of the human claudin18.2 antibody bound to HEK293/hClaudin18.2 cells and the free antibody concentration were calculated according to the concentration fluorescence value of the FITC anti-human IgG Fc antibody, and the Bmax and the dissociation constant KD of the antibody were calculated through Scatchard plots. The results are shown in Table 13.

Table 13. Cell-level affinity of humanized antibodies

| Antibody | IMAB362 | h1901-11 | h1902-5 |
|---|---|---|---|
| KD (nM) | 10.2 | 6.8 | 1.64 |

**Test Example 5. ADCC Effect Evaluation of Antibodies**

A variety of NUGC4 cells (with high, moderate and low expression of claudin18.2) were digested, centrifuged at 1000 rpm, resuspended, and counted. The cells were resuspended at a density of $3 \times 10^5$ cells/mL in phenol red-free RPMI 1640 (Gibco, 11835-030) supplemented with 10% FBS (New Zealand ultra-low IgG fetal bovine serum, Gibco, 1921005PJ). 25 μL of cells were added to each well in a 96-well plate (Corning, 3903) (7500 cells/well). An antibody was diluted into the phenol red-free medium to give a 3× antibody dilution, which was then added to the cell plate at 25 μL/well. The plate was incubated in a 37 °C incubator with 5% $CO_2$ for 0.5 h.

Effector cells (FcrR3A-V158-NFAT-RE-Jurkat cells) were harvested, centrifuged at 1000 rpm, resuspended, and counted. The cells were resuspended at a density of $3 \times 10^6$ cells/mL in phenol red-free RPMI 1640 supplemented with 10% FBS (New Zealand ultra-low IgG fetal bovine serum), and 25 μL of the cells were added to each well of the plate ($7.5 \times 10^4$ cells/well). The plate was incubated in a 37 °C incubator with 5% $CO_2$ for 6 h.

75 μL of Bright-Glo (Promega, E2610) was added to each well of the plate, and the chemical luminescence was detected using a microplate reader (PerkinElmer, VITOR3). The results (see Table 14 and FIGs. 3A-3C) show that both antibodies h1901-11 and h1902-5 show high ADCC activity in the NUGC4 cells with low (FIG. 3A), moderate (FIG. 3B) and high (FIG. 3C) expression of claudin18.2.

Table 14. ADCC effect of antibodies in NUGC4 Cells with varying expression levels of claudin18.2 Unit $IC_{50}$ (ng/mL)

| Expression level of claudin18.2 | h1901-11 | h1902-5 | IMAB362 |
|---|---|---|---|
| Low expression | 22.42 | 35.46 | 183.4 |
| Moderate expression | 15.35 | 30.00 | 210.4 |
| High expression | 26.17 | 32.16 | 132.6 |

**Test Example 6. Inhibition *of In Vitro* Proliferation of Tumor Cells by Compounds**

A. Purpose

This experiment was intended to test the inhibitory activity of the pharmaceutical compounds of the present disclosure against the *in vitro* proliferation of U87MG cells (glioma cells, Cell Bank, Chinese Academy of Sciences, Catalog # TCHu138) and SK-BR-3 tumor cells (human breast cancer cells, ATCC, Catalog # HTB-30). The cells were treated *in vitro* with a compound at different concentrations. After 6 days of culture, the proliferation of cells was tested using CTG (CellTiter-Glo® Luminescent Cell Viability Assay, Promega, Catalog # G7573) reagents, and the *in vitro* activity of the compound was evaluated according to the $IC_{50}$ value.

B. Method

The method of testing the inhibition of the *in vitro* proliferation of U87MG cells was described below as an example for the method of assaying for the inhibitory activity of the compounds of the present disclosure against the *in vitro* proliferation of tumor cells. The method is also applicable to, but not limited to, tests for inhibitory activity against the *in vitro* proliferation of other tumor cells.

1. Cell culture: U87MG and SK-BR-3 cells were cultured in EMEM medium (GE, Catalog # SH30024.01) containing 10% FBS and McCoy's 5A medium (Gibco, Catalog # 16600-108) containing 10% FBS, respectively.
2. Preparation of cells. U87MG and SK-BR-3 cells growing at log phase were washed once with PBS (phosphate buffer, Shanghai BasalMedia Technologies Co., Ltd.) and then digested with 2-3 mL of trypsin (0.25% Trypsin-EDTA (1×), Gibico, Life Technologies) for 2-3 min. After the cells were completely digested, 10-15 mL of cell culture media were added to elute the digested cells. The mixtures were centrifuged at 1000 rpm for 5 min, and the supernatants were discarded. Then the cells were resuspended in 10-20 mL of cell culture media to give single-cell suspensions.
3. Cell plating. The U87MG and SK-BR-3 single-cell suspensions were each well mixed and adjusted with cell culture media to cell densities of $2.75 \times 10^3$ cells/mL and $8.25 \times 10^3$ cells/mL, respectively. The adjusted cell suspensions were each well mixed and added to 96-well cell culture plates at 180 μL/well. To each of the peripheral wells of the 96-well plates was added 200 μL of media only. The plate was incubated in an incubator for 24 h (37 °C, 5% $CO_2$).
4. Preparation of compounds. A compound was dissolved in DMSO (dimethyl sulfoxide, Shanghai Titan Scientific Co., Ltd.) to prepare a stock solution at an initial concentration of 10 mM.

Small molecule compounds were prepared at an initial concentration of 500 nM as follows. Different test samples at concentration 100 μM (30 μL) were added to the first column of a 96-well U-bottom plate, and 20 μL of DMSO was added to each well of the second column through the eleventh column. The samples in the first column (10 μL) were added to the 20 μL of DMSO in the second column, and the mixtures were well mixed. The mixtures (10 μL) were added to the third column, and so on to the tenth column. The drugs in the plate (5 μL per well) were transferred to EMEM media (95 μL), and the mixtures were well mixed for later use.

ADCs were prepared at an initial concentration of 10 nM or 500 nM as follows.

Different test samples at concentration 100 nM or 5 μM (100 μL) were added to the first column of a 96-well plate, and 100 μL of PBS was added to each well of the second column through the eleventh column. The samples in the first column (50 μL) were added to the 100 μL of PBS in the second column, and the mixtures were well mixed. The mixtures (50 μL) were added to the third column, and so on, by 3-fold dilution, to the tenth column.

5. Sample addition. The test samples prepared at different concentrations (20 μL) were added to a culture plate, with two duplicate wells set for each sample. The plate was incubated in an incubator for 6 days (37 °C, 5% $CO_2$),

6. Color development. The 96-well cell culture plate was taken out, and 90 μL of CTG solution was added to each well, followed by 10 min of incubation at room temperature.

7. Plate reading. The 96-well cell culture plate was taken out and tested in a microplate reader (BMG labtech, PHERAstar FS) for chemiluminescence.

C. Data analysis

Data were processed and analyzed using Microsoft Excel and Graphpad Prism 5. The example results are shown in the table below.

Table 15. $IC_{50}$ values of the small molecule fragments of the present disclosure in inhibiting *in vitro* proliferation of SK-BR-3 cells and U87 cells

| Compound example | $IC_{50}$ (nM) | |
|---|---|---|
| | SK-BR-3 | U87 |
| Example 1 | 0.12 | 0.23 |
| Example 2 2-B with shorter retention time | 0.33 | 0.86 |
| Example 2 2-B with longer retention time | 8.11 | 2.31 |
| Example 3 Shorter retention time | 0.36 | 0.83 |
| Example 3 Longer retention time | 1.67 | 2.98 |
| Example 4 | 1.9 | / |
| Example 5 | / | 4.81 |
| Example 6 | / | 1.83 |
| Example 7 | / | 1.95 |

Conclusion: The small molecular fragments of the present disclosure have significant inhibitory activity against the proliferation of SK-BR-3 cells and U87 cells, and the chiral centers have certain influence on the inhibitory activity of the compounds.

**Test Example 7: Cell Viability Assays of ADC Molecules**

CellTiter-Glo luminescence cell viability assays were used to test ADC molecules for the *in vitro* killing effects on the human gastric cancer cell strain in this experiment. On day 1, NUGC4 cells with low, moderate and high claudin18.2 expression were harvested, adjusted to density 2.5 $\times$ $10^4$/mL, and added to a 96-well white transparent plate at 90 $\mu$L/well, with about 2500 cells per well. The cells were cultured overnight in a 37 °C incubator with 5% $CO_2$. On day 2, samples were 4$\times$ diluted serially from an initial concentration of 5 $\mu$M in a U-bottom 96-well plate to obtain 9 concentration points, and the diluted samples were added to the cell plate at 10 $\mu$L/well. The cells were cultured at 37 °C in 5% $CO_2$ for 6 days. On day 8, the cell culture plate was taken out, and Cell Titer-Glo Reagent were added at 50 $\mu$L/well. The plate was let stand at room temperature for 2-3 min and read on a PHERAstar FS plate reader for fluorescence values. Data analysis was performed using the GraphPad Prism software. See Table 16.

Table 16. *In vitro* killing effects of the ADC molecules of the present disclosure on cells

| ADC | NUGC4 cells with low claudin18.2 expression | | NUGC4 cells with moderate claudin18.2 expression | | NUGC4 cells with high claudin18.2 expression | |
|---|---|---|---|---|---|---|
| | $EC_{50}$ (nM) | Emax (%) | $EC_{50}$ (nM) | Emax (%) | $EC_{50}$ (nM) | Emax (%) |
| ADC-1 | 126.8 | 66.7 | 23.6 | 82.1 | 1.3 | 91.5 |
| ADC-2 | 109.0 | 69.8 | 16.8 | 82.3 | 1.9 | 91.1 |
| ADC-3 | >500 | 49.0 | | | 94 | 78.7 |
| ADC-4 | 299 | 61.03 | | | 12 | 84.97 |
| ADC-5 | 142 | 69.91 | | | 3.5 | 95.89 |
| ADC-6 | >500 | 45.22 | | | 11 | 78.00 |

(continued)

| ADC | NUGC4 cells with low claudin18.2 expression | | NUGC4 cells with moderate claudin18.2 expression | | NUGC4 cells with high claudin18.2 expression | |
|---|---|---|---|---|---|---|
| | $EC_{50}$ (nM) | Emax (%) | $EC_{50}$ (nM) | Emax (%) | $EC_{50}$ (nM) | Emax (%) |
| ADC-7 | 284 | 61.09 | | | 3.9 | 88.51 |
| ADC-8 | 154 | 66.74 | | | 1.3 | 97.15 |

Biological Evaluation *of In Vivo* Activity

**Test Example 8. Evaluation *of In Vivo* Efficacy of ADC Molecules**

Balb/c nude mice were inoculated subcutaneously in the right flank with human gastric cancer cells, NUGC4 cells (with moderate claudin18.2 expression) ($5 \times 10^6$ cells in 50% matrigel/mouse) and divided at day 0 into a total of 5 groups of 8. The mean tumor volume was about 84.41 $mm^3$.

Each mouse was intraperitoneally injected with an ADC at 0.1 mL/10 g body weight at days 0, 4 and 11, making a total of 3 injections.

Each mouse was intraperitoneally injected with an ADC at 0.1 mL/10 g body weight from the day of grouping at intervals of 5 days, for a total of 4 injections.

The tumor volumes and body weights were measured twice a week and the results were recorded.

Excel 2003 statistical software was used. The mean values were calculated as avg; the SD values were calculated as STDEV; the SEM values were calculated as STDEV/SQRT; and the inter-group difference P-value was calculated as TTEST.

Tumor volume (V) was calculated as: $V = 1/2 \times L_{long} \times L_{short}^2$

Relative volume (RTV) = VT/V0

Tumor inhibition rate (%) = (CRTV - TRTV)/CRTV (%)

where V0 and VT are the tumor volumes at the beginning of the experiment (the day of first administration is defined as day 0) and at the time of measurement, respectively; CRTV and TRTV are the relative tumor volumes of the blank control group and the experimental groups, respectively, at the end of the experiment. The results are shown in Table 17 and FIGs. 4 and 5.

Table 17. Results of inhibition of tumors by ADCs

| **Group** | Mean tumor volume ($mm^3$) | | Mean tumor volume ($mm^3$) | | Relative tumor volume | | **% tumor inhibition rate D32** |
|---|---|---|---|---|---|---|---|
| | **D0** | **SEM** | **D32** | **SEM** | **D0** | **SEM** | |
| Blank control group | 83.33 | 0.82 | 2067.0 | 102.24 | 24.83 | 1.27 | - |
| ADC-2 10 mpk | 83.93 | 1.65 | 263.13 | 44.17 | 3.11 | 0.51 | 87.47%** |
| ADC-2 3 mpk | 84.35 | 1.83 | 328.95 | 45.04 | 3.86 | 0.48 | 84.45%** |
| ADC-1 10 mpk | 83.60 | 1.61 | 123.80 | 20.99 | 1.48 | 0.25 | 94.04%** |
| ADC-1 3 mpk | 86.84 | 1.91 | 356.41 | 55.18 | 4.06 | 0.58 | 83.65%** |
| vs blank: ** $p < 0.01$. | | | | | | | |

## Claims

**1.** A ligand-drug conjugate of general formula (Pc-L-Y-D) or a pharmaceutically acceptable salt thereof:

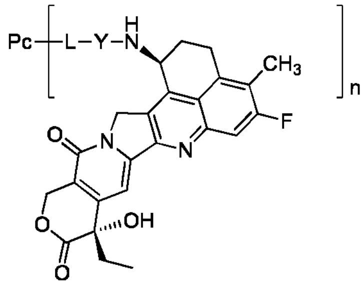

(Pc-L-Y-D)

wherein:

Y is selected from the group consisting of -O-$(CR^aR^b)_m$-$CR^1R^2$-C(O)-, -O-$CR^1R^2$-$(CR^aR^b)_m$-, -O-$CR^1R^2$-, -NH-$(CR^aR^b)_m$-$CR^1R^2$-C(O)- and -S-$(CR^aR^b)_m$-$CR^1R^2$-C(O)-;

$R^a$ and $R^b$ are identical or different and are each independently selected from the group consisting of hydrogen, deuterium, halogen, alkyl, haloalkyl, deuterated alkyl, alkoxy, hydroxy, amino, cyano, nitro, hydroxyalkyl, cycloalkyl and heterocyclyl; or, $R^a$ and $R^b$, together with carbon atoms connected thereto, form cycloalkyl or heterocyclyl;

$R^1$ is selected from the group consisting of halogen, haloalkyl, deuterated alkyl, cycloalkyl, cycloalkylalkyl, alkoxyalkyl, heterocyclyl, aryl and heteroaryl; $R^2$ is selected from the group consisting of hydrogen, halogen, haloalkyl, deuterated alkyl, cycloalkyl, cycloalkylalkyl, alkoxyalkyl, heterocyclyl, aryl and heteroaryl; or, $R^1$ and $R^2$, together with carbon atoms connected thereto, form cycloalkyl or heterocyclyl;

or, $R^a$ and $R^2$, together with carbon atoms connected thereto, form cycloalkyl or heterocyclyl;

m is an integer from 0 to 4;

n is a decimal or an integer from 1 to 10;

L is a linker unit;

Pc is an anti-claudin18.2 antibody or an antigen-binding fragment thereof.

**2.** The ligand-drug conjugate of general formula (Pc-L-Y-D) or a pharmaceutically acceptable salt thereof according to claim 1, wherein the anti-claudin18.2 antibody or the antigen-binding fragment thereof comprises a heavy chain variable region and a light chain variable region, wherein:

i) the heavy chain variable region comprises an HCDR1, an HCDR2 and an HCDR3 having sequences identical to those of an HCDR1, an HCDR2 and an HCDR3 of a heavy chain variable region set forth in SEQ ID NO: 3, and the light chain variable region comprises an LCDR1, an LCDR2 and an LCDR3 having sequences identical to those of an LCDR1, an LCDR2 and an LCDR3 of a light chain variable region set forth in SEQ ID NO: 4; or

ii) the heavy chain variable region comprises an HCDR1, an HCDR2 and an HCDR3 having sequences identical to those of an HCDR1, an HCDR2 and an HCDR3 of a heavy chain variable region set forth in SEQ ID NO: 5, and the light chain variable region comprises an LCDR1, an LCDR2 and an LCDR3 having sequences identical

to those of an LCDR1, an LCDR2 and an LCDR3 of a light chain variable region set forth in SEQ ID NO: 6.

**3.** The ligand-drug conjugate of general formula (Pc-L-Y-D) or a pharmaceutically acceptable salt thereof according to claim 1 or 2, wherein the anti-claudin18.2 antibody or the antigen-binding fragment thereof comprises a heavy chain variable region and a light chain variable region, wherein:

iii) the heavy chain variable region comprises an HCDR1, an HCDR2 and an HCDR3 having sequences set forth in SEQ ID NO: 9, SEQ ID NO: 10 and SEQ ID NO: 11, respectively, and the light chain variable region comprises an LCDR1, an LCDR2 and an LCDR3 having sequences set forth in SEQ ID NO: 12, SEQ ID NO: 13 and SEQ ID NO: 14, respectively; or
iv) the heavy chain variable region comprises an HCDR1, an HCDR2 and an HCDR3 having sequences set forth in SEQ ID NO: 15, SEQ ID NO: 16 and SEQ ID NO: 17, respectively, and the light chain variable region comprises an LCDR1, an LCDR2 and an LCDR3 having sequences set forth in SEQ ID NO: 18, SEQ ID NO: 19 and SEQ ID NO: 20, respectively.

**4.** The ligand-drug conjugate of general formula (Pc-L-Y-D) or the pharmaceutically acceptable salt thereof according to any one of claims 1 to 3, wherein the anti-claudin18.2 antibody is a murine antibody, a chimeric antibody or a humanized antibody.

**5.** The ligand-drug conjugate of general formula (Pc-L-Y-D) or the pharmaceutically acceptable salt thereof according to any one of claims 1 to 4, wherein the anti-claudin18.2 antibody or the antigen-binding fragment thereof comprises a heavy chain variable region and a light chain variable region, wherein:

(1) the heavy chain variable region has an amino acid sequence set forth in SEQ ID NO: 3 or having at least 90% identity thereto, and the light chain variable region has an amino acid sequence set forth in SEQ ID NO: 4 or having at least 90% identity thereto;
(2) the heavy chain variable region has an amino acid sequence set forth in SEQ ID NO: 24 or having at least 90% identity thereto, and the light chain variable region has an amino acid sequence set forth in SEQ ID NO: 21 or having at least 90% identity thereto;
(3) the heavy chain variable region has an amino acid sequence set forth in SEQ ID NO: 5 or having at least 90% identity thereto, and the light chain variable region has an amino acid sequence set forth in SEQ ID NO: 6 or having at least 90% identity thereto; or
(4) the heavy chain variable region has an amino acid sequence set forth in SEQ ID NO: 31 or having at least 90% identity thereto, and the light chain variable region has an amino acid sequence set forth in SEQ ID NO: 28 or having at least 90% identity thereto.

**6.** The ligand-drug conjugate of general formula (Pc-L-Y-D) or the pharmaceutically acceptable salt thereof according to any one of claims 1 to 5, wherein the anti-claudin18.2 antibody is a humanized antibody comprising a framework region derived from a human antibody or a framework region variant thereof, and the framework region variant has reverse mutations of up to 10 amino acids in a light chain framework region and/or a heavy chain framework region of the human antibody; preferably, the framework region variant comprises mutations selected from (a) or (b):

(a) one or more amino acid reverse mutations optionally selected from the group consisting of 22S, 85I and 87H, comprised in the light chain variable region; and/or one or more amino acid reverse mutations optionally selected from the group consisting of 48I, 82T and 69M, comprised in the heavy chain variable region; or
(b) one or more amino acid reverse mutations optionally selected from the group consisting of 4L and 22S, comprised in the light chain variable region; and/or one or more amino acid reverse mutations optionally selected from the group consisting of 38K, 40R, 48I, 66K, 67A, 69L, 71L and 73K, comprised in the heavy chain variable region;

preferably, the framework region variant comprises mutations selected from the group consisting of:

(a-1) 22S, 85I and 87H amino acid reverse mutations comprised in the light chain variable region, and 48I and 82T amino acid reverse mutations comprised in the heavy chain variable region; or
(b-1) 4L amino acid reverse mutation comprised in the light chain variable region.

**7.** The ligand-drug conjugate of general formula (Pc-L-Y-D) or the pharmaceutically acceptable salt thereof according to any one of claims 1 to 6, wherein the anti-claudin18.2 antibody or the antigen-binding fragment thereof comprises

a heavy chain variable region and a light chain variable region shown below:

(vii) the heavy chain variable region having a sequence set forth in SEQ ID NO: 3, and the light chain variable region having a sequence set forth in SEQ ID NO: 4;
(viii) the heavy chain variable region having a sequence set forth in SEQ ID NO: 24, SEQ ID NO: 25, SEQ ID NO: 26 or SEQ ID NO: 27, and the light chain variable region having a sequence set forth in SEQ ID NO: 21, SEQ ID NO: 22 or SEQ ID NO: 23;
(ix) the heavy chain variable region having a sequence set forth in SEQ ID NO: 5, and the light chain variable region having a sequence set forth in SEQ ID NO: 6; or
(x) the heavy chain variable region having a sequence set forth in SEQ ID NO: 31, SEQ ID NO: 32, SEQ ID NO: 33 or SEQ ID NO: 34, and the light chain variable region having a sequence set forth in SEQ ID NO: 28, SEQ ID NO: 29 or SEQ ID NO: 30; preferably, the anti-claudin18.2 antibody or the antigen-binding fragment thereof comprises a heavy chain variable region and a light chain variable region shown below:
(xi) the heavy chain variable region having a sequence set forth in SEQ ID NO: 31, and the light chain variable region having a sequence set forth in SEQ ID NO: 29; or
(xii) the heavy chain variable region having a sequence set forth in SEQ ID NO: 26, and the light chain variable region having a sequence set forth in SEQ ID NO: 23.

**8.** The ligand-drug conjugate of general formula (Pc-L-Y-D) or the pharmaceutically acceptable salt thereof according to any one of claims 1 to 7, wherein the anti-claudin18.2 antibody or the antigen-binding fragment thereof comprises a heavy chain constant region and a light chain constant region of the antibody;

preferably, the heavy chain constant region is selected from the group consisting of human IgG1, IgG2, IgG3 and IgG4 constant regions and conventional variants thereof, and the light chain constant region is selected from the group consisting of human antibody $\kappa$ and $\lambda$ chain constant regions and conventional variants thereof; more preferably, the anti-claudin18.2 antibody or the antigen-binding fragment thereof comprises a heavy chain constant region having a sequence set forth in SEQ ID NO: 7 and a light chain constant region having a sequence set forth in SEQ ID NO: 8;
most preferably, the anti-claudin18.2 antibody or the antigen-binding fragment thereof comprises: a heavy chain having at least 90% sequence identity to a heavy chain set forth in SEQ ID NO: 35 or SEQ ID NO: 42, and a light chain having at least 90% sequence identity to a light chain set forth in SEQ ID NO: 36 or SEQ ID NO: 39; or a heavy chain having at least 90% sequence identity to a heavy chain set forth in SEQ ID NO: 37 or SEQ ID NO: 49, and a light chain having at least 90% sequence identity to a light chain set forth in SEQ ID NO: 38 or SEQ ID NO: 46.

**9.** The ligand-drug conjugate of general formula (Pc-L-Y-D) or the pharmaceutically acceptable salt thereof according to any one of claims 1 to 8, wherein the anti-claudin18.2 antibody or the antigen-binding fragment thereof comprises:

(c) a heavy chain having a sequence set forth in SEQ ID NO: 35 and a light chain having a sequence set forth in SEQ ID NO: 36;
(d) a heavy chain having a sequence set forth in SEQ ID NO: 42, SEQ ID NO: 43, SEQ ID NO: 44 or SEQ ID NO: 45 and a light chain having a sequence set forth in SEQ ID NO: 39, SEQ ID NO: 40 or SEQ ID NO: 41;
(e) a heavy chain having a sequence set forth in SEQ ID NO: 37 and a light chain having a sequence set forth in SEQ ID NO: 38; or
(f) a heavy chain having a sequence set forth in SEQ ID NO: 49, SEQ ID NO: 50, SEQ ID NO: 51 or SEQ ID NO: 52 and a light chain having a sequence set forth in SEQ ID NO: 46, SEQ ID NO: 47 or SEQ ID NO: 48.

**10.** The ligand-drug conjugate of general formula (Pc-L-Y-D) or the pharmaceutically acceptable salt thereof according to any one of claims 1 to 9, wherein the anti-claudin18.2 antibody is selected from the group consisting of:

h1901-11, comprising a heavy chain having an amino acid sequence set forth in SEQ ID NO: 44 and a light chain set forth in SEQ ID NO: 41; and
h1902-5, comprising a heavy chain having an amino acid sequence set forth in SEQ ID NO: 49 and a light chain set forth in SEQ ID NO: 47.

**11.** The ligand-drug conjugate of general formula (Pc-L-Y-D) or the pharmaceutically acceptable salt thereof according to any one of claims 1 to 10, wherein n is a decimal or integer from 2 to 8, preferably a decimal or integer from 3.5 to 4.5.

**12.** The ligand-drug conjugate of general formula (Pc-L-Y-D) or the pharmaceutically acceptable salt thereof according to any one of claims 1 to 11, wherein:

Y is $-O-(CR^aR^b)_m-CR^1R^2-C(O)-$;
$R^a$ and $R^b$ are identical or different and are each independently selected from the group consisting of hydrogen, deuterium, halogen and alkyl;
$R^1$ is haloalkyl or $C_{3-6}$ cycloalkyl;
$R^2$ is selected from the group consisting of hydrogen, haloalkyl and $C_{3-6}$ cycloalkyl;
or, $R^1$ and $R^2$, together with carbon atoms connected thereto, form $C_{3-6}$ cycloalkyl;
m is 0 or 1.

**13.** The ligand-drug conjugate of general formula (Pc-L-Y-D) or the pharmaceutically acceptable salt thereof according to any one of claims 1 to 12, wherein Y is selected from the group consisting of:

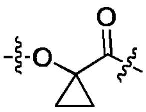 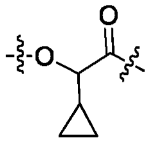 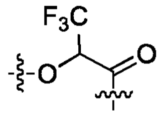 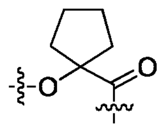 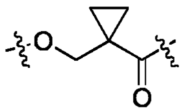 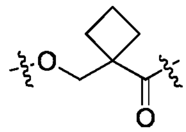

and

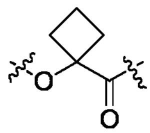 ;

wherein an O-terminus of Y is connected to the linker unit L.

**14.** The ligand-drug conjugate of general formula (Pc-L-Y-D) or the pharmaceutically acceptable salt thereof according to any one of claims 1 to 13, wherein the linker unit

-L- is $-L^1-L^2-L^3-L^4-$ , wherein
$L^1$ is selected from the group consisting of -(succinimidyl-3-yl-*N*)-W-C(O)-, $-CH_2-C(O)-NR^3-W-C(O)-$ and -C(O)-W-C(O)-, wherein W is selected from the group consisting of $C_{1-8}$ alkyl, $C_{1-8}$ alkyl-$C_{3-6}$ cycloalkyl and linear heteroalkyl of 1 to 8 chain atoms, and the heteroalkyl comprises 1 to 3 heteroatoms selected from the group consisting of N, O and S, wherein the $C_{1-8}$ alkyl, $C_{1-8}$ alkyl-$C_{3-6}$ cycloalkyl or linear heteroalkyl of 1 to 8 chain atoms is independently optionally further substituted with one or more substituents selected from the group consisting of halogen, hydroxy, cyano, amino, alkyl, chloroalkyl, deuterated alkyl, alkoxy and cycloalkyl;
$L^2$ is selected from the group consisting of $-NR^4(CH_2CH_2O)_{p^1}CH_2CH_2C(O)-$, $-NR^4(CH_2CH_2O)_{p^1}CH_2C(O)-$, $-S(CH_2)_{p^1}C(O)-$ and a chemical bond, wherein $p^1$ is an integer from 1 to 20;
$L^3$ is a peptide residue consisting of 2 to 7 amino acids, wherein the amino acids are selected from the group consisting of amino acid residues formed from amino acids from phenylalanine, glycine, valine, lysine, citrulline, serine, glutamic acid and aspartic acid, and are optionally further substituted with one or more substituents selected from the group consisting of halogen, hydroxy, cyano, amino, alkyl, chloroalkyl, deuterated alkyl, alkoxy and cycloalkyl;
$L^4$ is selected from the group consisting of $-NR^5(CR^6R^7)_t-$, $-C(O)NR^5-$, $-C(O)NR^5(CH_2)_t-$ and a chemical bond, wherein t is an integer from 1 to 6;
$R^3$, $R^4$ and $R^5$ are identical or different and are each independently selected from the group consisting of hydrogen, alkyl, haloalkyl, deuterated alkyl and hydroxyalkyl;
$R^6$ and $R^7$ are identical or different and are each independently selected from the group consisting of hydrogen, halogen, alkyl, haloalkyl, deuterated alkyl and hydroxyalkyl.

**15.** The ligand-drug conjugate of general formula (Pc-L-Y-D) or the pharmaceutically acceptable salt thereof according to any one of claims 1 to 14, wherein the linker unit

-L- is $-L^1-L^2-L^3-L^4-$, wherein

$L^1$ is

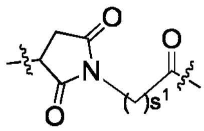

,

and $s^1$ is an integer from 2 to 8;
$L^2$ is a chemical bond;
$L^3$ is a tetrapeptide residue, preferably a tetrapeptide residue of GGFG;
$L^4$ is $-NR^5(CR^6R^7)t-$, wherein $R^5$, $R^6$ and $R^7$ are identical or different and are each independently hydrogen or alkyl, and t is 1 or 2;
wherein $L^1$ terminus is connected to Pc, and $L^4$ terminus is connected to Y.

**16.** The ligand-drug conjugate of general formula (Pc-L-Y-D) or the pharmaceutically acceptable salt thereof according to any one of claims 1 to 15, wherein -L- is:

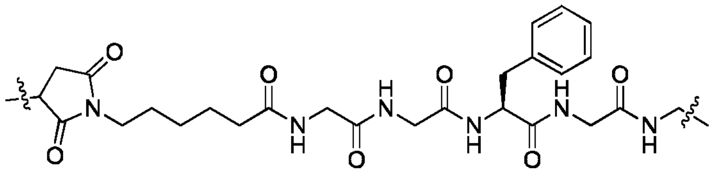

.

**17.** The ligand-drug conjugate of general formula (Pc-L-Y-D) or the pharmaceutically acceptable salt thereof according to any one of claims 1 to 16, wherein -L-Y- is optionally selected from the group consisting of:

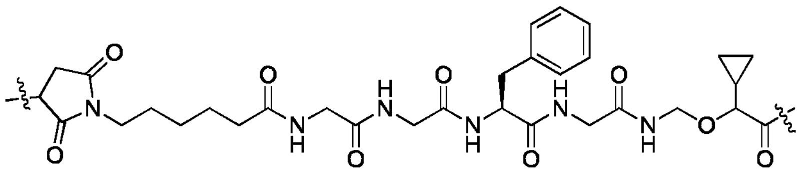

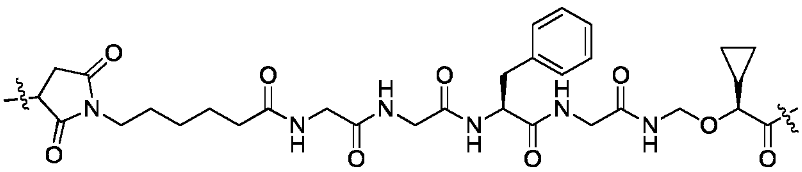

and

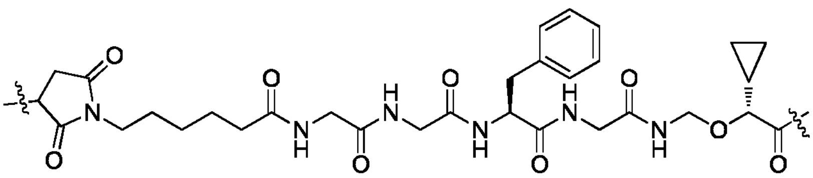

.

**18.** The ligand-drug conjugate of general formula (Pc-L-Y-D) or the pharmaceutically acceptable salt thereof according to any one of claims 1 to 17, wherein the ligand-drug conjugate of general formula (Pc-L-Y-D) or the pharmaceutically acceptable salt thereof is a ligand-drug conjugate of general formula ($Pc-L_a-Y-D$) or a pharmaceutically acceptable salt thereof,

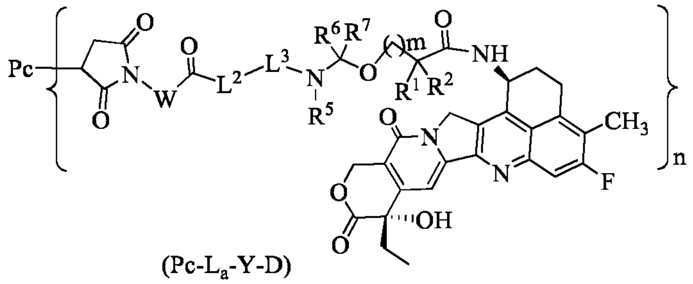

(Pc-$L_a$-Y-D)

wherein:

W, $L^2$, $L^3$, $R^5$, $R^6$ and $R^7$ are as defined in claim 14;
Pc, n, $R^1$, $R^2$ and m are as defined in claim 1.

**19.** The ligand-drug conjugate of general formula (Pc-L-Y-D) or the pharmaceutically acceptable salt thereof according to any one of claims 1 to 18, wherein the ligand-drug conjugate of general formula (Pc-L-Y-D) or the pharmaceutically acceptable salt thereof is a ligand-drug conjugate of general formula (Pc-$L_b$-Y-D) or a pharmaceutically acceptable salt thereof,

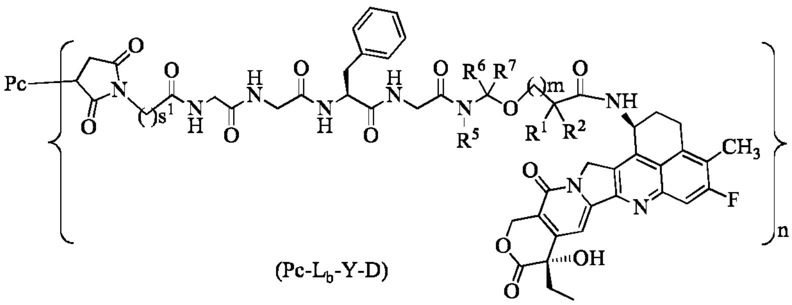

(Pc-$L_b$-Y-D)

wherein:

$s^1$ is an integer from 2 to 8;
Pc, $R^1$, $R^2$, $R^5$~$R^7$, m and n are as defined in claim 18.

**20.** The ligand-drug conjugate of general formula (Pc-L-Y-D) or the pharmaceutically acceptable salt thereof according to any one of claims 1 to 19, wherein the ligand-drug conjugate is selected from the group consisting of:

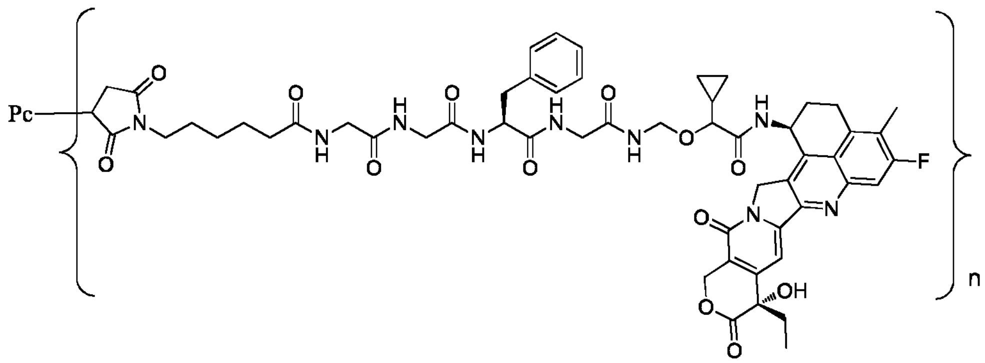

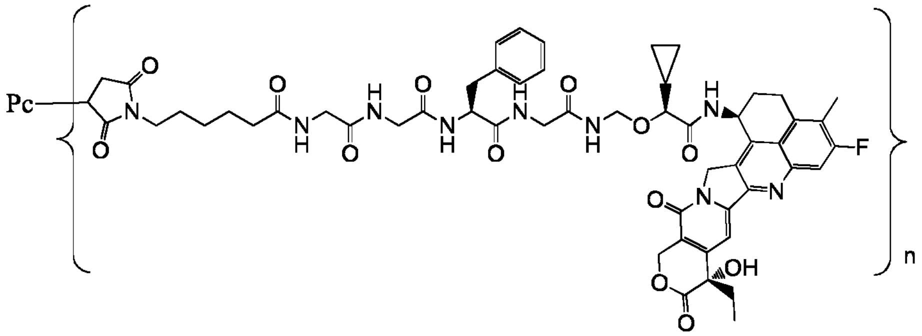

and

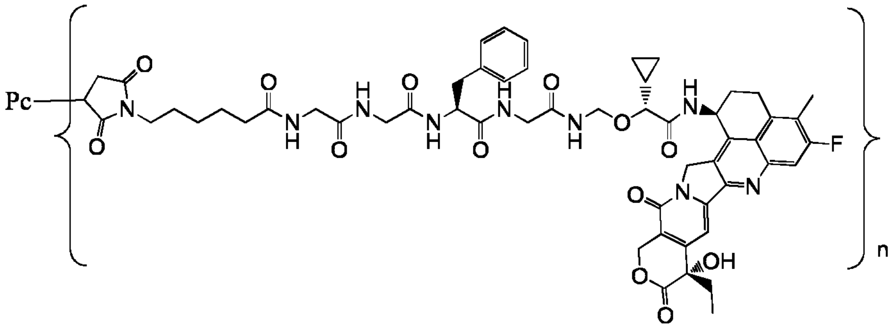

wherein Pc and n are as defined in claim 1.

**21.** The ligand-drug conjugate of general formula (Pc-L-Y-D) or the pharmaceutically acceptable salt thereof according to any one of claims 1 to 20, wherein the ligand-drug conjugate is selected from the group consisting of:

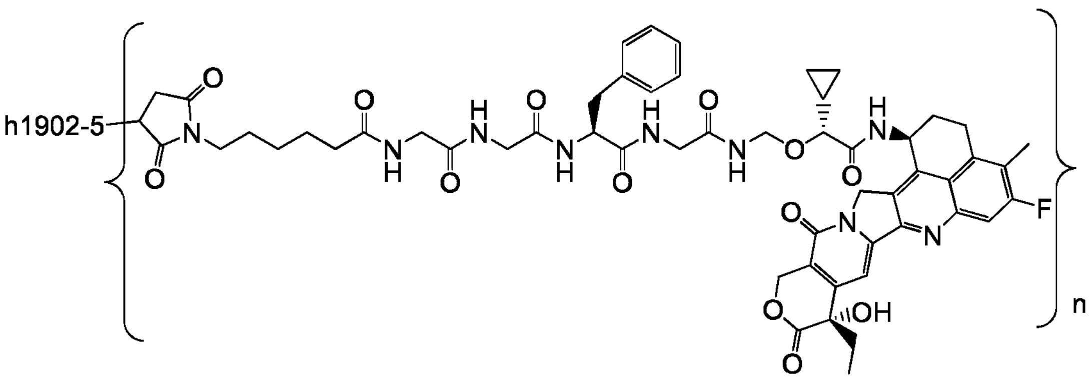

and

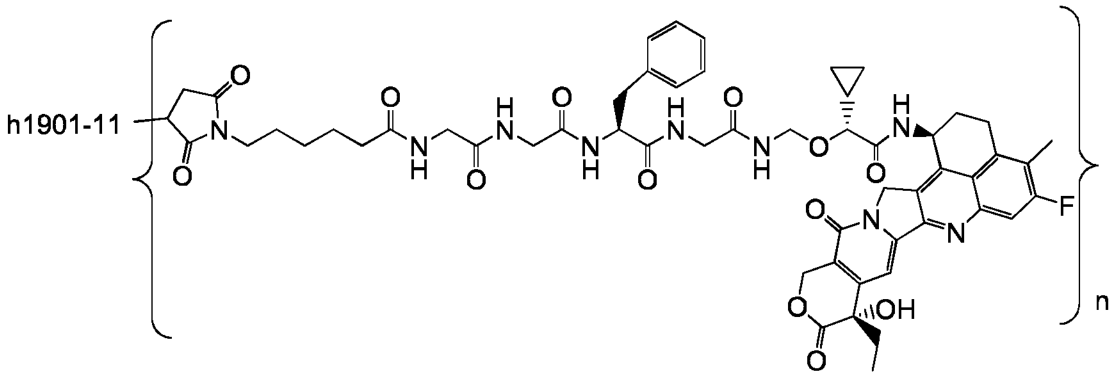

wherein n is as defined in claim 1, and the antibodies h1902-5 and h1901-11 are as defined in claim 10.

**22.** A method for preparing a ligand-drug conjugate of general formula (Pc-$L_a$-Y-D) or a pharmaceutically acceptable salt thereof, comprising the following steps:

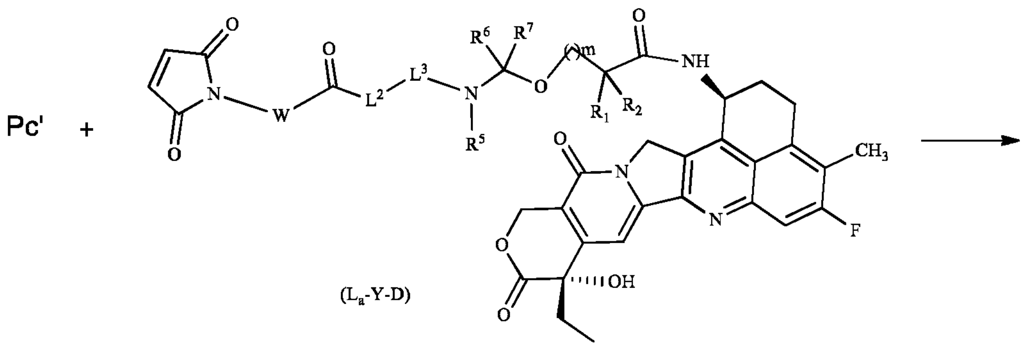

($L_a$-Y-D)

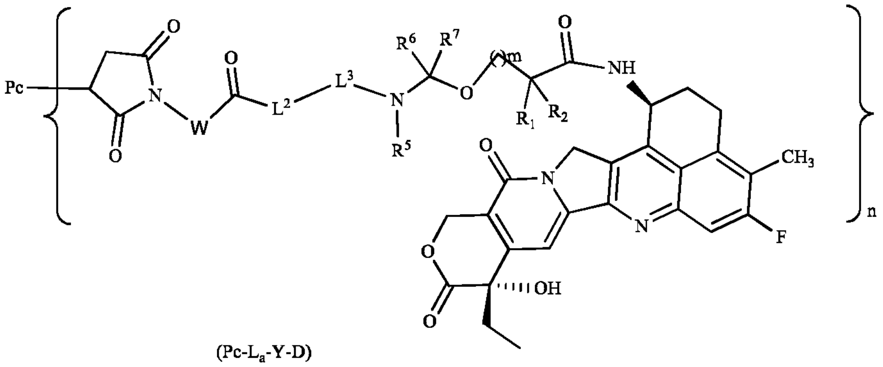

(Pc-$L_a$-Y-D)

subjecting Pc' and a compound of general formula ($L_a$-Y-D) to a coupling reaction to give a compound of general formula (Pc-$L_a$-Y-D);
wherein:

Pc is an anti-claudin18.2 antibody or an antigen-binding fragment thereof, and Pc' is obtained by reduction of Pc;
W, $L^2$, $L^3$, $R^1$, $R^2$, $R^5$-$R^7$, m and n are as defined in claim 18.

**23.** A pharmaceutical composition comprising the ligand-drug conjugate or the pharmaceutically acceptable salt thereof according to any one of claims 1 to 21 and one or more pharmaceutically acceptable excipients, diluents or carriers.

**24.** Use of the ligand-drug conjugate or the pharmaceutically acceptable salt thereof according to any one of claims 1 to 21 or the pharmaceutical composition according to claim 23 in preparing a medicament for treating a claudin18.2-mediated disease or condition.

**25.** The use according to claim 24, wherein the claudin18.2-mediated disease or condition is a cancer with high claudin18.2 expression.

**26.** Use of the ligand-drug conjugate or the pharmaceutically acceptable salt thereof according to any one of claims 1 to 21 or the pharmaceutical composition according to claim 23 in preparing a medicament for treating and/or preventing a tumor and cancer, wherein the tumor and cancer are preferably head and neck squamous cell carcinoma, head and neck cancer, brain cancer, neuroglioma, glioblastoma multiforme, neuroblastoma, central nervous system carcinoma, neuroendocrine tumor, throat cancer, nasopharyngeal cancer, esophageal cancer, thyroid cancer, malignant pleural mesothelioma, lung cancer, breast cancer, liver cancer, hepatobiliary cancer, pancreatic cancer, stomach cancer, gastrointestinal cancer, intestinal cancer, colon cancer, colorectal cancer, kidney cancer, clear cell renal cell carcinoma, ovarian cancer, endometrial cancer, cervical cancer, bladder cancer, prostate cancer, testicular cancer, skin cancer, melanoma, leukemia, lymphoma, bone cancer, chondrosarcoma, myeloma, multiple myeloma, myelodysplastic syndrome, Krukenberg tumor, myeloproliferative tumor, squamous cell carcinoma, Ewing's sarcoma, systemic light chain amyloidosis or Merkel cell carcinoma; more preferably, the lymphoma is selected from the group consisting of Hodgkin's lymphoma, non-Hodgkin's lymphoma, diffuse large B-cell lymphoma, follicular lymphoma, primary mediastinal large B-cell lymphoma, mantle cell lymphoma, small lymphocytic lymphoma, large B-cell lymphoma rich in T-cells/histiocytes and lymphoplasmacytic lymphoma, the lung cancer is selected from the group consisting of non-small cell lung cancer and small cell lung cancer, and the leukemia is selected from the group consisting of chronic myeloid leukemia, acute myeloid leukemia, lymphocytic leukemia, lymphoblastic leukemia, acute lymphoblastic leukemia, chronic lymphocytic leukemia and myeloid cell leukemia.

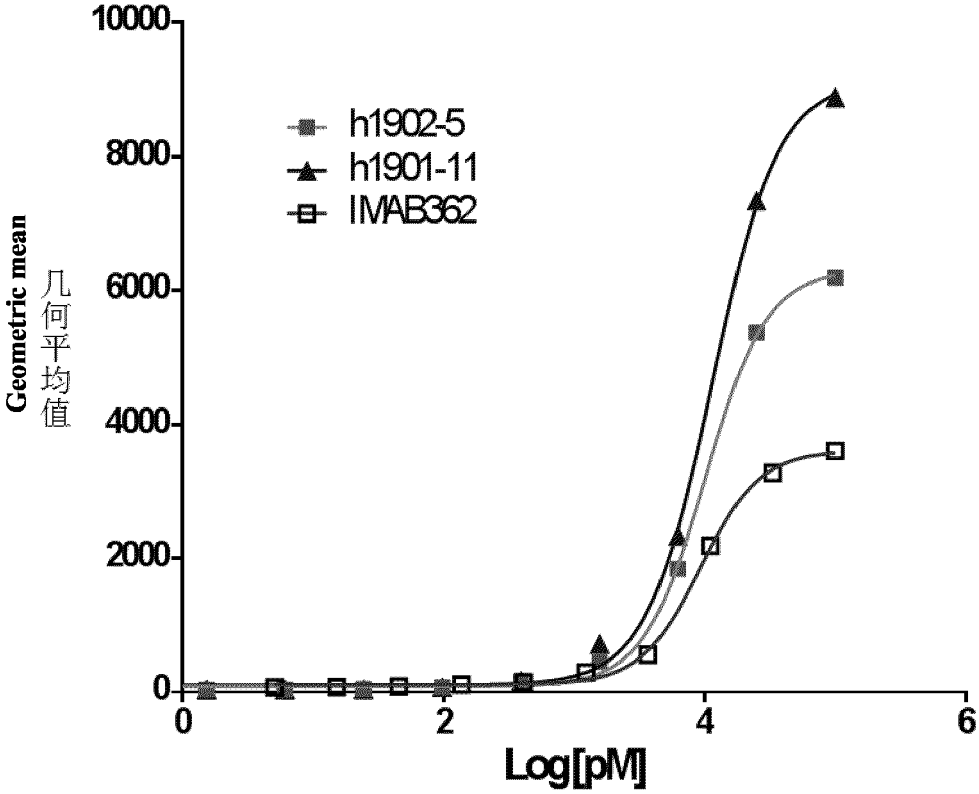
10000
8000
6000
4000
2000
0
0
2
4
6
h1902-5
h1901-11
IMAB362
Geometric mean
几何平均值
Log[pM]

FIG. 1

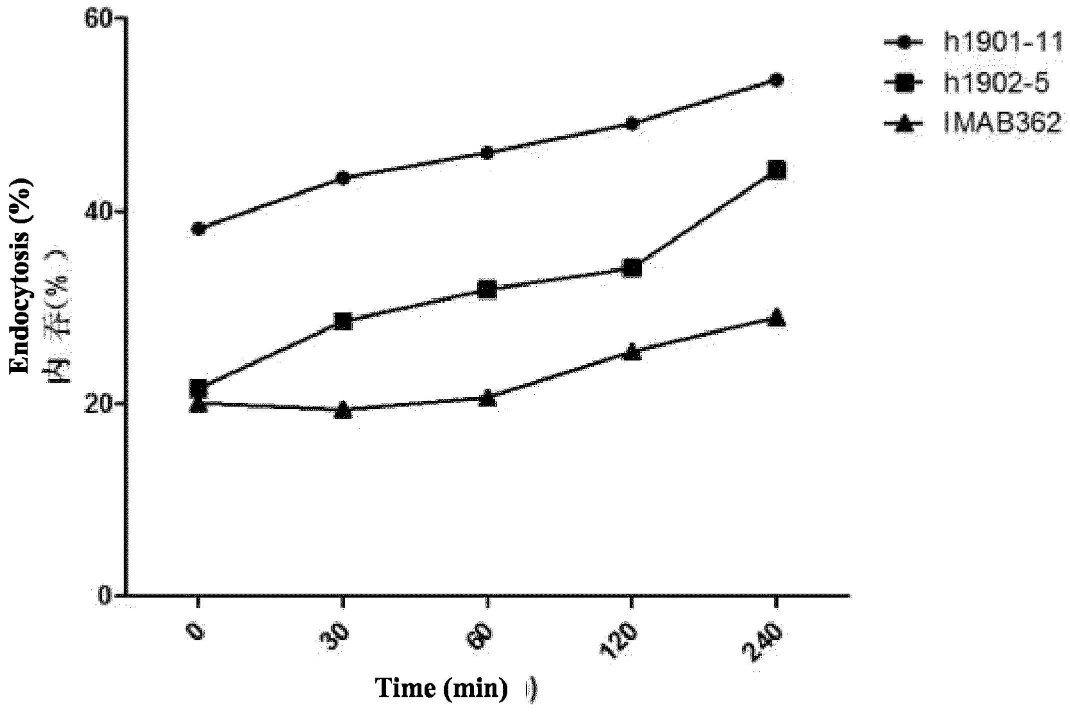
60
40
20
0
h1901-11
h1902-5
IMAB362
Endocytosis (%)
内吞(%)
0
30
60
120
240
Time (min)

FIG. 2

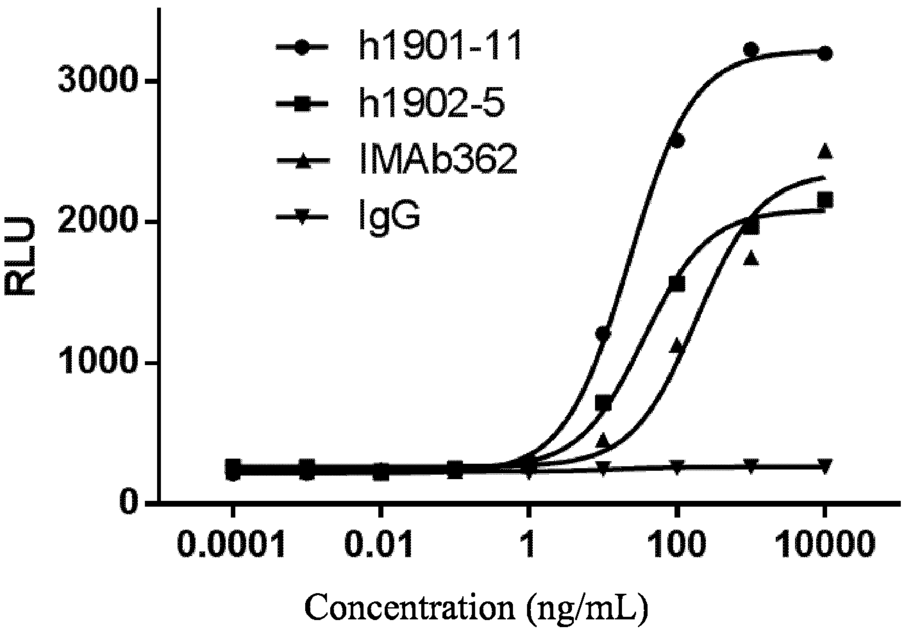
h1901-11
h1902-5
IMAb362
IgG
RLU
3000
2000
1000
0
0.0001
0.01
1
100
10000
Concentration (ng/mL)

FIG. 3A

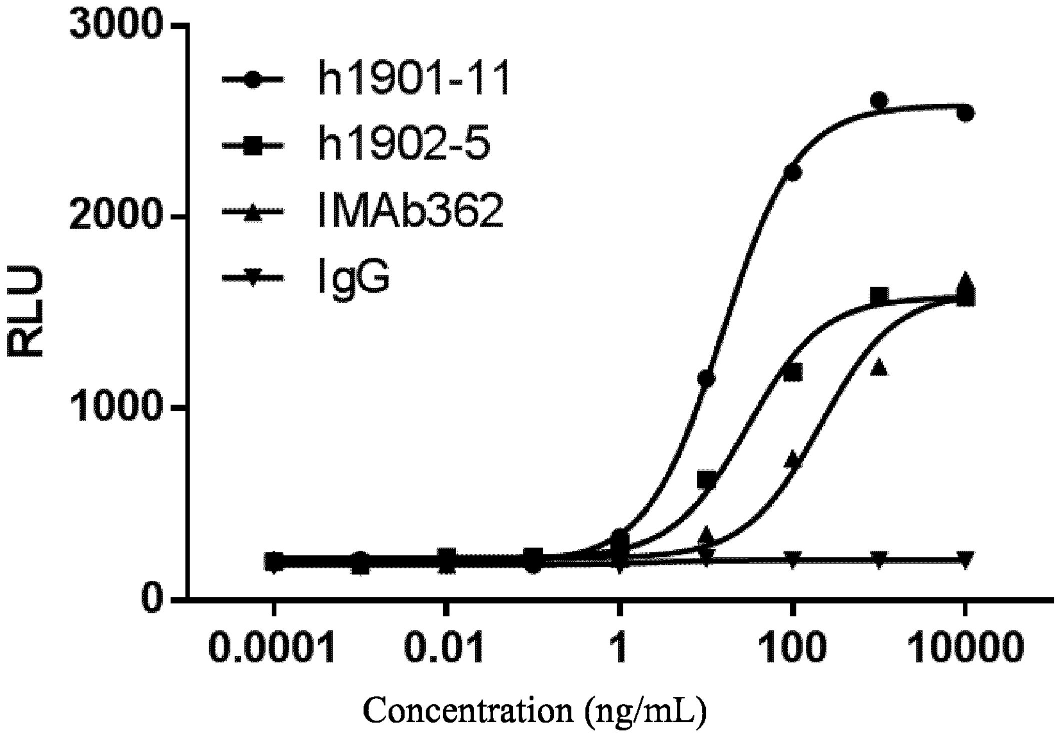
h1901-11
h1902-5
IMAb362
IgG
RLU
3000
2000
1000
0
0.0001
0.01
1
100
10000
Concentration (ng/mL)

FIG. 3B

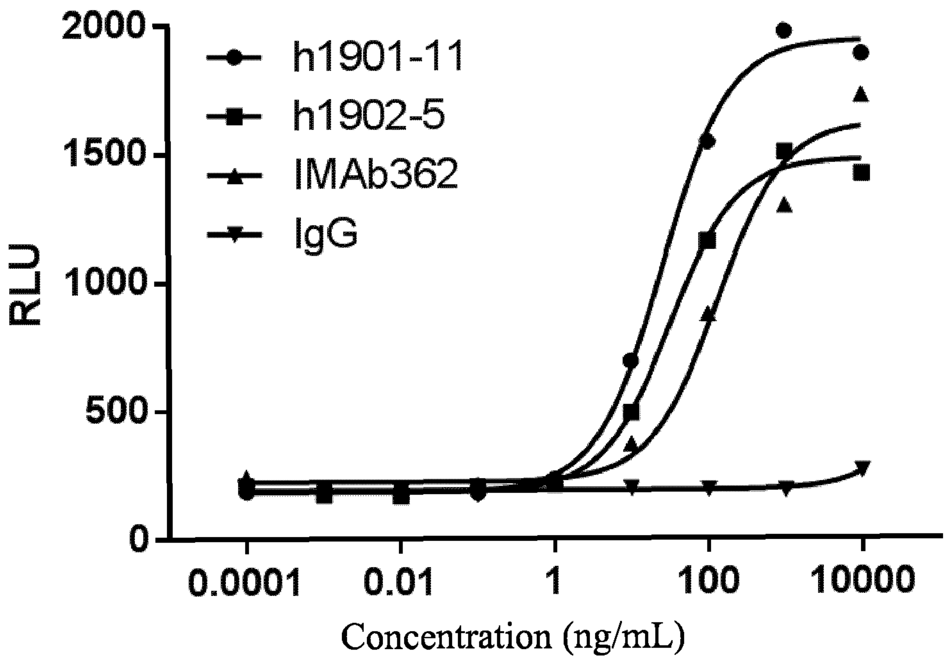
2000
1500
1000
500
0
RLU
h1901-11
h1902-5
IMAb362
IgG
0.0001
0.01
1
100
10000
Concentration (ng/mL)

FIG. 3C

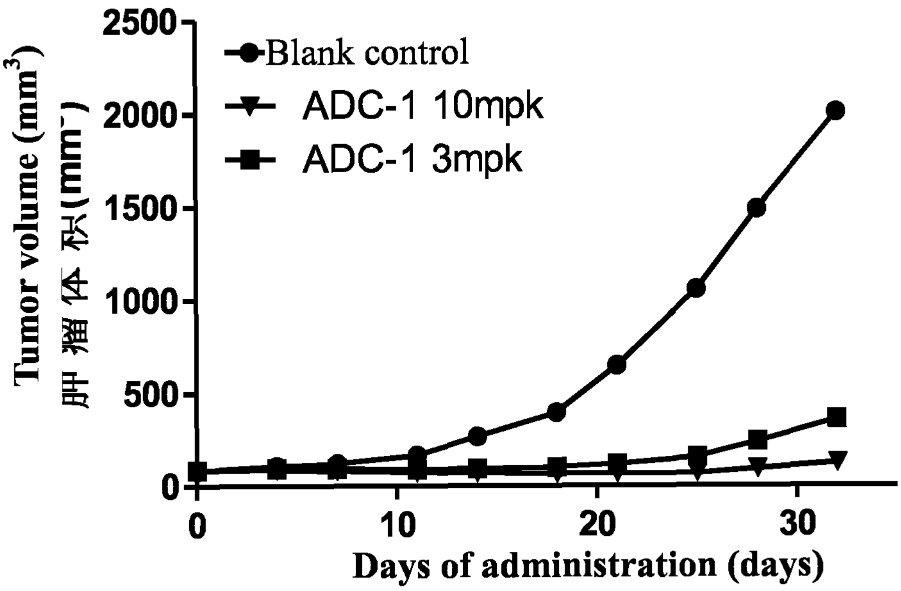
2500
2000
1500
1000
500
0
Tumor volume (mm3)
Blank control
ADC-1 10mpk
ADC-1 3mpk
0
10
20
30
Days of administration (days)

FIG. 4

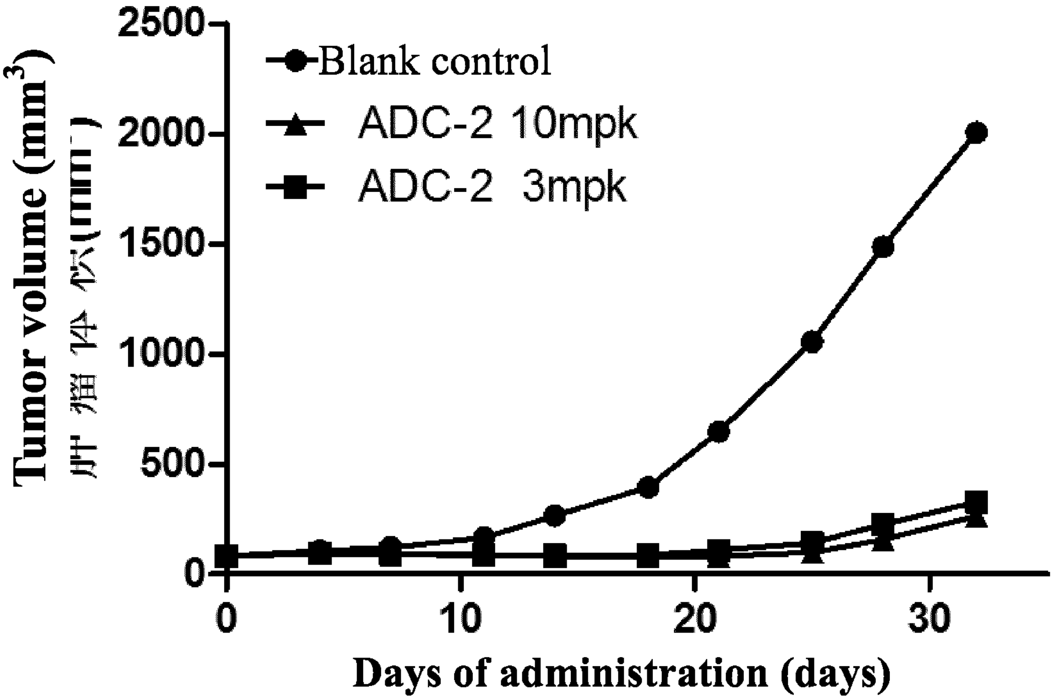
Tumor volume (mm3)
肿瘤体积(mm3)
2500
2000
1500
1000
500
0
Blank control
ADC-2 10mpk
ADC-2 3mpk
0
10
20
30
Days of administration (days)

FIG. 5

## INTERNATIONAL SEARCH REPORT

International application No.
**PCT/CN2020/135680**

**A. CLASSIFICATION OF SUBJECT MATTER**

A61K 47/68(2017.01)i； A61K 39/395(2006.01)i； A61K 31/4745(2006.01)i； A61P 35/00(2006.01)i

According to International Patent Classification (IPC) or to both national classification and IPC

**B. FIELDS SEARCHED**

Minimum documentation searched (classification system followed by classification symbols)

A61K A61P

Documentation searched other than minimum documentation to the extent that such documents are included in the fields searched

Electronic data base consulted during the international search (name of data base and, where practicable, search terms used)

CNABS, CNTXT, SIPOABS, DWPI, VEN, WOTXT, USTXT, EPTXT, CNKI, 万方数据库, WANFANG DATA, baidu学术, BAIDU XUESHU, PUBMED, ELSEVIER, SPRINGER, ISI web of knowledge, sciencedirect, NCBI, Genbank, EMBL, NONCODE, STN, 中国专利生物序列检索系统, Chinese Patent Biological Sequence Retrieval System: 依喜替康, 依沙替康, exatecan, DX-8951, 171335-80-1, 抗密蛋白, claudin18.2, CLDN18, 抗体-药物偶联物, 配体-药物偶联, antibody drug conjugate, ADC, 环烷基, 环基, 芳基, 环丙烷, cycloalkyl, aryl, cyclopropane, 江苏恒瑞, hengrui,exatecan, 杨阳, 许建烟, 陶维康

**C. DOCUMENTS CONSIDERED TO BE RELEVANT**

| Category* | Citation of document, with indication, where appropriate, of the relevant passages | Relevant to claim No. |
|---|---|---|
| Y | CN 104755494 A (DAIICHI SANKYO COMPANY, LIMITED) 01 July 2015 (2015-07-01) claims 1-49, description, paragraphs [0491]-[0544], and embodiment 43 | 1, 4-6, 8, 11-26 |
| Y | OGITANI, Y. et al. "DS-8201a, A Novel HER2-Targeting ADC with a Novel DNA Topoisomerase I Inhibitor, Demonstrates a Promising Antitumor Efficacy with Differentiation from T-DM1" *Clinical Cancer Research,* Vol. 22, No. 20, 29 March 2016 (2016-03-29), pp. 5097-5106 | 1, 4-6, 8, 11-26 |
| Y | CN 109762067 A (BEIJING MABWORKS BIOTECH CO., LTD.) 17 May 2019 (2019-05-17) claims 1-18, and description, paragraphs [0024], [0104]-[0113], [0130], [0131], [0142]-[0144], [0184]-[0187] and [0203]-[0213] | 1, 4-6, 8, 11-26 |
| Y | CN 107667118 A (GANYMED PHARMACEUTICALS AG et al.) 06 February 2018 (2018-02-06) claims 1-68 | 1, 4-6, 8, 11-26 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **25 February 2021** | **10 March 2021** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088**<br>**China** | |
| Facsimile No. **(86-10)62019451** | Telephone No. |

Form PCT/ISA/210 (second sheet) (January 2015)

**INTERNATIONAL SEARCH REPORT**

International application No.
**PCT/CN2020/135680**

**C. DOCUMENTS CONSIDERED TO BE RELEVANT**

| Category* | Citation of document, with indication, where appropriate, of the relevant passages | Relevant to claim No. |
|---|---|---|
| PY | WO 2019/242505 A1 (SHANGHAI L&L BIOPHARMA CO., LTD.) 26 December 2019 (2019-12-26)<br>claims 1-34 | 1, 4-6, 8, 11-26 |
| PY | CN 111110862 A (SHANGHAI L&L BIOPHARMA CO., LTD.) 08 May 2020 (2020-05-08)<br>claims 1-19 | 1, 4-6, 8, 11-26 |
| PY | CN 111689980 A (SICHUAN BAILI PHARMACEUTICAL CO., LTD.) 22 September 2020 (2020-09-22)<br>claims 1-10 | 1-26 |
| PY | WO 2020/063673 A1 (JIANGSU HENGRUI MEDICINE CO., LTD. et al.) 02 April 2020 (2020-04-02)<br>claims 1-28 | 1-26 |
| PY | WO 2020/063676 A1 (JIANGSU HENGRUI MEDICINE CO., LTD. et al.) 02 April 2020 (2020-04-02)<br>claims 1-46 | 1-26 |
| PY | WO 2020/244657 A1 (SHANGHAI HANSOH BIOMEDICAL CO., LTD. et al.) 10 December 2020 (2020-12-10)<br>claims 16-35 | 1-26 |
| PY | WO 2020/200196 A1 (JIANGSU HENGRUI MEDICINE CO., LTD. et al.) 08 October 2020 (2020-10-08)<br>claims 1-17 | 1-26 |
| A | CN 108101825 A (SHANGHAI QINGRUN PHARMACEUTICAL TECHNOLOGY CO., LTD.) 01 June 2018 (2018-06-01)<br>entire document | 1-26 |
| A | CN 109106951 A (SICHUAN BAILI PHARMACEUTICAL CO., LTD.) 01 January 2019 (2019-01-01)<br>entire document | 1-26 |

Form PCT/ISA/210 (second sheet) (January 2015)

**INTERNATIONAL SEARCH REPORT**

International application No.
**PCT/CN2020/135680**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed:
      - ☑ in the form of an Annex C/ST.25 text file.
      - ☐ on paper or in the form of an image file.

   b. ☐ furnished together with the international application under PCT Rule 13*ter*.1(a) for the purposes of international search only in the form of an Annex C/ST.25 text file.

   c. ☐ furnished subsequent to the international filing date for the purposes of international search only:
      - ☐ in the form of an Annex C/ST.25 text file (Rule 13*ter*.1(a)).
      - ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

Form PCT/ISA/210 (continuation of first sheet) (January 2015)

**INTERNATIONAL SEARCH REPORT**
**Information on patent family members**

International application No.
**PCT/CN2020/135680**

| Patent document cited in search report | | | Publication date (day/month/year) | Patent family member(s) | | | Publication date (day/month/year) |
|---|---|---|---|---|---|---|---|
| CN | 104755494 | A | 01 July 2015 | MX | 2015003903 | A | 17 July 2015 |
| | | | | TW | 201920098 | A | 01 June 2019 |
| | | | | NZ | 705394 | A | 26 October 2018 |
| | | | | PL | 2907824 | T3 | 31 August 2018 |
| | | | | DK | 2907824 | T3 | 23 July 2018 |
| | | | | KR | 20190135559 | A | 06 December 2019 |
| | | | | JP | 5953378 | B2 | 20 July 2016 |
| | | | | TW | I615152 | B | 21 February 2018 |
| | | | | AU | 2013328111 | B2 | 02 November 2017 |
| | | | | IL | 271760 | D0 | 27 February 2020 |
| | | | | CN | 104755494 | B | 07 September 2018 |
| | | | | EP | 2907824 | B1 | 11 April 2018 |
| | | | | MX | 364484 | B | 29 April 2019 |
| | | | | WO | 2014057687 | A1 | 17 April 2014 |
| | | | | JP | 6371452 | B2 | 08 August 2018 |
| | | | | CA | 3021435 | A1 | 17 April 2014 |
| | | | | JP | 2018008982 | A | 18 January 2018 |
| | | | | US | 2016279259 | A1 | 29 September 2016 |
| | | | | RU | 2018128384 | A | 02 October 2018 |
| | | | | JP | WO2014057687 | A1 | 05 September 2016 |
| | | | | EP | 3342785 | B1 | 25 December 2019 |
| | | | | US | 2019008981 | A1 | 10 January 2019 |
| | | | | KR | 20180030734 | A | 23 March 2018 |
| | | | | NZ | 746440 | A | 29 November 2019 |
| | | | | US | 2020282073 | A1 | 10 September 2020 |
| | | | | US | 10195288 | B2 | 05 February 2019 |
| | | | | AU | 2018200308 | A1 | 01 February 2018 |
| | | | | NZ | 740948 | A | 29 November 2019 |
| | | | | AU | 2020200548 | A1 | 13 February 2020 |
| | | | | PH | 12015500667 | A1 | 18 May 2015 |
| | | | | TW | I696611 | B | 21 June 2020 |
| | | | | TW | I650312 | B | 11 February 2019 |
| | | | | HK | 1256631 | A1 | 27 September 2019 |
| | | | | ES | 2671644 | T3 | 07 June 2018 |
| | | | | HK | 1210477 | A1 | 22 April 2016 |
| | | | | CA | 2885800 | C | 04 December 2018 |
| | | | | IL | 238143 | D0 | 31 May 2015 |
| | | | | RS | 60000 | B1 | 30 April 2020 |
| | | | | PH | 12018501433 | A1 | 27 February 2019 |
| | | | | PT | 2907824 | T | 07 June 2018 |
| | | | | EP | 2907824 | A4 | 08 June 2016 |
| | | | | KR | 101901558 | B1 | 21 September 2018 |
| | | | | NZ | 746439 | A | 29 November 2019 |
| | | | | US | 9808537 | B2 | 07 November 2017 |
| | | | | CN | 109081871 | A | 25 December 2018 |
| | | | | TR | 201809636 | T4 | 23 July 2018 |
| | | | | RS | 57278 | B1 | 31 August 2018 |
| | | | | US | 2015297748 | A1 | 22 October 2015 |
| | | | | RU | 2664465 | C2 | 17 August 2018 |
| CN | 109762067 | A | 17 May 2019 | EP | 3683239 | A1 | 22 July 2020 |

Form PCT/ISA/210 (patent family annex) (January 2015)

**INTERNATIONAL SEARCH REPORT**
**Information on patent family members**

International application No.
**PCT/CN2020/135680**

| Patent document cited in search report | | | Publication date (day/month/year) | Patent family member(s) | | | Publication date (day/month/year) |
|---|---|---|---|---|---|---|---|
| | | | | WO | 2020147321 | A1 | 23 July 2020 |
| | | | | US | 10421817 | B1 | 24 September 2019 |
| | | | | CN | 109762067 | B | 28 February 2020 |
| CN | 107667118 | A | 06 February 2018 | ZA | 201705923 | B | 26 June 2019 |
| | | | | RU | 2017139490 | A3 | 17 January 2020 |
| | | | | MX | 2017013075 | A | 19 February 2018 |
| | | | | KR | 20180082325 | A | 18 July 2018 |
| | | | | EP | 3283521 | A1 | 21 February 2018 |
| | | | | US | 2018117174 | A1 | 03 May 2018 |
| | | | | HK | 1247210 | A1 | 21 September 2018 |
| | | | | CA | 2982401 | A1 | 20 October 2016 |
| | | | | IL | 254085 | D0 | 31 October 2017 |
| | | | | WO | 2016166122 | A1 | 20 October 2016 |
| | | | | AU | 2016249782 | A1 | 02 November 2017 |
| | | | | WO | 2016165762 | A1 | 20 October 2016 |
| | | | | RU | 2017139490 | A | 15 May 2019 |
| | | | | BR | 112017018521 | A2 | 17 April 2018 |
| | | | | JP | 2018513146 | A | 24 May 2018 |
| | | | | SG | 11201708271 T | A | 29 November 2017 |
| WO | 2019/242505 | A1 | 26 December 2019 | CN | 111867630 | A | 30 October 2020 |
| CN | 111110862 | A | 08 May 2020 | | None | | |
| CN | 111689980 | A | 22 September 2020 | | None | | |
| WO | 2020/063673 | A1 | 02 April 2020 | TW | 202028242 | A | 01 August 2020 |
| WO | 2020/063676 | A1 | 02 April 2020 | TW | 202027795 | A | 01 August 2020 |
| WO | 2020/244657 | A1 | 10 December 2020 | | None | | |
| WO | 2020/200196 | A1 | 08 October 2020 | | None | | |
| CN | 108101825 | A | 01 June 2018 | | None | | |
| CN | 109106951 | A | 01 January 2019 | WO | 2019034176 | A1 | 21 February 2019 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 201911273041
- CN 202011060513
- WO 2020200196 A1
- WO 2016166122 A
- WO 2016165762 A
- US 20050238649 A1
- US 5208020 A
- CN 2019107873 W
- EP 0737686 A1
- WO 2013106717 A
- WO 201396771 A
- US 200520645 B
- CN 105829346 A
- EP 2907824 A


### Non-patent literature cited in the description


- **CHARI et al.** *Cancer Research,* 1992, vol. 52, 127-131
- *J. biol. chem,* 1968, vol. 243, 3558
- **WARD et al.** *Nature,* 1989, vol. 341, 544-546
- **BIRD et al.** *Science,* 1988, vol. 242, 423-426
- **HUSTON et al.** *Proc. Natl. Acad. Sci USA,* 1988, vol. 85, 5879-5883
- **HOLLIGER et al.** *Proc. Natl. Acad. Sci. USA,* 1993, vol. 90, 6444-6448
- **ALFTHAN et al.** *Protein Eng.,* 1995, vol. 8, 725-731
- **CHOI et al.** *Eur. J. Immunol.,* 2001, vol. 31, 94-106
- **HU et al.** *Cancer Res.,* 1996, vol. 56, 3055-3061
- **KIPRIYANOV et al.** *J. Mol. Biol.,* 1999, vol. 293, 41-56
- **ROOVERS et al.** *Cancer Immunol.,* 2001
- **KABAT E.A. et al.** Sequences of proteins of immunological interest. NIH Publication, 1991, 91-3242
- **MARTIN, ACR.** *Protein Sequence and Structure Analysis of Antibody Variable Domains[J,* 2001
- **LEFRANC M.P.** *Dev. Comp. Immunol.,* 2003, vol. 27, 55-77
- Epitope Mapping Protocols in Methods in Molecular B iology. 1996, vol. 66
- Antibodies: A Laboratory Manual. Cold Spring Harbor Press
- **LEFRANC, G.** the Immunoglobulin FactsBook. Academic Press, 2001
- *Tetrahedron Letters,* 1984, vol. 25 (12), 1269-72
- *Journal of the American Chemical Society,* 2014, vol. 136 (22), 8138-8142

SEQUENCE LISTING

<110> Jiangsu Hengrui Medicine Co., Ltd.
Shanghai Hengrui Pharmaceutical Co., Ltd.

<120> ANTI-CLAUDIN ANTIBODY-DRUG CONJUGATE AND PHARMACEUTICAL USE THEREOF

<130> 702137CPCT

<140> PCT/CN2020/135680
<141> 2020-12-11

<150> CN201911273041.7
<151> 2019-12-12

<150> CN202011060513.3
<151> 2020-09-30

<160> 55

<170> Patent-In 3.5

<210> 1
<211> 261
<212> PRT
<213> Homo sapiens

<400> 1
Met Ala Val Thr Ala Cys Gln Gly Leu Gly Phe Val Val Ser Leu Ile
1 5 10 15
Gly Ile Ala Gly Ile Ile Ala Ala Thr Cys Met Asp Gln Trp Ser Thr
20 25 30
Gln Asp Leu Tyr Asn Asn Pro Val Thr Ala Val Phe Asn Tyr Gln Gly
35 40 45
Leu Trp Arg Ser Cys Val Arg Glu Ser Ser Gly Phe Thr Glu Cys Arg
50 55 60
Gly Tyr Phe Thr Leu Leu Gly Leu Pro Ala Met Leu Gln Ala Val Arg
65 70 75 80
Ala Leu Met Ile Val Gly Ile Val Leu Gly Ala Ile Gly Leu Leu Val
85 90 95
Ser Ile Phe Ala Leu Lys Cys Ile Arg Ile Gly Ser Met Glu Asp Ser
100 105 110
Ala Lys Ala Asn Met Thr Leu Thr Ser Gly Ile Met Phe Ile Val Ser
115 120 125
Gly Leu Cys Ala Ile Ala Gly Val Ser Val Phe Ala Asn Met Leu Val
130 135 140
Thr Asn Phe Trp Met Ser Thr Ala Asn Met Tyr Thr Gly Met Gly Gly
145 150 155 160
Met Val Gln Thr Val Gln Thr Arg Tyr Thr Phe Gly Ala Ala Leu Phe
165 170 175
Val Gly Trp Val Ala Gly Gly Leu Thr Leu Ile Gly Gly Val Met Met
180 185 190
Cys Ile Ala Cys Arg Gly Leu Ala Pro Glu Glu Thr Asn Tyr Lys Ala
195 200 205
Val Ser Tyr His Ala Ser Gly His Ser Val Ala Tyr Lys Pro Gly Gly
210 215 220
Phe Lys Ala Ser Thr Gly Phe Gly Ser Asn Thr Lys Asn Lys Lys Ile
225 230 235 240
Tyr Asp Gly Gly Ala Arg Thr Glu Asp Glu Val Gln Ser Tyr Pro Ser
245 250 255
Lys His Asp Tyr Val
260

<210> 2
<211> 3350
<212> DNA
<213> Homo sapiens

<400> 2
agaattgcgc tgtccacttg tcgtgtggct ctgtgtcgac actgtgcgcc accatggccg 60
tgactgcctg tcagggcttg gggttcgtgg tttcactgat tgggattgcg ggcatcattg 120
ctgccacctg catggaccag tggagcaccc aagacttgta caacaacccc gtaacagctg 180
ttttcaacta ccagggggctg tggcgctcct gtgtccgaga gagctctggc ttcaccgagt 240
gccggggcta cttcaccctg ctggggctgc cagccatgct gcaggcagtg cgagccctga 300
tgatcgtagg catcgtcctg ggtgccattg gcctcctggt atccatcttt gccctgaaat 360
gcatccgcat tggcagcatg gaggactctg ccaaagccaa catgacactg acctccggga 420
tcatgttcat tgtctcaggt ctttgtgcaa ttgctggagt gtctgtgttt gccaacatgc 480
tggtgactaa cttctggatg tccacagcta acatgtacac cggcatgggt gggatggtgc 540
agactgttca gaccaggtac acatttggtg cggctctgtt cgtgggctgg gtcgctggag 600
gcctcacact aattgggggt gtgatgatgt gcatcgcctg ccggggcctg gcaccagaag 660
aaaccaacta caaagccgtt tcttatcatg cctcaggcca cagtgttgcc tacaagcctg 720
gaggcttcaa ggccagcact ggctttgggt ccaacaccaa aaacaagaag atatacgatg 780
gaggtgcccg cacagaggac gaggtacaat cttatccttc caagcacgac tatgtgtaat 840
gctctaagac ctctcagcac gggcggaaga aactcccgga gagctcaccc aaaaaacaag 900
gagatcccat ctagatttct tcttgctttt gactcacagc tggaagttag aaaagcctcg 960
atttcatctt tggagaggcc aaatggtctt agcctcagtc tctgtctcta aatattccac 1020
cataaaacag ctgagttatt tatgaattag aggctatagc tcacattttc aatcctctat 1080
ttcttttttt aaatataact ttctactctg atgagagaat gtggttttaa tctctctctc 1140
acattttgat gatttagaca gactccccct cttcctccta gtcaataaac ccattgatga 1200
tctatttccc agcttatccc caagaaaact tttgaaagga aagagtagac ccaaagatgt 1260
tattttctgc tgtttgaatt ttgtctcccc acccccaact tggctagtaa taaacactta 1320
ctgaagaaga agcaataaga gaaagatatt tgtaatctct ccagcccatg atctcggttt 1380
tcttacactg tgatcttaaa agttaccaaa ccaaagtcat tttcagtttg aggcaaccaa 1440
acctttctac tgctgttgac atcttcttat tacagcaaca ccattctagg agtttcctga 1500
gctctccact ggagtcctct ttctgtcgcg ggtcagaaat tgtccctaga tgaatgagaa 1560
aattattttt tttaatttaa gtcctaaata tagttaaaat aaataatgtt ttagtaaaat 1620
gatacactat ctctgtgaaa tagcctcacc cctacatgtg gatagaagga aatgaaaaaa 1680
taattgcttt gacattgtct atatggtact ttgtaaagtc atgcttaagt acaaattcca 1740
tgaaaagctc actgatccta attctttccc tttgaggtct ctatggctct gattgtacat 1800
gatagtaagt gtaagccatg taaaaagtaa ataatgtctg ggcacagtgg ctcacgcctg 1860
taatcctagc actttgggag gctgaggagg aaggatcact tgagcccaga agttcgagac 1920
tagcctgggc aacatggaga agccctgtct ctacaaaata cagagagaaa aaatcagcca 1980
gtcatggtgg cctacacctg tagtcccagc attccgggag gctgaggtgg gaggatcact 2040
tgagcccagg gaggttgggg ctgcagtgag ccatgatcac accactgcac tccagccagg 2100
tgacatagcg agatcctgtc taaaaaaata aaaaataaat aatggaacac agcaagtcct 2160
aggaagtagg ttaaaactaa ttctttaaaa aaaaaaaaaa gttgagcctg aattaaatgt 2220
aatgtttcca agtgacaggt atccacattt gcatggttac aagccactgc cagttagcag 2280
tagcactttc ctggcactgt ggtcggtttt gttttgtttt gctttgttta gagacggggt 2340
ctcactttcc aggctggcct caaactcctg cactcaagca attcttctac cctggcctcc 2400
caagtagctg gaattacagg tgtgcgccat cacaactagc tggtggtcag ttttgttact 2460
ctgagagctg ttcacttctc tgaattcacc tagagtggtt ggaccatcag atgtttgggc 2520
aaaactgaaa gctctttgca accacacacc ttccctgagc ttacatcact gccctttga 2580
gcagaaagtc taaattcctt ccaagacagt agaattccat cccagtacca aagccagata 2640
ggcccccctag gaaactgagg taagagcagt ctctaaaaac tacccacagc agcattggtg 2700
caggggaact tggccattag gttattattt gagaggaaag tcctcacatc aatagtacat 2760
atgaaagtga cctccaaggg gattggtgaa tactcataag gatcttcagg ctgaacagac 2820
tatgtctggg gaaagaacgg attatgcccc attaaataac aagttgtgtt caagagtcag 2880
agcagtgagc tcagaggccc ttctcactga gacagcaaca tttaaaccaa accagaggaa 2940
gtatttgtgg aactcactgc ctcagtttgg gtaaaggatg agcagacaag tcaactaaag 3000
aaaaaagaaa agcaaggagg agggttgagc aatctagagc atggagtttg ttaagtgctc 3060
tctggatttg agttgaagag catccatttg agttgaaggc cacagggcac aatgagctct 3120
cccttctacc accagaaagt ccctggtcag gtctcaggta gtgcggtgtg gctcagctgg 3180
gtttttaatt agcgcattct ctatccaaca tttaattgtt tgaaagcctc catatagtta 3240
gattgtgctt tgtaattttg ttgttgttgc tctatcttat tgtatatgca ttgagtatta 3300
acctgaatgt tttgttactt aaatattaaa aacactgtta tcctacagtt 3350

<210> 3
<211> 120
<212> PRT
<213> Mus musculus

<220>
<223> Heavy chain variable region of mAb1901 murine antibody

<400> 3
```
Glu Val Gln Leu Met Glu Ser Gly Gly Gly Leu Val Lys Pro Gly Gly
1               5                   10                  15
Ser Leu Lys Leu Ser Cys Ala Ala Ser Gly Phe Thr Phe Ser Asp Tyr
            20                  25                  30
Gly Ile His Trp Val Arg Gln Ala Pro Glu Met Gly Leu Glu Trp Ile
        35                  40                  45
Ala Tyr Ile Ser Arg Gly Ser Ser Thr Ile Tyr Tyr Ala Asp Thr Val
    50                  55                  60
Lys Gly Arg Phe Thr Met Ser Arg Asp Asn Ala Lys Asn Thr Leu Phe
65                  70                  75                  80
Leu Gln Met Thr Ser Leu Arg Ser Glu Asp Thr Ala Met Tyr Tyr Cys
                85                  90                  95
Ala Arg Gly Gly Tyr Asp Thr Arg Asn Ala Met Asp Tyr Trp Gly Gln
            100                 105                 110
Gly Thr Ser Val Thr Val Ser Ser
        115                 120
```

<210> 4
<211> 113
<212> PRT
<213> Mus musculus

<220>
<223> Light chain variable region of mAb1901 murine antibody

<400> 4
```
Asp Ile Val Met Thr Gln Ser Pro Ser Ser Leu Ser Val Ser Ala Gly
1               5                   10                  15
Glu Lys Val Thr Met Ser Cys Lys Ser Ser Gln Ser Leu Leu Asn Ser
            20                  25                  30
Gly Asn Gln Lys Asn Tyr Leu Ala Trp Tyr Gln Gln Lys Pro Gly Gln
        35                  40                  45
Pro Pro Lys Leu Leu Ile Tyr Gly Ala Ser Thr Arg Ala Ser Gly Val
    50                  55                  60
Pro Asp Arg Phe Thr Gly Ser Gly Ser Gly Thr Asp Phe Thr Leu Thr
65                  70                  75                  80
Ile Ser Ser Val Gln Ala Glu Asp Leu Ala Ile Tyr His Cys Gln Asn
                85                  90                  95
Asp Leu Tyr Tyr Pro Leu Thr Phe Gly Ala Gly Thr Lys Leu Glu Leu
            100                 105                 110
Lys
```

<210> 5
<211> 118
<212> PRT
<213> Mus musculus

<220>
<223> Heavy chain variable region of mAb1902 murine antibody

<400> 5
Glu Val Gln Leu Gln Glu Ser Gly Ala Glu Leu Val Lys Pro Gly Ala
1 5 10 15
Ser Val Lys Leu Ser Cys Lys Ala Ser Gly Tyr Ile Phe Thr Ser Tyr
20 25 30
Trp Met His Trp Val Lys Gln Arg Pro Gly Gln Gly Leu Glu Trp Ile
35 40 45
Gly Met Ile His Pro Asn Ser Gly Ser Thr Asn Tyr Asn Glu Lys Phe
50 55 60
Lys Gly Lys Ala Thr Leu Thr Leu Asp Lys Ser Ser Ser Thr Ala Tyr
65 70 75 80
Met Gln Leu Ser Ser Leu Pro Ser Glu Asp Ser Ala Val Tyr Tyr Cys
85 90 95
Ala Arg Leu Lys Thr Gly Asn Ser Phe Asp Tyr Trp Gly Gln Gly Thr
100 105 110
Thr Leu Thr Val Ser Ser
115

<210> 6
<211> 113
<212> PRT
<213> Mus musculus

<220>
<223> Light chain variable region of mAb1902 murine antibody

<400> 6
Asp Ile Val Leu Thr Gln Ser Pro Ser Ser Leu Thr Val Thr Ala Gly
1 5 10 15
Glu Lys Val Thr Met Ser Cys Lys Ser Ser Gln Ser Leu Leu Asn Ser
20 25 30
Gly Asn Gln Lys Asn Tyr Leu Thr Trp Tyr Gln Gln Lys Pro Gly Gln
35 40 45
Pro Pro Lys Leu Leu Ile Tyr Trp Ala Ser Thr Arg Glu Ser Gly Val
50 55 60
Pro Asp Arg Phe Thr Gly Ser Gly Ser Gly Thr Asp Phe Thr Leu Thr
65 70 75 80
Ile Ser Ser Val Gln Ala Glu Asp Leu Ala Ile Tyr Tyr Cys Gln Asn
85 90 95
Ala Tyr Thr Tyr Pro Phe Thr Phe Gly Ser Gly Thr Lys Leu Glu Ile
100 105 110
Lys

<210> 7
<211> 330
<212> PRT
<213> Homo sapiens

<220>
<223> Heavy chain constant region of human IgG1 antibody

<400> 7
Ala Ser Thr Lys Gly Pro Ser Val Phe Pro Leu Ala Pro Ser Ser Lys
1 5 10 15
Ser Thr Ser Gly Gly Thr Ala Ala Leu Gly Cys Leu Val Lys Asp Tyr
20 25 30
Phe Pro Glu Pro Val Thr Val Ser Trp Asn Ser Gly Ala Leu Thr Ser
35 40 45
Gly Val His Thr Phe Pro Ala Val Leu Gln Ser Ser Gly Leu Tyr Ser
50 55 60

```
Leu Ser Ser Val Val Thr Val Pro Ser Ser Ser Leu Gly Thr Gln Thr
65                  70                  75                  80
Tyr Ile Cys Asn Val Asn His Lys Pro Ser Asn Thr Lys Val Asp Lys
                85                  90                  95
Lys Val Glu Pro Lys Ser Cys Asp Lys Thr His Thr Cys Pro Pro Cys
            100                 105                 110
Pro Ala Pro Glu Leu Leu Gly Gly Pro Ser Val Phe Leu Phe Pro Pro
        115                 120                 125
Lys Pro Lys Asp Thr Leu Met Ile Ser Arg Thr Pro Glu Val Thr Cys
    130                 135                 140
Val Val Val Asp Val Ser His Glu Asp Pro Glu Val Lys Phe Asn Trp
145                 150                 155                 160
Tyr Val Asp Gly Val Glu Val His Asn Ala Lys Thr Lys Pro Arg Glu
                165                 170                 175
Glu Gln Tyr Asn Ser Thr Tyr Arg Val Val Ser Val Leu Thr Val Leu
            180                 185                 190
His Gln Asp Trp Leu Asn Gly Lys Glu Tyr Lys Cys Lys Val Ser Asn
        195                 200                 205
Lys Ala Leu Pro Ala Pro Ile Glu Lys Thr Ile Ser Lys Ala Lys Gly
    210                 215                 220
Gln Pro Arg Glu Pro Gln Val Tyr Thr Leu Pro Pro Ser Arg Asp Glu
225                 230                 235                 240
Leu Thr Lys Asn Gln Val Ser Leu Thr Cys Leu Val Lys Gly Phe Tyr
                245                 250                 255
Pro Ser Asp Ile Ala Val Glu Trp Glu Ser Asn Gly Gln Pro Glu Asn
            260                 265                 270
Asn Tyr Lys Thr Thr Pro Pro Val Leu Asp Ser Asp Gly Ser Phe Phe
        275                 280                 285
Leu Tyr Ser Lys Leu Thr Val Asp Lys Ser Arg Trp Gln Gln Gly Asn
    290                 295                 300
Val Phe Ser Cys Ser Val Met His Glu Ala Leu His Asn His Tyr Thr
305                 310                 315                 320
Gln Lys Ser Leu Ser Leu Ser Pro Gly Lys
                325                 330
```

<210> 8
<211> 107
<212> PRT
<213> Homo sapiens

<220>
<223> Human antibody kappa light chain constant region

<400> 8

```
Arg Thr Val Ala Ala Pro Ser Val Phe Ile Phe Pro Pro Ser Asp Glu
1               5                   10                  15
Gln Leu Lys Ser Gly Thr Ala Ser Val Val Cys Leu Leu Asn Asn Phe
            20                  25                  30
Tyr Pro Arg Glu Ala Lys Val Gln Trp Lys Val Asp Asn Ala Leu Gln
        35                  40                  45
Ser Gly Asn Ser Gln Glu Ser Val Thr Glu Gln Asp Ser Lys Asp Ser
    50                  55                  60
Thr Tyr Ser Leu Ser Ser Thr Leu Thr Leu Ser Lys Ala Asp Tyr Glu
65                  70                  75                  80
Lys His Lys Val Tyr Ala Cys Glu Val Thr His Gln Gly Leu Ser Ser
                85                  90                  95
Pro Val Thr Lys Ser Phe Asn Arg Gly Glu Cys
            100                 105
```

<210> 9
<211> 5
<212> PRT

<213> Artificial sequence

<220>
<223> mAb1901 HCDR1

<400> 9
Asp Tyr Gly Ile His
1 5

<210> 10
<211> 17
<212> PRT
<213> Artificial sequence

<220>
<223> mAb1901 HCDR2

<400> 10
Tyr Ile Ser Arg Gly Ser Ser Thr Ile Tyr Tyr Ala Asp Thr Val Lys
1 5 10 15
Gly

<210> 11
<211> 11
<212> PRT
<213> Artificial sequence

<220>
<223> mAb1901 HCDR3

<400> 11
Gly Gly Tyr Asp Thr Arg Asn Ala Met Asp Tyr
1 5 10

<210> 12
<211> 17
<212> PRT
<213> Artificial sequence

<220>
<223> mAb1901 LCDR1

<400> 12
Lys Ser Ser Gln Ser Leu Leu Asn Ser Gly Asn Gln Lys Asn Tyr Leu
1 5 10 15
Ala

<210> 13
<211> 7
<212> PRT
<213> Artificial sequence

<220>
<223> mAb1901 LCDR2

<400> 13

Gly Ala Ser Thr Arg Ala Ser
1               5

<210>  14
<211>  9
<212>  PRT
<213>  Artificial sequence

<220>
<223>  mAb1901 LCDR3

<400>  14
Gln Asn Asp Leu Tyr Tyr Pro Leu Thr
1               5

<210>  15
<211>  5
<212>  PRT
<213>  Artificial sequence

<220>
<223>  mAb1902 HCDR1

<400>  15
Ser Tyr Trp Met His
1               5

<210>  16
<211>  18
<212>  PRT
<213>  Artificial sequence

<220>
<223>  mAb1902 HCDR2

<400>  16
Met Ile His Pro Asn Ser Gly Ser Thr Asn Tyr Asn Glu Lys Phe Lys
1               5                   10                  15
Gly Arg

<210>  17
<211>  9
<212>  PRT
<213>  Artificial sequence

<220>
<223>  mAb1902 HCDR3

<400>  17
Leu Lys Thr Gly Asn Ser Phe Asp Tyr
1               5

<210>  18
<211>  17
<212>  PRT
<213>  Artificial sequence

<220>

<223> mAb1902 LCDR1

<400> 18
Lys Ser Ser Gln Ser Leu Leu Asn Ser Gly Asn Gln Lys Asn Tyr Leu
1 5 10 15
Thr

<210> 19
<211> 7
<212> PRT
<213> Artificial sequence

<220>
<223> mAb1902 LCDR2

<400> 19
Trp Ala Ser Thr Arg Glu Ser
1 5

<210> 20
<211> 9
<212> PRT
<213> Artificial sequence

<220>
<223> mAb1902 LCDR3

<400> 20
Gln Asn Ala Tyr Thr Tyr Pro Phe Thr
1 5

<210> 21
<211> 113
<212> PRT
<213> Artificial sequence

<220>
<223> VL1

<400> 21
Asp Ile Val Met Thr Gln Ser Pro Asp Ser Leu Ala Val Ser Leu Gly
1 5 10 15
Glu Arg Ala Thr Ile Asn Cys Lys Ser Ser Gln Ser Leu Leu Asn Ser
20 25 30
Gly Asn Gln Lys Asn Tyr Leu Ala Trp Tyr Gln Gln Lys Pro Gly Gln
35 40 45
Pro Pro Lys Leu Leu Ile Tyr Gly Ala Ser Thr Arg Ala Ser Gly Val
50 55 60
Pro Asp Arg Phe Ser Gly Ser Gly Ser Gly Thr Asp Phe Thr Leu Thr
65 70 75 80
Ile Ser Ser Leu Gln Ala Glu Asp Val Ala Val Tyr Tyr Cys Gln Asn
85 90 95
Asp Leu Tyr Tyr Pro Leu Thr Phe Gly Gln Gly Thr Lys Leu Glu Ile
100 105 110
Lys

<210> 22

<211> 113
<212> PRT
<213> Artificial sequence

<220>
<223> VL2

<400> 22
Asp Ile Val Met Thr Gln Ser Pro Asp Ser Leu Ala Val Ser Leu Gly
1 5 10 15
Glu Arg Ala Thr Ile Ser Cys Lys Ser Ser Gln Ser Leu Leu Asn Ser
20 25 30
Gly Asn Gln Lys Asn Tyr Leu Ala Trp Tyr Gln Gln Lys Pro Gly Gln
35 40 45
Pro Pro Lys Leu Leu Ile Tyr Gly Ala Ser Thr Arg Ala Ser Gly Val
50 55 60
Pro Asp Arg Phe Ser Gly Ser Gly Ser Gly Thr Asp Phe Thr Leu Thr
65 70 75 80
Ile Ser Ser Leu Gln Ala Glu Asp Val Ala Val Tyr Tyr Cys Gln Asn
85 90 95
Asp Leu Tyr Tyr Pro Leu Thr Phe Gly Gln Gly Thr Lys Leu Glu Ile
100 105 110
Lys

<210> 23
<211> 113
<212> PRT
<213> Artificial sequence

<220>
<223> VL3

<400> 23
Asp Ile Val Met Thr Gln Ser Pro Asp Ser Leu Ala Val Ser Leu Gly
1 5 10 15
Glu Arg Ala Thr Ile Ser Cys Lys Ser Ser Gln Ser Leu Leu Asn Ser
20 25 30
Gly Asn Gln Lys Asn Tyr Leu Ala Trp Tyr Gln Gln Lys Pro Gly Gln
35 40 45
Pro Pro Lys Leu Leu Ile Tyr Gly Ala Ser Thr Arg Ala Ser Gly Val
50 55 60
Pro Asp Arg Phe Ser Gly Ser Gly Ser Gly Thr Asp Phe Thr Leu Thr
65 70 75 80
Ile Ser Ser Leu Gln Ala Glu Asp Val Ala Ile Tyr His Cys Gln Asn
85 90 95
Asp Leu Tyr Tyr Pro Leu Thr Phe Gly Gln Gly Thr Lys Leu Glu Ile
100 105 110
Lys

<210> 24
<211> 120
<212> PRT
<213> Artificial sequence

<220>
<223> VH1

<400> 24

Glu Val Gln Leu Val Glu Ser Gly Gly Gly Leu Val Gln Pro Gly Gly
1 5 10 15
Ser Leu Arg Leu Ser Cys Ala Ala Ser Gly Phe Thr Phe Ser Asp Tyr
20 25 30
Gly Ile His Trp Val Arg Gln Ala Pro Gly Lys Gly Leu Glu Trp Val
35 40 45
Ala Tyr Ile Ser Arg Gly Ser Ser Thr Ile Tyr Tyr Ala Asp Thr Val
50 55 60
Lys Gly Arg Phe Thr Ile Ser Arg Asp Asn Ala Lys Asn Ser Leu Tyr
65 70 75 80
Leu Gln Met Asn Ser Leu Arg Ala Glu Asp Thr Ala Val Tyr Tyr Cys
85 90 95
Ala Arg Gly Gly Tyr Asp Thr Arg Asn Ala Met Asp Tyr Trp Gly Gln
100 105 110
Gly Thr Thr Val Thr Val Ser Ser
115 120

<210> 25
<211> 120
<212> PRT
<213> Artificial sequence

<220>
<223> VH2

<400> 25
Glu Val Gln Leu Val Glu Ser Gly Gly Gly Leu Val Gln Pro Gly Gly
1 5 10 15
Ser Leu Arg Leu Ser Cys Ala Ala Ser Gly Phe Thr Phe Ser Asp Tyr
20 25 30
Gly Ile His Trp Val Arg Gln Ala Pro Gly Lys Gly Leu Glu Trp Val
35 40 45
Ala Tyr Ile Ser Arg Gly Ser Ser Thr Ile Tyr Tyr Ala Asp Thr Val
50 55 60
Lys Gly Arg Phe Thr Ile Ser Arg Asp Asn Ala Lys Asn Ser Leu Tyr
65 70 75 80
Leu Gln Met Thr Ser Leu Arg Ala Glu Asp Thr Ala Val Tyr Tyr Cys
85 90 95
Ala Arg Gly Gly Tyr Asp Thr Arg Asn Ala Met Asp Tyr Trp Gly Gln
100 105 110
Gly Thr Thr Val Thr Val Ser Ser
115 120

<210> 26
<211> 120
<212> PRT
<213> Artificial sequence

<220>
<223> VH3

<400> 26
Glu Val Gln Leu Val Glu Ser Gly Gly Gly Leu Val Gln Pro Gly Gly
1 5 10 15
Ser Leu Arg Leu Ser Cys Ala Ala Ser Gly Phe Thr Phe Ser Asp Tyr
20 25 30
Gly Ile His Trp Val Arg Gln Ala Pro Gly Lys Gly Leu Glu Trp Ile
35 40 45
Ala Tyr Ile Ser Arg Gly Ser Ser Thr Ile Tyr Tyr Ala Asp Thr Val
50 55 60
Lys Gly Arg Phe Thr Ile Ser Arg Asp Asn Ala Lys Asn Ser Leu Tyr

```
65                  70                  75                  80
Leu Gln Met Thr Ser Leu Arg Ala Glu Asp Thr Ala Val Tyr Tyr Cys
                85                  90                  95
Ala Arg Gly Gly Tyr Asp Thr Arg Asn Ala Met Asp Tyr Trp Gly Gln
            100                 105                 110
Gly Thr Thr Val Thr Val Ser Ser
        115                 120

<210>  27
<211>  120
<212>  PRT
<213>  Artificial sequence

<220>
<223>  VH4


<400>  27
Glu Val Gln Leu Val Glu Ser Gly Gly Gly Leu Val Gln Pro Gly Gly
1               5                   10                  15
Ser Leu Arg Leu Ser Cys Ala Ala Ser Gly Phe Thr Phe Ser Asp Tyr
            20                  25                  30
Gly Ile His Trp Val Arg Gln Ala Pro Gly Lys Gly Leu Glu Trp Val
        35                  40                  45
Ala Tyr Ile Ser Arg Gly Ser Ser Thr Ile Tyr Tyr Ala Asp Thr Val
    50                  55                  60
Lys Gly Arg Phe Thr Met Ser Arg Asp Asn Ala Lys Asn Ser Leu Tyr
65                  70                  75                  80
Leu Gln Met Thr Ser Leu Arg Ala Glu Asp Thr Ala Val Tyr Tyr Cys
                85                  90                  95
Ala Arg Gly Gly Tyr Asp Thr Arg Asn Ala Met Asp Tyr Trp Gly Gln
            100                 105                 110
Gly Thr Thr Val Thr Val Ser Ser
        115                 120

<210>  28
<211>  113
<212>  PRT
<213>  Artificial sequence

<220>
<223>  VL11


<400>  28
Asp Ile Val Met Thr Gln Ser Pro Asp Ser Leu Ala Val Ser Leu Gly
1               5                   10                  15
Glu Arg Ala Thr Ile Asn Cys Lys Ser Ser Gln Ser Leu Leu Asn Ser
            20                  25                  30
Gly Asn Gln Lys Asn Tyr Leu Thr Trp Tyr Gln Gln Lys Pro Gly Gln
        35                  40                  45
Pro Pro Lys Leu Leu Ile Tyr Trp Ala Ser Thr Arg Glu Ser Gly Val
    50                  55                  60
Pro Asp Arg Phe Ser Gly Ser Gly Ser Gly Thr Asp Phe Thr Leu Thr
65                  70                  75                  80
Ile Ser Ser Leu Gln Ala Glu Asp Val Ala Val Tyr Tyr Cys Gln Asn
                85                  90                  95
Ala Tyr Thr Tyr Pro Phe Thr Phe Gly Gln Gly Thr Lys Leu Glu Ile
            100                 105                 110
Lys


<210>  29
```

<211> 113
<212> PRT
<213> Artificial sequence

<220>
<223> VL12

<400> 29
Asp Ile Val Leu Thr Gln Ser Pro Asp Ser Leu Ala Val Ser Leu Gly
1 5 10 15
Glu Arg Ala Thr Ile Asn Cys Lys Ser Ser Gln Ser Leu Leu Asn Ser
20 25 30
Gly Asn Gln Lys Asn Tyr Leu Thr Trp Tyr Gln Gln Lys Pro Gly Gln
35 40 45
Pro Pro Lys Leu Leu Ile Tyr Trp Ala Ser Thr Arg Glu Ser Gly Val
50 55 60
Pro Asp Arg Phe Ser Gly Ser Gly Ser Gly Thr Asp Phe Thr Leu Thr
65 70 75 80
Ile Ser Ser Leu Gln Ala Glu Asp Val Ala Val Tyr Tyr Cys Gln Asn
85 90 95
Ala Tyr Thr Tyr Pro Phe Thr Phe Gly Gln Gly Thr Lys Leu Glu Ile
100 105 110
Lys

<210> 30
<211> 113
<212> PRT
<213> Artificial sequence

<220>
<223> VL13

<400> 30
Asp Ile Val Leu Thr Gln Ser Pro Asp Ser Leu Ala Val Ser Leu Gly
1 5 10 15
Glu Arg Ala Thr Ile Ser Cys Lys Ser Ser Gln Ser Leu Leu Asn Ser
20 25 30
Gly Asn Gln Lys Asn Tyr Leu Thr Trp Tyr Gln Gln Lys Pro Gly Gln
35 40 45
Pro Pro Lys Leu Leu Ile Tyr Trp Ala Ser Thr Arg Glu Ser Gly Val
50 55 60
Pro Asp Arg Phe Ser Gly Ser Gly Ser Gly Thr Asp Phe Thr Leu Thr
65 70 75 80
Ile Ser Ser Leu Gln Ala Glu Asp Val Ala Val Tyr Tyr Cys Gln Asn
85 90 95
Ala Tyr Thr Tyr Pro Phe Thr Phe Gly Gln Gly Thr Lys Leu Glu Ile
100 105 110
Lys

<210> 31
<211> 118
<212> PRT
<213> Artificial sequence

<220>
<223> VH11

<400> 31

Glu Val Gln Leu Val Gln Ser Gly Ala Glu Val Lys Lys Pro Gly Ala
1               5                   10                  15
Ser Val Lys Val Ser Cys Lys Ala Ser Gly Tyr Thr Phe Thr Ser Tyr
            20                  25                  30
Trp Met His Trp Val Arg Gln Ala Pro Gly Gln Arg Leu Glu Trp Met
        35                  40                  45
Gly Met Ile His Pro Asn Ser Gly Ser Thr Asn Tyr Asn Glu Lys Phe
    50                  55                  60
Lys Gly Arg Val Thr Ile Thr Arg Asp Thr Ser Ala Ser Thr Ala Tyr
65                  70                  75                  80
Met Glu Leu Ser Ser Leu Arg Ser Glu Asp Thr Ala Val Tyr Tyr Cys
                85                  90                  95
Ala Arg Leu Lys Thr Gly Asn Ser Phe Asp Tyr Trp Gly Gln Gly Thr
            100                 105                 110
Thr Val Thr Val Ser Ser
        115

<210> 32
<211> 118
<212> PRT
<213> Artificial sequence

<220>
<223> VH12

<400> 32
Glu Val Gln Leu Val Gln Ser Gly Ala Glu Val Lys Lys Pro Gly Ala
1               5                   10                  15
Ser Val Lys Val Ser Cys Lys Ala Ser Gly Tyr Thr Phe Thr Ser Tyr
            20                  25                  30
Trp Met His Trp Val Arg Gln Ala Pro Gly Gln Arg Leu Glu Trp Met
        35                  40                  45
Gly Met Ile His Pro Asn Ser Gly Ser Thr Asn Tyr Asn Glu Lys Phe
    50                  55                  60
Lys Gly Arg Val Thr Leu Thr Leu Asp Lys Ser Ala Ser Thr Ala Tyr
65                  70                  75                  80
Met Glu Leu Ser Ser Leu Arg Ser Glu Asp Thr Ala Val Tyr Tyr Cys
                85                  90                  95
Ala Arg Leu Lys Thr Gly Asn Ser Phe Asp Tyr Trp Gly Gln Gly Thr
            100                 105                 110
Thr Val Thr Val Ser Ser
        115

<210> 33
<211> 118
<212> PRT
<213> Artificial sequence

<220>
<223> VH13

<400> 33
Glu Val Gln Leu Val Gln Ser Gly Ala Glu Val Lys Lys Pro Gly Ala
1               5                   10                  15
Ser Val Lys Val Ser Cys Lys Ala Ser Gly Tyr Thr Phe Thr Ser Tyr
            20                  25                  30
Trp Met His Trp Val Arg Gln Ala Pro Gly Gln Arg Leu Glu Trp Ile
        35                  40                  45
Gly Met Ile His Pro Asn Ser Gly Ser Thr Asn Tyr Asn Glu Lys Phe
    50                  55                  60
Lys Gly Lys Ala Thr Leu Thr Leu Asp Lys Ser Ala Ser Thr Ala Tyr

65 70 75 80
Met Glu Leu Ser Ser Leu Arg Ser Glu Asp Thr Ala Val Tyr Tyr Cys
85 90 95
Ala Arg Leu Lys Thr Gly Asn Ser Phe Asp Tyr Trp Gly Gln Gly Thr
100 105 110
Thr Val Thr Val Ser Ser
115

<210> 34
<211> 118
<212> PRT
<213> Artificial sequence

<220>
<223> VH14

<400> 34
Glu Val Gln Leu Val Gln Ser Gly Ala Glu Val Lys Lys Pro Gly Ala
1 5 10 15
Ser Val Lys Val Ser Cys Lys Ala Ser Gly Tyr Thr Phe Thr Ser Tyr
20 25 30
Trp Met His Trp Val Lys Gln Arg Pro Gly Gln Arg Leu Glu Trp Ile
35 40 45
Gly Met Ile His Pro Asn Ser Gly Ser Thr Asn Tyr Asn Glu Lys Phe
50 55 60
Lys Gly Lys Ala Thr Leu Thr Leu Asp Lys Ser Ala Ser Thr Ala Tyr
65 70 75 80
Met Glu Leu Ser Ser Leu Arg Ser Glu Asp Thr Ala Val Tyr Tyr Cys
85 90 95
Ala Arg Leu Lys Thr Gly Asn Ser Phe Asp Tyr Trp Gly Gln Gly Thr
100 105 110
Thr Val Thr Val Ser Ser
115

<210> 35
<211> 450
<212> PRT
<213> Artificial sequence

<220>
<223> ch1901 heavy chain

<400> 35
Glu Val Gln Leu Met Glu Ser Gly Gly Gly Leu Val Lys Pro Gly Gly
1 5 10 15
Ser Leu Lys Leu Ser Cys Ala Ala Ser Gly Phe Thr Phe Ser Asp Tyr
20 25 30
Gly Ile His Trp Val Arg Gln Ala Pro Glu Met Gly Leu Glu Trp Ile
35 40 45
Ala Tyr Ile Ser Arg Gly Ser Ser Thr Ile Tyr Tyr Ala Asp Thr Val
50 55 60
Lys Gly Arg Phe Thr Met Ser Arg Asp Asn Ala Lys Asn Thr Leu Phe
65 70 75 80
Leu Gln Met Thr Ser Leu Arg Ser Glu Asp Thr Ala Met Tyr Tyr Cys
85 90 95
Ala Arg Gly Gly Tyr Asp Thr Arg Asn Ala Met Asp Tyr Trp Gly Gln
100 105 110
Gly Thr Ser Val Thr Val Ser Ser Ala Ser Thr Lys Gly Pro Ser Val
115 120 125
Phe Pro Leu Ala Pro Ser Ser Lys Ser Thr Ser Gly Gly Thr Ala Ala
130 135 140

Leu Gly Cys Leu Val Lys Asp Tyr Phe Pro Glu Pro Val Thr Val Ser
145 150 155 160
Trp Asn Ser Gly Ala Leu Thr Ser Gly Val His Thr Phe Pro Ala Val
165 170 175
Leu Gln Ser Ser Gly Leu Tyr Ser Leu Ser Ser Val Val Thr Val Pro
180 185 190
Ser Ser Ser Leu Gly Thr Gln Thr Tyr Ile Cys Asn Val Asn His Lys
195 200 205
Pro Ser Asn Thr Lys Val Asp Lys Lys Val Glu Pro Lys Ser Cys Asp
210 215 220
Lys Thr His Thr Cys Pro Pro Cys Pro Ala Pro Glu Leu Leu Gly Gly
225 230 235 240
Pro Ser Val Phe Leu Phe Pro Pro Lys Pro Lys Asp Thr Leu Met Ile
245 250 255
Ser Arg Thr Pro Glu Val Thr Cys Val Val Val Asp Val Ser His Glu
260 265 270
Asp Pro Glu Val Lys Phe Asn Trp Tyr Val Asp Gly Val Glu Val His
275 280 285
Asn Ala Lys Thr Lys Pro Arg Glu Glu Gln Tyr Asn Ser Thr Tyr Arg
290 295 300
Val Val Ser Val Leu Thr Val Leu His Gln Asp Trp Leu Asn Gly Lys
305 310 315 320
Glu Tyr Lys Cys Lys Val Ser Asn Lys Ala Leu Pro Ala Pro Ile Glu
325 330 335
Lys Thr Ile Ser Lys Ala Lys Gly Gln Pro Arg Glu Pro Gln Val Tyr
340 345 350
Thr Leu Pro Pro Ser Arg Asp Glu Leu Thr Lys Asn Gln Val Ser Leu
355 360 365
Thr Cys Leu Val Lys Gly Phe Tyr Pro Ser Asp Ile Ala Val Glu Trp
370 375 380
Glu Ser Asn Gly Gln Pro Glu Asn Asn Tyr Lys Thr Thr Pro Pro Val
385 390 395 400
Leu Asp Ser Asp Gly Ser Phe Phe Leu Tyr Ser Lys Leu Thr Val Asp
405 410 415
Lys Ser Arg Trp Gln Gln Gly Asn Val Phe Ser Cys Ser Val Met His
420 425 430
Glu Ala Leu His Asn His Tyr Thr Gln Lys Ser Leu Ser Leu Ser Pro
435 440 445
Gly Lys
450

<210> 36
<211> 220
<212> PRT
<213> Artificial sequence

<220>
<223> ch1901 light chain

<400> 36
Asp Ile Val Met Thr Gln Ser Pro Ser Ser Leu Ser Val Ser Ala Gly
1 5 10 15
Glu Lys Val Thr Met Ser Cys Lys Ser Ser Gln Ser Leu Leu Asn Ser
20 25 30
Gly Asn Gln Lys Asn Tyr Leu Ala Trp Tyr Gln Gln Lys Pro Gly Gln
35 40 45
Pro Pro Lys Leu Leu Ile Tyr Gly Ala Ser Thr Arg Ala Ser Gly Val
50 55 60
Pro Asp Arg Phe Thr Gly Ser Gly Ser Gly Thr Asp Phe Thr Leu Thr
65 70 75 80
Ile Ser Ser Val Gln Ala Glu Asp Leu Ala Ile Tyr His Cys Gln Asn
85 90 95

Asp Leu Tyr Tyr Pro Leu Thr Phe Gly Ala Gly Thr Lys Leu Glu Leu
100 105 110
Lys Arg Thr Val Ala Ala Pro Ser Val Phe Ile Phe Pro Pro Ser Asp
115 120 125
Glu Gln Leu Lys Ser Gly Thr Ala Ser Val Val Cys Leu Leu Asn Asn
130 135 140
Phe Tyr Pro Arg Glu Ala Lys Val Gln Trp Lys Val Asp Asn Ala Leu
145 150 155 160
Gln Ser Gly Asn Ser Gln Glu Ser Val Thr Glu Gln Asp Ser Lys Asp
165 170 175
Ser Thr Tyr Ser Leu Ser Ser Thr Leu Thr Leu Ser Lys Ala Asp Tyr
180 185 190
Glu Lys His Lys Val Tyr Ala Cys Glu Val Thr His Gln Gly Leu Ser
195 200 205
Ser Pro Val Thr Lys Ser Phe Asn Arg Gly Glu Cys
210 215 220

<210> 37
<211> 448
<212> PRT
<213> Artificial sequence

<220>
<223> ch1902 heavy chain

<400> 37
Glu Val Gln Leu Gln Glu Ser Gly Ala Glu Leu Val Lys Pro Gly Ala
1 5 10 15
Ser Val Lys Leu Ser Cys Lys Ala Ser Gly Tyr Ile Phe Thr Ser Tyr
20 25 30
Trp Met His Trp Val Lys Gln Arg Pro Gly Gln Gly Leu Glu Trp Ile
35 40 45
Gly Met Ile His Pro Asn Ser Gly Ser Thr Asn Tyr Asn Glu Lys Phe
50 55 60
Lys Gly Lys Ala Thr Leu Thr Leu Asp Lys Ser Ser Ser Thr Ala Tyr
65 70 75 80
Met Gln Leu Ser Ser Leu Pro Ser Glu Asp Ser Ala Val Tyr Tyr Cys
85 90 95
Ala Arg Leu Lys Thr Gly Asn Ser Phe Asp Tyr Trp Gly Gln Gly Thr
100 105 110
Thr Leu Thr Val Ser Ser Ala Ser Thr Lys Gly Pro Ser Val Phe Pro
115 120 125
Leu Ala Pro Ser Ser Lys Ser Thr Ser Gly Gly Thr Ala Ala Leu Gly
130 135 140
Cys Leu Val Lys Asp Tyr Phe Pro Glu Pro Val Thr Val Ser Trp Asn
145 150 155 160
Ser Gly Ala Leu Thr Ser Gly Val His Thr Phe Pro Ala Val Leu Gln
165 170 175
Ser Ser Gly Leu Tyr Ser Leu Ser Ser Val Val Thr Val Pro Ser Ser
180 185 190
Ser Leu Gly Thr Gln Thr Tyr Ile Cys Asn Val Asn His Lys Pro Ser
195 200 205
Asn Thr Lys Val Asp Lys Lys Val Glu Pro Lys Ser Cys Asp Lys Thr
210 215 220
His Thr Cys Pro Pro Cys Pro Ala Pro Glu Leu Leu Gly Gly Pro Ser
225 230 235 240
Val Phe Leu Phe Pro Pro Lys Pro Lys Asp Thr Leu Met Ile Ser Arg
245 250 255
Thr Pro Glu Val Thr Cys Val Val Val Asp Val Ser His Glu Asp Pro
260 265 270
Glu Val Lys Phe Asn Trp Tyr Val Asp Gly Val Glu Val His Asn Ala
275 280 285

Lys Thr Lys Pro Arg Glu Glu Gln Tyr Asn Ser Thr Tyr Arg Val Val
    290                 295                 300
Ser Val Leu Thr Val Leu His Gln Asp Trp Leu Asn Gly Lys Glu Tyr
305                 310                 315                 320
Lys Cys Lys Val Ser Asn Lys Ala Leu Pro Ala Pro Ile Glu Lys Thr
                325                 330                 335
Ile Ser Lys Ala Lys Gly Gln Pro Arg Glu Pro Gln Val Tyr Thr Leu
            340                 345                 350
Pro Pro Ser Arg Asp Glu Leu Thr Lys Asn Gln Val Ser Leu Thr Cys
        355                 360                 365
Leu Val Lys Gly Phe Tyr Pro Ser Asp Ile Ala Val Glu Trp Glu Ser
    370                 375                 380
Asn Gly Gln Pro Glu Asn Asn Tyr Lys Thr Thr Pro Pro Val Leu Asp
385                 390                 395                 400
Ser Asp Gly Ser Phe Phe Leu Tyr Ser Lys Leu Thr Val Asp Lys Ser
                405                 410                 415
Arg Trp Gln Gln Gly Asn Val Phe Ser Cys Ser Val Met His Glu Ala
            420                 425                 430
Leu His Asn His Tyr Thr Gln Lys Ser Leu Ser Leu Ser Pro Gly Lys
        435                 440                 445

<210>  38
<211>  220
<212>  PRT
<213>  Artificial sequence

<220>
<223>  ch1902 light chain

<400>  38
Asp Ile Val Leu Thr Gln Ser Pro Ser Ser Leu Thr Val Thr Ala Gly
1               5                   10                  15
Glu Lys Val Thr Met Ser Cys Lys Ser Ser Gln Ser Leu Leu Asn Ser
            20                  25                  30
Gly Asn Gln Lys Asn Tyr Leu Thr Trp Tyr Gln Gln Lys Pro Gly Gln
        35                  40                  45
Pro Pro Lys Leu Leu Ile Tyr Trp Ala Ser Thr Arg Glu Ser Gly Val
    50                  55                  60
Pro Asp Arg Phe Thr Gly Ser Gly Ser Gly Thr Asp Phe Thr Leu Thr
65                  70                  75                  80
Ile Ser Ser Val Gln Ala Glu Asp Leu Ala Ile Tyr Tyr Cys Gln Asn
                85                  90                  95
Ala Tyr Thr Tyr Pro Phe Thr Phe Gly Ser Gly Thr Lys Leu Glu Ile
            100                 105                 110
Lys Arg Thr Val Ala Ala Pro Ser Val Phe Ile Phe Pro Pro Ser Asp
        115                 120                 125
Glu Gln Leu Lys Ser Gly Thr Ala Ser Val Val Cys Leu Leu Asn Asn
    130                 135                 140
Phe Tyr Pro Arg Glu Ala Lys Val Gln Trp Lys Val Asp Asn Ala Leu
145                 150                 155                 160
Gln Ser Gly Asn Ser Gln Glu Ser Val Thr Glu Gln Asp Ser Lys Asp
                165                 170                 175
Ser Thr Tyr Ser Leu Ser Ser Thr Leu Thr Leu Ser Lys Ala Asp Tyr
            180                 185                 190
Glu Lys His Lys Val Tyr Ala Cys Glu Val Thr His Gln Gly Leu Ser
        195                 200                 205
Ser Pro Val Thr Lys Ser Phe Asn Arg Gly Glu Cys
    210                 215                 220

<210>  39
<211>  220
<212>  PRT

<213> Artificial sequence

<220>
<223> L1

<400> 39
Asp Ile Val Met Thr Gln Ser Pro Asp Ser Leu Ala Val Ser Leu Gly
1 5 10 15
Glu Arg Ala Thr Ile Asn Cys Lys Ser Ser Gln Ser Leu Leu Asn Ser
20 25 30
Gly Asn Gln Lys Asn Tyr Leu Ala Trp Tyr Gln Gln Lys Pro Gly Gln
35 40 45
Pro Pro Lys Leu Leu Ile Tyr Gly Ala Ser Thr Arg Ala Ser Gly Val
50 55 60
Pro Asp Arg Phe Ser Gly Ser Gly Ser Gly Thr Asp Phe Thr Leu Thr
65 70 75 80
Ile Ser Ser Leu Gln Ala Glu Asp Val Ala Val Tyr Tyr Cys Gln Asn
85 90 95
Asp Leu Tyr Tyr Pro Leu Thr Phe Gly Gln Gly Thr Lys Leu Glu Ile
100 105 110
Lys Arg Thr Val Ala Ala Pro Ser Val Phe Ile Phe Pro Pro Ser Asp
115 120 125
Glu Gln Leu Lys Ser Gly Thr Ala Ser Val Val Cys Leu Leu Asn Asn
130 135 140
Phe Tyr Pro Arg Glu Ala Lys Val Gln Trp Lys Val Asp Asn Ala Leu
145 150 155 160
Gln Ser Gly Asn Ser Gln Glu Ser Val Thr Glu Gln Asp Ser Lys Asp
165 170 175
Ser Thr Tyr Ser Leu Ser Ser Thr Leu Thr Leu Ser Lys Ala Asp Tyr
180 185 190
Glu Lys His Lys Val Tyr Ala Cys Glu Val Thr His Gln Gly Leu Ser
195 200 205
Ser Pro Val Thr Lys Ser Phe Asn Arg Gly Glu Cys
210 215 220

<210> 40
<211> 220
<212> PRT
<213> Artificial sequence

<220>
<223> L2

<400> 40
Asp Ile Val Met Thr Gln Ser Pro Asp Ser Leu Ala Val Ser Leu Gly
1 5 10 15
Glu Arg Ala Thr Ile Ser Cys Lys Ser Ser Gln Ser Leu Leu Asn Ser
20 25 30
Gly Asn Gln Lys Asn Tyr Leu Ala Trp Tyr Gln Gln Lys Pro Gly Gln
35 40 45
Pro Pro Lys Leu Leu Ile Tyr Gly Ala Ser Thr Arg Ala Ser Gly Val
50 55 60
Pro Asp Arg Phe Ser Gly Ser Gly Ser Gly Thr Asp Phe Thr Leu Thr
65 70 75 80
Ile Ser Ser Leu Gln Ala Glu Asp Val Ala Val Tyr Tyr Cys Gln Asn
85 90 95
Asp Leu Tyr Tyr Pro Leu Thr Phe Gly Gln Gly Thr Lys Leu Glu Ile
100 105 110
Lys Arg Thr Val Ala Ala Pro Ser Val Phe Ile Phe Pro Pro Ser Asp
115 120 125
Glu Gln Leu Lys Ser Gly Thr Ala Ser Val Val Cys Leu Leu Asn Asn

130 135 140
Phe Tyr Pro Arg Glu Ala Lys Val Gln Trp Lys Val Asp Asn Ala Leu
145 150 155 160
Gln Ser Gly Asn Ser Gln Glu Ser Val Thr Glu Gln Asp Ser Lys Asp
165 170 175
Ser Thr Tyr Ser Leu Ser Ser Thr Leu Thr Leu Ser Lys Ala Asp Tyr
180 185 190
Glu Lys His Lys Val Tyr Ala Cys Glu Val Thr His Gln Gly Leu Ser
195 200 205
Ser Pro Val Thr Lys Ser Phe Asn Arg Gly Glu Cys
210 215 220

<210> 41
<211> 220
<212> PRT
<213> Artificial sequence

<220>
<223> L3

<400> 41
Asp Ile Val Met Thr Gln Ser Pro Asp Ser Leu Ala Val Ser Leu Gly
1 5 10 15
Glu Arg Ala Thr Ile Ser Cys Lys Ser Ser Gln Ser Leu Leu Asn Ser
20 25 30
Gly Asn Gln Lys Asn Tyr Leu Ala Trp Tyr Gln Gln Lys Pro Gly Gln
35 40 45
Pro Pro Lys Leu Leu Ile Tyr Gly Ala Ser Thr Arg Ala Ser Gly Val
50 55 60
Pro Asp Arg Phe Ser Gly Ser Gly Ser Gly Thr Asp Phe Thr Leu Thr
65 70 75 80
Ile Ser Ser Leu Gln Ala Glu Asp Val Ala Ile Tyr His Cys Gln Asn
85 90 95
Asp Leu Tyr Tyr Pro Leu Thr Phe Gly Gln Gly Thr Lys Leu Glu Ile
100 105 110
Lys Arg Thr Val Ala Ala Pro Ser Val Phe Ile Phe Pro Pro Ser Asp
115 120 125
Glu Gln Leu Lys Ser Gly Thr Ala Ser Val Val Cys Leu Leu Asn Asn
130 135 140
Phe Tyr Pro Arg Glu Ala Lys Val Gln Trp Lys Val Asp Asn Ala Leu
145 150 155 160
Gln Ser Gly Asn Ser Gln Glu Ser Val Thr Glu Gln Asp Ser Lys Asp
165 170 175
Ser Thr Tyr Ser Leu Ser Ser Thr Leu Thr Leu Ser Lys Ala Asp Tyr
180 185 190
Glu Lys His Lys Val Tyr Ala Cys Glu Val Thr His Gln Gly Leu Ser
195 200 205
Ser Pro Val Thr Lys Ser Phe Asn Arg Gly Glu Cys
210 215 220

<210> 42
<211> 450
<212> PRT
<213> Artificial sequence

<220>
<223> H1

<400> 42
Glu Val Gln Leu Val Glu Ser Gly Gly Gly Leu Val Gln Pro Gly Gly
1 5 10 15

Ser Leu Arg Leu Ser Cys Ala Ala Ser Gly Phe Thr Phe Ser Asp Tyr
20 25 30
Gly Ile His Trp Val Arg Gln Ala Pro Gly Lys Gly Leu Glu Trp Val
35 40 45
Ala Tyr Ile Ser Arg Gly Ser Ser Thr Ile Tyr Tyr Ala Asp Thr Val
50 55 60
Lys Gly Arg Phe Thr Ile Ser Arg Asp Asn Ala Lys Asn Ser Leu Tyr
65 70 75 80
Leu Gln Met Asn Ser Leu Arg Ala Glu Asp Thr Ala Val Tyr Tyr Cys
85 90 95
Ala Arg Gly Gly Tyr Asp Thr Arg Asn Ala Met Asp Tyr Trp Gly Gln
100 105 110
Gly Thr Thr Val Thr Val Ser Ser Ala Ser Thr Lys Gly Pro Ser Val
115 120 125
Phe Pro Leu Ala Pro Ser Ser Lys Ser Thr Ser Gly Gly Thr Ala Ala
130 135 140
Leu Gly Cys Leu Val Lys Asp Tyr Phe Pro Glu Pro Val Thr Val Ser
145 150 155 160
Trp Asn Ser Gly Ala Leu Thr Ser Gly Val His Thr Phe Pro Ala Val
165 170 175
Leu Gln Ser Ser Gly Leu Tyr Ser Leu Ser Ser Val Val Thr Val Pro
180 185 190
Ser Ser Ser Leu Gly Thr Gln Thr Tyr Ile Cys Asn Val Asn His Lys
195 200 205
Pro Ser Asn Thr Lys Val Asp Lys Lys Val Glu Pro Lys Ser Cys Asp
210 215 220
Lys Thr His Thr Cys Pro Pro Cys Pro Ala Pro Glu Leu Leu Gly Gly
225 230 235 240
Pro Ser Val Phe Leu Phe Pro Pro Lys Pro Lys Asp Thr Leu Met Ile
245 250 255
Ser Arg Thr Pro Glu Val Thr Cys Val Val Val Asp Val Ser His Glu
260 265 270
Asp Pro Glu Val Lys Phe Asn Trp Tyr Val Asp Gly Val Glu Val His
275 280 285
Asn Ala Lys Thr Lys Pro Arg Glu Glu Gln Tyr Asn Ser Thr Tyr Arg
290 295 300
Val Val Ser Val Leu Thr Val Leu His Gln Asp Trp Leu Asn Gly Lys
305 310 315 320
Glu Tyr Lys Cys Lys Val Ser Asn Lys Ala Leu Pro Ala Pro Ile Glu
325 330 335
Lys Thr Ile Ser Lys Ala Lys Gly Gln Pro Arg Glu Pro Gln Val Tyr
340 345 350
Thr Leu Pro Pro Ser Arg Asp Glu Leu Thr Lys Asn Gln Val Ser Leu
355 360 365
Thr Cys Leu Val Lys Gly Phe Tyr Pro Ser Asp Ile Ala Val Glu Trp
370 375 380
Glu Ser Asn Gly Gln Pro Glu Asn Asn Tyr Lys Thr Thr Pro Pro Val
385 390 395 400
Leu Asp Ser Asp Gly Ser Phe Phe Leu Tyr Ser Lys Leu Thr Val Asp
405 410 415
Lys Ser Arg Trp Gln Gln Gly Asn Val Phe Ser Cys Ser Val Met His
420 425 430
Glu Ala Leu His Asn His Tyr Thr Gln Lys Ser Leu Ser Leu Ser Pro
435 440 445
Gly Lys
450

<210> 43
<211> 450
<212> PRT
<213> Artificial sequence

<220>

<223> H2

<400> 43

```
Glu Val Gln Leu Val Glu Ser Gly Gly Gly Leu Val Gln Pro Gly Gly
1               5                   10                  15
Ser Leu Arg Leu Ser Cys Ala Ala Ser Gly Phe Thr Phe Ser Asp Tyr
            20                  25                  30
Gly Ile His Trp Val Arg Gln Ala Pro Gly Lys Gly Leu Glu Trp Val
        35                  40                  45
Ala Tyr Ile Ser Arg Gly Ser Ser Thr Ile Tyr Tyr Ala Asp Thr Val
    50                  55                  60
Lys Gly Arg Phe Thr Ile Ser Arg Asp Asn Ala Lys Asn Ser Leu Tyr
65                  70                  75                  80
Leu Gln Met Thr Ser Leu Arg Ala Glu Asp Thr Ala Val Tyr Tyr Cys
                85                  90                  95
Ala Arg Gly Gly Tyr Asp Thr Arg Asn Ala Met Asp Tyr Trp Gly Gln
            100                 105                 110
Gly Thr Thr Val Thr Val Ser Ser Ala Ser Thr Lys Gly Pro Ser Val
        115                 120                 125
Phe Pro Leu Ala Pro Ser Ser Lys Ser Thr Ser Gly Gly Thr Ala Ala
    130                 135                 140
Leu Gly Cys Leu Val Lys Asp Tyr Phe Pro Glu Pro Val Thr Val Ser
145                 150                 155                 160
Trp Asn Ser Gly Ala Leu Thr Ser Gly Val His Thr Phe Pro Ala Val
                165                 170                 175
Leu Gln Ser Ser Gly Leu Tyr Ser Leu Ser Ser Val Val Thr Val Pro
            180                 185                 190
Ser Ser Ser Leu Gly Thr Gln Thr Tyr Ile Cys Asn Val Asn His Lys
        195                 200                 205
Pro Ser Asn Thr Lys Val Asp Lys Lys Val Glu Pro Lys Ser Cys Asp
    210                 215                 220
Lys Thr His Thr Cys Pro Pro Cys Pro Ala Pro Glu Leu Leu Gly Gly
225                 230                 235                 240
Pro Ser Val Phe Leu Phe Pro Pro Lys Pro Lys Asp Thr Leu Met Ile
                245                 250                 255
Ser Arg Thr Pro Glu Val Thr Cys Val Val Val Asp Val Ser His Glu
            260                 265                 270
Asp Pro Glu Val Lys Phe Asn Trp Tyr Val Asp Gly Val Glu Val His
        275                 280                 285
Asn Ala Lys Thr Lys Pro Arg Glu Glu Gln Tyr Asn Ser Thr Tyr Arg
    290                 295                 300
Val Val Ser Val Leu Thr Val Leu His Gln Asp Trp Leu Asn Gly Lys
305                 310                 315                 320
Glu Tyr Lys Cys Lys Val Ser Asn Lys Ala Leu Pro Ala Pro Ile Glu
                325                 330                 335
Lys Thr Ile Ser Lys Ala Lys Gly Gln Pro Arg Glu Pro Gln Val Tyr
            340                 345                 350
Thr Leu Pro Pro Ser Arg Asp Glu Leu Thr Lys Asn Gln Val Ser Leu
        355                 360                 365
Thr Cys Leu Val Lys Gly Phe Tyr Pro Ser Asp Ile Ala Val Glu Trp
    370                 375                 380
Glu Ser Asn Gly Gln Pro Glu Asn Asn Tyr Lys Thr Thr Pro Pro Val
385                 390                 395                 400
Leu Asp Ser Asp Gly Ser Phe Phe Leu Tyr Ser Lys Leu Thr Val Asp
                405                 410                 415
Lys Ser Arg Trp Gln Gln Gly Asn Val Phe Ser Cys Ser Val Met His
            420                 425                 430
Glu Ala Leu His Asn His Tyr Thr Gln Lys Ser Leu Ser Leu Ser Pro
        435                 440                 445
Gly Lys
    450
```

```
<210>  44
<211>  450
<212>  PRT
<213>  Artificial sequence

<220>
<223>  H3


<400>  44
Glu Val Gln Leu Val Glu Ser Gly Gly Gly Leu Val Gln Pro Gly Gly
1               5                   10                  15
Ser Leu Arg Leu Ser Cys Ala Ala Ser Gly Phe Thr Phe Ser Asp Tyr
            20                  25                  30
Gly Ile His Trp Val Arg Gln Ala Pro Gly Lys Gly Leu Glu Trp Ile
        35                  40                  45
Ala Tyr Ile Ser Arg Gly Ser Ser Thr Ile Tyr Tyr Ala Asp Thr Val
    50                  55                  60
Lys Gly Arg Phe Thr Ile Ser Arg Asp Asn Ala Lys Asn Ser Leu Tyr
65                  70                  75                  80
Leu Gln Met Thr Ser Leu Arg Ala Glu Asp Thr Ala Val Tyr Tyr Cys
                85                  90                  95
Ala Arg Gly Gly Tyr Asp Thr Arg Asn Ala Met Asp Tyr Trp Gly Gln
            100                 105                 110
Gly Thr Thr Val Thr Val Ser Ser Ala Ser Thr Lys Gly Pro Ser Val
        115                 120                 125
Phe Pro Leu Ala Pro Ser Ser Lys Ser Thr Ser Gly Gly Thr Ala Ala
    130                 135                 140
Leu Gly Cys Leu Val Lys Asp Tyr Phe Pro Glu Pro Val Thr Val Ser
145                 150                 155                 160
Trp Asn Ser Gly Ala Leu Thr Ser Gly Val His Thr Phe Pro Ala Val
                165                 170                 175
Leu Gln Ser Ser Gly Leu Tyr Ser Leu Ser Ser Val Val Thr Val Pro
            180                 185                 190
Ser Ser Ser Leu Gly Thr Gln Thr Tyr Ile Cys Asn Val Asn His Lys
        195                 200                 205
Pro Ser Asn Thr Lys Val Asp Lys Lys Val Glu Pro Lys Ser Cys Asp
    210                 215                 220
Lys Thr His Thr Cys Pro Pro Cys Pro Ala Pro Glu Leu Leu Gly Gly
225                 230                 235                 240
Pro Ser Val Phe Leu Phe Pro Pro Lys Pro Lys Asp Thr Leu Met Ile
                245                 250                 255
Ser Arg Thr Pro Glu Val Thr Cys Val Val Val Asp Val Ser His Glu
            260                 265                 270
Asp Pro Glu Val Lys Phe Asn Trp Tyr Val Asp Gly Val Glu Val His
        275                 280                 285
Asn Ala Lys Thr Lys Pro Arg Glu Glu Gln Tyr Asn Ser Thr Tyr Arg
    290                 295                 300
Val Val Ser Val Leu Thr Val Leu His Gln Asp Trp Leu Asn Gly Lys
305                 310                 315                 320
Glu Tyr Lys Cys Lys Val Ser Asn Lys Ala Leu Pro Ala Pro Ile Glu
                325                 330                 335
Lys Thr Ile Ser Lys Ala Lys Gly Gln Pro Arg Glu Pro Gln Val Tyr
            340                 345                 350
Thr Leu Pro Pro Ser Arg Asp Glu Leu Thr Lys Asn Gln Val Ser Leu
        355                 360                 365
Thr Cys Leu Val Lys Gly Phe Tyr Pro Ser Asp Ile Ala Val Glu Trp
    370                 375                 380
Glu Ser Asn Gly Gln Pro Glu Asn Asn Tyr Lys Thr Thr Pro Pro Val
385                 390                 395                 400
Leu Asp Ser Asp Gly Ser Phe Phe Leu Tyr Ser Lys Leu Thr Val Asp
                405                 410                 415
Lys Ser Arg Trp Gln Gln Gly Asn Val Phe Ser Cys Ser Val Met His
```

```
            420                 425                 430
Glu Ala Leu His Asn His Tyr Thr Gln Lys Ser Leu Ser Leu Ser Pro
        435                 440                 445
Gly Lys
    450

<210>  45
<211>  450
<212>  PRT
<213>  Artificial sequence

<220>
<223>  H4


<400>  45
Glu Val Gln Leu Val Glu Ser Gly Gly Gly Leu Val Gln Pro Gly Gly
1               5                   10                  15
Ser Leu Arg Leu Ser Cys Ala Ala Ser Gly Phe Thr Phe Ser Asp Tyr
            20                  25                  30
Gly Ile His Trp Val Arg Gln Ala Pro Gly Lys Gly Leu Glu Trp Val
        35                  40                  45
Ala Tyr Ile Ser Arg Gly Ser Ser Thr Ile Tyr Tyr Ala Asp Thr Val
    50                  55                  60
Lys Gly Arg Phe Thr Met Ser Arg Asp Asn Ala Lys Asn Ser Leu Tyr
65                  70                  75                  80
Leu Gln Met Thr Ser Leu Arg Ala Glu Asp Thr Ala Val Tyr Tyr Cys
                85                  90                  95
Ala Arg Gly Gly Tyr Asp Thr Arg Asn Ala Met Asp Tyr Trp Gly Gln
            100                 105                 110
Gly Thr Thr Val Thr Val Ser Ser Ala Ser Thr Lys Gly Pro Ser Val
        115                 120                 125
Phe Pro Leu Ala Pro Ser Ser Lys Ser Thr Ser Gly Gly Thr Ala Ala
    130                 135                 140
Leu Gly Cys Leu Val Lys Asp Tyr Phe Pro Glu Pro Val Thr Val Ser
145                 150                 155                 160
Trp Asn Ser Gly Ala Leu Thr Ser Gly Val His Thr Phe Pro Ala Val
                165                 170                 175
Leu Gln Ser Ser Gly Leu Tyr Ser Leu Ser Ser Val Val Thr Val Pro
            180                 185                 190
Ser Ser Ser Leu Gly Thr Gln Thr Tyr Ile Cys Asn Val Asn His Lys
        195                 200                 205
Pro Ser Asn Thr Lys Val Asp Lys Lys Val Glu Pro Lys Ser Cys Asp
    210                 215                 220
Lys Thr His Thr Cys Pro Pro Cys Pro Ala Pro Glu Leu Leu Gly Gly
225                 230                 235                 240
Pro Ser Val Phe Leu Phe Pro Pro Lys Pro Lys Asp Thr Leu Met Ile
                245                 250                 255
Ser Arg Thr Pro Glu Val Thr Cys Val Val Val Asp Val Ser His Glu
            260                 265                 270
Asp Pro Glu Val Lys Phe Asn Trp Tyr Val Asp Gly Val Glu Val His
        275                 280                 285
Asn Ala Lys Thr Lys Pro Arg Glu Glu Gln Tyr Asn Ser Thr Tyr Arg
    290                 295                 300
Val Val Ser Val Leu Thr Val Leu His Gln Asp Trp Leu Asn Gly Lys
305                 310                 315                 320
Glu Tyr Lys Cys Lys Val Ser Asn Lys Ala Leu Pro Ala Pro Ile Glu
                325                 330                 335
Lys Thr Ile Ser Lys Ala Lys Gly Gln Pro Arg Glu Pro Gln Val Tyr
            340                 345                 350
Thr Leu Pro Pro Ser Arg Asp Glu Leu Thr Lys Asn Gln Val Ser Leu
        355                 360                 365
Thr Cys Leu Val Lys Gly Phe Tyr Pro Ser Asp Ile Ala Val Glu Trp
```

370 375 380
Glu Ser Asn Gly Gln Pro Glu Asn Asn Tyr Lys Thr Thr Pro Pro Val
385 390 395 400
Leu Asp Ser Asp Gly Ser Phe Phe Leu Tyr Ser Lys Leu Thr Val Asp
405 410 415
Lys Ser Arg Trp Gln Gln Gly Asn Val Phe Ser Cys Ser Val Met His
420 425 430
Glu Ala Leu His Asn His Tyr Thr Gln Lys Ser Leu Ser Leu Ser Pro
435 440 445
Gly Lys
450

<210> 46
<211> 220
<212> PRT
<213> Artificial sequence

<220>
<223> L11

<400> 46
Asp Ile Val Met Thr Gln Ser Pro Asp Ser Leu Ala Val Ser Leu Gly
1 5 10 15
Glu Arg Ala Thr Ile Asn Cys Lys Ser Ser Gln Ser Leu Leu Asn Ser
20 25 30
Gly Asn Gln Lys Asn Tyr Leu Thr Trp Tyr Gln Gln Lys Pro Gly Gln
35 40 45
Pro Pro Lys Leu Leu Ile Tyr Trp Ala Ser Thr Arg Glu Ser Gly Val
50 55 60
Pro Asp Arg Phe Ser Gly Ser Gly Ser Gly Thr Asp Phe Thr Leu Thr
65 70 75 80
Ile Ser Ser Leu Gln Ala Glu Asp Val Ala Val Tyr Tyr Cys Gln Asn
85 90 95
Ala Tyr Thr Tyr Pro Phe Thr Phe Gly Gln Gly Thr Lys Leu Glu Ile
100 105 110
Lys Arg Thr Val Ala Ala Pro Ser Val Phe Ile Phe Pro Pro Ser Asp
115 120 125
Glu Gln Leu Lys Ser Gly Thr Ala Ser Val Val Cys Leu Leu Asn Asn
130 135 140
Phe Tyr Pro Arg Glu Ala Lys Val Gln Trp Lys Val Asp Asn Ala Leu
145 150 155 160
Gln Ser Gly Asn Ser Gln Glu Ser Val Thr Glu Gln Asp Ser Lys Asp
165 170 175
Ser Thr Tyr Ser Leu Ser Ser Thr Leu Thr Leu Ser Lys Ala Asp Tyr
180 185 190
Glu Lys His Lys Val Tyr Ala Cys Glu Val Thr His Gln Gly Leu Ser
195 200 205
Ser Pro Val Thr Lys Ser Phe Asn Arg Gly Glu Cys
210 215 220

<210> 47
<211> 220
<212> PRT
<213> Artificial sequence

<220>
<223> L12

<400> 47
Asp Ile Val Leu Thr Gln Ser Pro Asp Ser Leu Ala Val Ser Leu Gly
1 5 10 15

Glu Arg Ala Thr Ile Asn Cys Lys Ser Ser Gln Ser Leu Leu Asn Ser
20 25 30
Gly Asn Gln Lys Asn Tyr Leu Thr Trp Tyr Gln Gln Lys Pro Gly Gln
35 40 45
Pro Pro Lys Leu Leu Ile Tyr Trp Ala Ser Thr Arg Glu Ser Gly Val
50 55 60
Pro Asp Arg Phe Ser Gly Ser Gly Ser Gly Thr Asp Phe Thr Leu Thr
65 70 75 80
Ile Ser Ser Leu Gln Ala Glu Asp Val Ala Val Tyr Tyr Cys Gln Asn
85 90 95
Ala Tyr Thr Tyr Pro Phe Thr Phe Gly Gln Gly Thr Lys Leu Glu Ile
100 105 110
Lys Arg Thr Val Ala Ala Pro Ser Val Phe Ile Phe Pro Pro Ser Asp
115 120 125
Glu Gln Leu Lys Ser Gly Thr Ala Ser Val Val Cys Leu Leu Asn Asn
130 135 140
Phe Tyr Pro Arg Glu Ala Lys Val Gln Trp Lys Val Asp Asn Ala Leu
145 150 155 160
Gln Ser Gly Asn Ser Gln Glu Ser Val Thr Glu Gln Asp Ser Lys Asp
165 170 175
Ser Thr Tyr Ser Leu Ser Ser Thr Leu Thr Leu Ser Lys Ala Asp Tyr
180 185 190
Glu Lys His Lys Val Tyr Ala Cys Glu Val Thr His Gln Gly Leu Ser
195 200 205
Ser Pro Val Thr Lys Ser Phe Asn Arg Gly Glu Cys
210 215 220

<210> 48
<211> 220
<212> PRT
<213> Artificial sequence

<220>
<223> L13

<400> 48
Asp Ile Val Leu Thr Gln Ser Pro Asp Ser Leu Ala Val Ser Leu Gly
1 5 10 15
Glu Arg Ala Thr Ile Ser Cys Lys Ser Ser Gln Ser Leu Leu Asn Ser
20 25 30
Gly Asn Gln Lys Asn Tyr Leu Thr Trp Tyr Gln Gln Lys Pro Gly Gln
35 40 45
Pro Pro Lys Leu Leu Ile Tyr Trp Ala Ser Thr Arg Glu Ser Gly Val
50 55 60
Pro Asp Arg Phe Ser Gly Ser Gly Ser Gly Thr Asp Phe Thr Leu Thr
65 70 75 80
Ile Ser Ser Leu Gln Ala Glu Asp Val Ala Val Tyr Tyr Cys Gln Asn
85 90 95
Ala Tyr Thr Tyr Pro Phe Thr Phe Gly Gln Gly Thr Lys Leu Glu Ile
100 105 110
Lys Arg Thr Val Ala Ala Pro Ser Val Phe Ile Phe Pro Pro Ser Asp
115 120 125
Glu Gln Leu Lys Ser Gly Thr Ala Ser Val Val Cys Leu Leu Asn Asn
130 135 140
Phe Tyr Pro Arg Glu Ala Lys Val Gln Trp Lys Val Asp Asn Ala Leu
145 150 155 160
Gln Ser Gly Asn Ser Gln Glu Ser Val Thr Glu Gln Asp Ser Lys Asp
165 170 175
Ser Thr Tyr Ser Leu Ser Ser Thr Leu Thr Leu Ser Lys Ala Asp Tyr
180 185 190
Glu Lys His Lys Val Tyr Ala Cys Glu Val Thr His Gln Gly Leu Ser
195 200 205

Ser Pro Val Thr Lys Ser Phe Asn Arg Gly Glu Cys
210 215 220

<210> 49
<211> 448
<212> PRT
<213> Artificial sequence

<220>
<223> H11

<400> 49
Glu Val Gln Leu Val Gln Ser Gly Ala Glu Val Lys Lys Pro Gly Ala
1 5 10 15
Ser Val Lys Val Ser Cys Lys Ala Ser Gly Tyr Thr Phe Thr Ser Tyr
20 25 30
Trp Met His Trp Val Arg Gln Ala Pro Gly Gln Arg Leu Glu Trp Met
35 40 45
Gly Met Ile His Pro Asn Ser Gly Ser Thr Asn Tyr Asn Glu Lys Phe
50 55 60
Lys Gly Arg Val Thr Ile Thr Arg Asp Thr Ser Ala Ser Thr Ala Tyr
65 70 75 80
Met Glu Leu Ser Ser Leu Arg Ser Glu Asp Thr Ala Val Tyr Tyr Cys
85 90 95
Ala Arg Leu Lys Thr Gly Asn Ser Phe Asp Tyr Trp Gly Gln Gly Thr
100 105 110
Thr Val Thr Val Ser Ser Ala Ser Thr Lys Gly Pro Ser Val Phe Pro
115 120 125
Leu Ala Pro Ser Ser Lys Ser Thr Ser Gly Gly Thr Ala Ala Leu Gly
130 135 140
Cys Leu Val Lys Asp Tyr Phe Pro Glu Pro Val Thr Val Ser Trp Asn
145 150 155 160
Ser Gly Ala Leu Thr Ser Gly Val His Thr Phe Pro Ala Val Leu Gln
165 170 175
Ser Ser Gly Leu Tyr Ser Leu Ser Ser Val Val Thr Val Pro Ser Ser
180 185 190
Ser Leu Gly Thr Gln Thr Tyr Ile Cys Asn Val Asn His Lys Pro Ser
195 200 205
Asn Thr Lys Val Asp Lys Lys Val Glu Pro Lys Ser Cys Asp Lys Thr
210 215 220
His Thr Cys Pro Pro Cys Pro Ala Pro Glu Leu Leu Gly Gly Pro Ser
225 230 235 240
Val Phe Leu Phe Pro Pro Lys Pro Lys Asp Thr Leu Met Ile Ser Arg
245 250 255
Thr Pro Glu Val Thr Cys Val Val Val Asp Val Ser His Glu Asp Pro
260 265 270
Glu Val Lys Phe Asn Trp Tyr Val Asp Gly Val Glu Val His Asn Ala
275 280 285
Lys Thr Lys Pro Arg Glu Glu Gln Tyr Asn Ser Thr Tyr Arg Val Val
290 295 300
Ser Val Leu Thr Val Leu His Gln Asp Trp Leu Asn Gly Lys Glu Tyr
305 310 315 320
Lys Cys Lys Val Ser Asn Lys Ala Leu Pro Ala Pro Ile Glu Lys Thr
325 330 335
Ile Ser Lys Ala Lys Gly Gln Pro Arg Glu Pro Gln Val Tyr Thr Leu
340 345 350
Pro Pro Ser Arg Asp Glu Leu Thr Lys Asn Gln Val Ser Leu Thr Cys
355 360 365
Leu Val Lys Gly Phe Tyr Pro Ser Asp Ile Ala Val Glu Trp Glu Ser
370 375 380
Asn Gly Gln Pro Glu Asn Asn Tyr Lys Thr Thr Pro Pro Val Leu Asp
385 390 395 400

Ser Asp Gly Ser Phe Phe Leu Tyr Ser Lys Leu Thr Val Asp Lys Ser
405 410 415
Arg Trp Gln Gln Gly Asn Val Phe Ser Cys Ser Val Met His Glu Ala
420 425 430
Leu His Asn His Tyr Thr Gln Lys Ser Leu Ser Leu Ser Pro Gly Lys
435 440 445

<210> 50
<211> 448
<212> PRT
<213> Artificial sequence

<220>
<223> H12

<400> 50
Glu Val Gln Leu Val Gln Ser Gly Ala Glu Val Lys Lys Pro Gly Ala
1 5 10 15
Ser Val Lys Val Ser Cys Lys Ala Ser Gly Tyr Thr Phe Thr Ser Tyr
20 25 30
Trp Met His Trp Val Arg Gln Ala Pro Gly Gln Arg Leu Glu Trp Met
35 40 45
Gly Met Ile His Pro Asn Ser Gly Ser Thr Asn Tyr Asn Glu Lys Phe
50 55 60
Lys Gly Arg Val Thr Leu Thr Leu Asp Lys Ser Ala Ser Thr Ala Tyr
65 70 75 80
Met Glu Leu Ser Ser Leu Arg Ser Glu Asp Thr Ala Val Tyr Tyr Cys
85 90 95
Ala Arg Leu Lys Thr Gly Asn Ser Phe Asp Tyr Trp Gly Gln Gly Thr
100 105 110
Thr Val Thr Val Ser Ser Ala Ser Thr Lys Gly Pro Ser Val Phe Pro
115 120 125
Leu Ala Pro Ser Ser Lys Ser Thr Ser Gly Gly Thr Ala Ala Leu Gly
130 135 140
Cys Leu Val Lys Asp Tyr Phe Pro Glu Pro Val Thr Val Ser Trp Asn
145 150 155 160
Ser Gly Ala Leu Thr Ser Gly Val His Thr Phe Pro Ala Val Leu Gln
165 170 175
Ser Ser Gly Leu Tyr Ser Leu Ser Ser Val Val Thr Val Pro Ser Ser
180 185 190
Ser Leu Gly Thr Gln Thr Tyr Ile Cys Asn Val Asn His Lys Pro Ser
195 200 205
Asn Thr Lys Val Asp Lys Lys Val Glu Pro Lys Ser Cys Asp Lys Thr
210 215 220
His Thr Cys Pro Pro Cys Pro Ala Pro Glu Leu Leu Gly Gly Pro Ser
225 230 235 240
Val Phe Leu Phe Pro Pro Lys Pro Lys Asp Thr Leu Met Ile Ser Arg
245 250 255
Thr Pro Glu Val Thr Cys Val Val Val Asp Val Ser His Glu Asp Pro
260 265 270
Glu Val Lys Phe Asn Trp Tyr Val Asp Gly Val Glu Val His Asn Ala
275 280 285
Lys Thr Lys Pro Arg Glu Glu Gln Tyr Asn Ser Thr Tyr Arg Val Val
290 295 300
Ser Val Leu Thr Val Leu His Gln Asp Trp Leu Asn Gly Lys Glu Tyr
305 310 315 320
Lys Cys Lys Val Ser Asn Lys Ala Leu Pro Ala Pro Ile Glu Lys Thr
325 330 335
Ile Ser Lys Ala Lys Gly Gln Pro Arg Glu Pro Gln Val Tyr Thr Leu
340 345 350
Pro Pro Ser Arg Asp Glu Leu Thr Lys Asn Gln Val Ser Leu Thr Cys
355 360 365

Leu Val Lys Gly Phe Tyr Pro Ser Asp Ile Ala Val Glu Trp Glu Ser
    370                 375                 380
Asn Gly Gln Pro Glu Asn Asn Tyr Lys Thr Thr Pro Pro Val Leu Asp
385                 390                 395                 400
Ser Asp Gly Ser Phe Phe Leu Tyr Ser Lys Leu Thr Val Asp Lys Ser
                405                 410                 415
Arg Trp Gln Gln Gly Asn Val Phe Ser Cys Ser Val Met His Glu Ala
            420                 425                 430
Leu His Asn His Tyr Thr Gln Lys Ser Leu Ser Leu Ser Pro Gly Lys
        435                 440                 445

<210> 51
<211> 448
<212> PRT
<213> Artificial sequence

<220>
<223> H13

<400> 51
Glu Val Gln Leu Val Gln Ser Gly Ala Glu Val Lys Lys Pro Gly Ala
1               5                   10                  15
Ser Val Lys Val Ser Cys Lys Ala Ser Gly Tyr Thr Phe Thr Ser Tyr
            20                  25                  30
Trp Met His Trp Val Arg Gln Ala Pro Gly Gln Arg Leu Glu Trp Ile
        35                  40                  45
Gly Met Ile His Pro Asn Ser Gly Ser Thr Asn Tyr Asn Glu Lys Phe
    50                  55                  60
Lys Gly Lys Ala Thr Leu Thr Leu Asp Lys Ser Ala Ser Thr Ala Tyr
65                  70                  75                  80
Met Glu Leu Ser Ser Leu Arg Ser Glu Asp Thr Ala Val Tyr Tyr Cys
                85                  90                  95
Ala Arg Leu Lys Thr Gly Asn Ser Phe Asp Tyr Trp Gly Gln Gly Thr
            100                 105                 110
Thr Val Thr Val Ser Ser Ala Ser Thr Lys Gly Pro Ser Val Phe Pro
        115                 120                 125
Leu Ala Pro Ser Ser Lys Ser Thr Ser Gly Gly Thr Ala Ala Leu Gly
    130                 135                 140
Cys Leu Val Lys Asp Tyr Phe Pro Glu Pro Val Thr Val Ser Trp Asn
145                 150                 155                 160
Ser Gly Ala Leu Thr Ser Gly Val His Thr Phe Pro Ala Val Leu Gln
                165                 170                 175
Ser Ser Gly Leu Tyr Ser Leu Ser Ser Val Val Thr Val Pro Ser Ser
            180                 185                 190
Ser Leu Gly Thr Gln Thr Tyr Ile Cys Asn Val Asn His Lys Pro Ser
        195                 200                 205
Asn Thr Lys Val Asp Lys Lys Val Glu Pro Lys Ser Cys Asp Lys Thr
    210                 215                 220
His Thr Cys Pro Pro Cys Pro Ala Pro Glu Leu Leu Gly Gly Pro Ser
225                 230                 235                 240
Val Phe Leu Phe Pro Pro Lys Pro Lys Asp Thr Leu Met Ile Ser Arg
                245                 250                 255
Thr Pro Glu Val Thr Cys Val Val Val Asp Val Ser His Glu Asp Pro
            260                 265                 270
Glu Val Lys Phe Asn Trp Tyr Val Asp Gly Val Glu Val His Asn Ala
        275                 280                 285
Lys Thr Lys Pro Arg Glu Glu Gln Tyr Asn Ser Thr Tyr Arg Val Val
    290                 295                 300
Ser Val Leu Thr Val Leu His Gln Asp Trp Leu Asn Gly Lys Glu Tyr
305                 310                 315                 320
Lys Cys Lys Val Ser Asn Lys Ala Leu Pro Ala Pro Ile Glu Lys Thr
                325                 330                 335

```
Ile Ser Lys Ala Lys Gly Gln Pro Arg Glu Pro Gln Val Tyr Thr Leu
            340                 345                 350
Pro Pro Ser Arg Asp Glu Leu Thr Lys Asn Gln Val Ser Leu Thr Cys
        355                 360                 365
Leu Val Lys Gly Phe Tyr Pro Ser Asp Ile Ala Val Glu Trp Glu Ser
    370                 375                 380
Asn Gly Gln Pro Glu Asn Asn Tyr Lys Thr Thr Pro Pro Val Leu Asp
385                 390                 395                 400
Ser Asp Gly Ser Phe Phe Leu Tyr Ser Lys Leu Thr Val Asp Lys Ser
                405                 410                 415
Arg Trp Gln Gln Gly Asn Val Phe Ser Cys Ser Val Met His Glu Ala
            420                 425                 430
Leu His Asn His Tyr Thr Gln Lys Ser Leu Ser Leu Ser Pro Gly Lys
        435                 440                 445

<210>  52
<211>  448
<212>  PRT
<213>  Artificial sequence

<220>
<223>  H14


<400>  52
Glu Val Gln Leu Val Gln Ser Gly Ala Glu Val Lys Lys Pro Gly Ala
1               5                   10                  15
Ser Val Lys Val Ser Cys Lys Ala Ser Gly Tyr Thr Phe Thr Ser Tyr
            20                  25                  30
Trp Met His Trp Val Lys Gln Arg Pro Gly Gln Arg Leu Glu Trp Ile
        35                  40                  45
Gly Met Ile His Pro Asn Ser Gly Ser Thr Asn Tyr Asn Glu Lys Phe
    50                  55                  60
Lys Gly Lys Ala Thr Leu Thr Leu Asp Lys Ser Ala Ser Thr Ala Tyr
65                  70                  75                  80
Met Glu Leu Ser Ser Leu Arg Ser Glu Asp Thr Ala Val Tyr Tyr Cys
                85                  90                  95
Ala Arg Leu Lys Thr Gly Asn Ser Phe Asp Tyr Trp Gly Gln Gly Thr
            100                 105                 110
Thr Val Thr Val Ser Ser Ala Ser Thr Lys Gly Pro Ser Val Phe Pro
        115                 120                 125
Leu Ala Pro Ser Ser Lys Ser Thr Ser Gly Gly Thr Ala Ala Leu Gly
    130                 135                 140
Cys Leu Val Lys Asp Tyr Phe Pro Glu Pro Val Thr Val Ser Trp Asn
145                 150                 155                 160
Ser Gly Ala Leu Thr Ser Gly Val His Thr Phe Pro Ala Val Leu Gln
                165                 170                 175
Ser Ser Gly Leu Tyr Ser Leu Ser Ser Val Val Thr Val Pro Ser Ser
            180                 185                 190
Ser Leu Gly Thr Gln Thr Tyr Ile Cys Asn Val Asn His Lys Pro Ser
        195                 200                 205
Asn Thr Lys Val Asp Lys Lys Val Glu Pro Lys Ser Cys Asp Lys Thr
    210                 215                 220
His Thr Cys Pro Pro Cys Pro Ala Pro Glu Leu Leu Gly Gly Pro Ser
225                 230                 235                 240
Val Phe Leu Phe Pro Pro Lys Pro Lys Asp Thr Leu Met Ile Ser Arg
                245                 250                 255
Thr Pro Glu Val Thr Cys Val Val Val Asp Val Ser His Glu Asp Pro
            260                 265                 270
Glu Val Lys Phe Asn Trp Tyr Val Asp Gly Val Glu Val His Asn Ala
        275                 280                 285
Lys Thr Lys Pro Arg Glu Glu Gln Tyr Asn Ser Thr Tyr Arg Val Val
    290                 295                 300
```

Ser Val Leu Thr Val Leu His Gln Asp Trp Leu Asn Gly Lys Glu Tyr
305 310 315 320
Lys Cys Lys Val Ser Asn Lys Ala Leu Pro Ala Pro Ile Glu Lys Thr
325 330 335
Ile Ser Lys Ala Lys Gly Gln Pro Arg Glu Pro Gln Val Tyr Thr Leu
340 345 350
Pro Pro Ser Arg Asp Glu Leu Thr Lys Asn Gln Val Ser Leu Thr Cys
355 360 365
Leu Val Lys Gly Phe Tyr Pro Ser Asp Ile Ala Val Glu Trp Glu Ser
370 375 380
Asn Gly Gln Pro Glu Asn Asn Tyr Lys Thr Thr Pro Pro Val Leu Asp
385 390 395 400
Ser Asp Gly Ser Phe Phe Leu Tyr Ser Lys Leu Thr Val Asp Lys Ser
405 410 415
Arg Trp Gln Gln Gly Asn Val Phe Ser Cys Ser Val Met His Glu Ala
420 425 430
Leu His Asn His Tyr Thr Gln Lys Ser Leu Ser Leu Ser Pro Gly Lys
435 440 445

<210> 53
<211> 448
<212> PRT
<213> Artificial sequence

<220>
<223> IMAB-362 heavy chain

<400> 53
Gln Val Gln Leu Gln Gln Pro Gly Ala Glu Leu Val Arg Pro Gly Ala
1 5 10 15
Ser Val Lys Leu Ser Cys Lys Ala Ser Gly Tyr Thr Phe Thr Ser Tyr
20 25 30
Trp Ile Asn Trp Val Lys Gln Arg Pro Gly Gln Gly Leu Glu Trp Ile
35 40 45
Gly Asn Ile Tyr Pro Ser Asp Ser Tyr Thr Asn Tyr Asn Gln Lys Phe
50 55 60
Lys Asp Lys Ala Thr Leu Thr Val Asp Lys Ser Ser Ser Thr Ala Tyr
65 70 75 80
Met Gln Leu Ser Ser Pro Thr Ser Glu Asp Ser Ala Val Tyr Tyr Cys
85 90 95
Thr Arg Ser Trp Arg Gly Asn Ser Phe Asp Tyr Trp Gly Gln Gly Thr
100 105 110
Thr Leu Thr Val Ser Ser Ala Ser Thr Lys Gly Pro Ser Val Phe Pro
115 120 125
Leu Ala Pro Ser Ser Lys Ser Thr Ser Gly Gly Thr Ala Ala Leu Gly
130 135 140
Cys Leu Val Lys Asp Tyr Phe Pro Glu Pro Val Thr Val Ser Trp Asn
145 150 155 160
Ser Gly Ala Leu Thr Ser Gly Val His Thr Phe Pro Ala Val Leu Gln
165 170 175
Ser Ser Gly Leu Tyr Ser Leu Ser Ser Val Val Thr Val Pro Ser Ser
180 185 190
Ser Leu Gly Thr Gln Thr Tyr Ile Cys Asn Val Asn His Lys Pro Ser
195 200 205
Asn Thr Lys Val Asp Lys Arg Val Glu Pro Lys Ser Cys Asp Lys Thr
210 215 220
His Thr Cys Pro Pro Cys Pro Ala Pro Glu Leu Leu Gly Gly Pro Ser
225 230 235 240
Val Phe Leu Phe Pro Pro Lys Pro Lys Asp Thr Leu Met Ile Ser Arg
245 250 255
Thr Pro Glu Val Thr Cys Val Val Val Asp Val Ser His Glu Asp Pro
260 265 270

```
Glu Val Lys Phe Asn Trp Tyr Val Asp Gly Val Glu Val His Asn Ala
        275                 280                 285
Lys Thr Lys Pro Arg Glu Glu Gln Tyr Asn Ser Thr Tyr Arg Val Val
    290                 295                 300
Ser Val Leu Thr Val Leu His Gln Asp Trp Leu Asn Gly Lys Glu Tyr
305                 310                 315                 320
Lys Cys Lys Val Ser Asn Lys Ala Leu Pro Ala Pro Ile Glu Lys Thr
                325                 330                 335
Ile Ser Lys Ala Lys Gly Gln Pro Arg Glu Pro Gln Val Tyr Thr Leu
            340                 345                 350
Pro Pro Ser Arg Glu Glu Met Thr Lys Asn Gln Val Ser Leu Thr Cys
        355                 360                 365
Leu Val Lys Gly Phe Tyr Pro Ser Asp Ile Ala Val Glu Trp Glu Ser
    370                 375                 380
Asn Gly Gln Pro Glu Asn Asn Tyr Lys Thr Thr Pro Pro Val Leu Asp
385                 390                 395                 400
Ser Asp Gly Ser Phe Phe Leu Tyr Ser Lys Leu Thr Val Asp Lys Ser
                405                 410                 415
Arg Trp Gln Gln Gly Asn Val Phe Ser Cys Ser Val Met His Glu Ala
            420                 425                 430
Leu His Asn His Tyr Thr Gln Lys Ser Leu Ser Leu Ser Pro Gly Lys
        435                 440                 445
```

<210> 54
<211> 220
<212> PRT
<213> Artificial sequence

<220>
<223> IMAB-362 light chain

<400> 54

```
Asp Ile Val Met Thr Gln Ser Pro Ser Ser Leu Thr Val Thr Ala Gly
1               5                   10                  15
Glu Lys Val Thr Met Ser Cys Lys Ser Ser Gln Ser Leu Leu Asn Ser
            20                  25                  30
Gly Asn Gln Lys Asn Tyr Leu Thr Trp Tyr Gln Gln Lys Pro Gly Gln
        35                  40                  45
Pro Pro Lys Leu Leu Ile Tyr Trp Ala Ser Thr Arg Glu Ser Gly Val
    50                  55                  60
Pro Asp Arg Phe Thr Gly Ser Gly Ser Gly Thr Asp Phe Thr Leu Thr
65                  70                  75                  80
Ile Ser Ser Val Gln Ala Glu Asp Leu Ala Val Tyr Tyr Cys Gln Asn
                85                  90                  95
Asp Tyr Ser Tyr Pro Phe Thr Phe Gly Ser Gly Thr Lys Leu Glu Ile
            100                 105                 110
Lys Arg Thr Val Ala Ala Pro Ser Val Phe Ile Phe Pro Pro Ser Asp
        115                 120                 125
Glu Gln Leu Lys Ser Gly Thr Ala Ser Val Val Cys Leu Leu Asn Asn
    130                 135                 140
Phe Tyr Pro Arg Glu Ala Lys Val Gln Trp Lys Val Asp Asn Ala Leu
145                 150                 155                 160
Gln Ser Gly Asn Ser Gln Glu Ser Val Thr Glu Gln Asp Ser Lys Asp
                165                 170                 175
Ser Thr Tyr Ser Leu Ser Ser Thr Leu Thr Leu Ser Lys Ala Asp Tyr
            180                 185                 190
Glu Lys His Lys Val Tyr Ala Cys Glu Val Thr His Gln Gly Leu Ser
        195                 200                 205
Ser Pro Val Thr Lys Ser Phe Asn Arg Gly Glu Cys
    210                 215                 220
```

<210> 55

```
<211>  4
<212>  PRT
<213>  Artificial sequence

<220>
<223>  Tetrapeptide linker


<400>  55
Gly Gly Phe Gly
1           4
```